# FASCICULE DE BREVET EUROPEEN

(11) **EP 0 820 455 B1**
(45) Date de publication et mention de la délivrance du brevet: **31.10.2001**
(21) Numéro de dépôt: 96911018.8
(22) Date de dépôt: 02.04.1996
(51) Int. Cl.: C07D 487/04, A61K 31/495, C07D 233/90, C07F 9/6561, C07F 9/6506, A61K 31/675

(54) **DERIVES DE 5H, 10H-IMIDAZO [1,2-a] INDENO [1,2-e] PYRAZIN-4-ONE, LEUR PREPARATION ET LES MEDICAMENTS LES CONTENANT**
5H, 10H-IMIDAZO [1,2-a] INDENO [1,2-e] PYRAZIN -4-ONE DERIVATE IHRE HERSTELLUNG UND DIESE ENTHALTENDE ARZNEIMITTEL
5H, 10H-IMIDAZO [1,2-a]INDENO [1,2-e]PYRAZIN-4-ONE DERIVATIVES, PREPARATION THEREOF, AND DRUGS CONTAINING SAID DERIVATIVES

(30) Priorité: 05.04.1995 FR 9504013
(43) Date de publication de la demande: 28.01.1998
(73) Titulaire: Aventis Pharma S.A., 92160 Antony (FR)
(72) Inventeur: ALOUP, Jean-Claude, F-94290 Villeneuve-le-Roi (FR); AUDIAU, François, F-94220 Charenton-le-Pont (FR); BARREAU, Michel, F-91230 Montgeron (FR); DAMOUR, Dominique, F-75014 Paris (FR); GENEVOIS-BORELLA, Arielle, F-94320 Thiais (FR); HARDY, Jean-Claude, F-95800 Cergy-Saint-Christophe (FR); JIMONET, Patrick, F-78450 Villepreux (FR); MANFRE, Franco, F-94450 Limeil-Brevannes (FR); MIGNANI, Serge, F-92290 Chatenay-Malabry (FR); NEMECEK, Patrick, F-94320 Thiais (FR); RIBEILL, Yves, F-91360 Villemoisson-sur-Orge (FR)
(74) Mandataire: Savina, Jacques
(86) Numéro de dépôt international: FR9600496
(87) Numéro de publication internationale: WO9631511

(56) Documents cités:
- WO-A-94/07893
- FR-A- 2 696 466

## Description

La présente invention concerne des composés de formule : 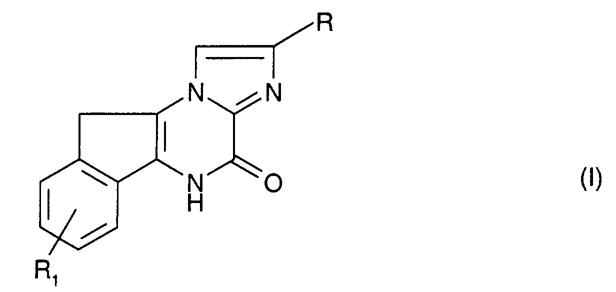 leurs sels, leurs énantiomères et diastéréoisomères, leur préparation et les médicaments les contenant.
Des antagonistes NMDA et AMPA dérivés de 5H,10H-imidazo[1,2-a]pyrazin-4-one sont décrits dans la demande de brevet WO9407893.
Dans la formule (I),
- R représente un atome d'hydrogène ou un radical carboxy, alcoxycarbonyle, -CO-NR₄R₅, -PO₃H₂ ou -CH₂OH,
- R₁ représente un radical -alk-NH₂, -alk-NH-CO-R₃, -alk-COOR₄, -alk-CO-NR₅R₆ ou -CO-NH-R₇,
- R₃ représente un radical alkyle, phényle, phénylalkyle, cycloalkyle ou -NR₆R₈,
- R₄ représente un atome d'hydrogène ou un radical alkyle,
- R₅ représente un atome d'hydrogène ou un radical alkyle, phényle, cycloalkyle ou phénylalkyle,
- R₆ représente un atome d'hydrogène ou un radical alkyle,
ou bien R₅ et R₆ forment avec l'atome d'azote auquel ils sont rattachés un hétérocycle mono ou polycyclique saturé ou insaturé contenant 1 à 6 atomes de carbone et éventuellement un ou plusieurs autres hétéroatomes choisis parmi O, S, N,
- R₇ représente un radical phényle, phénylalkyle ou -alk-COOR₄,
- R₈ représente un atome d'hydrogène ou un radical alkyle, cycloalkyle ou phénylalkyle,
- alk représente un radical alkylène.

Sauf mention contraire, dans les définitions qui précédent et celles qui suivent, les radicaux et portions alcoxy, alkyle et alkylène contiennent 1 à 6 atomes de carbone et sont en chaîne droite ou ramifiée et les radicaux cycloalkyle contiennent 3 à 6 atomes de carbone.

De préférence, lorsque R₅ et R₆ forment avec l'atome d'azote auquel ils sont rattachés un hétérocycle celui-ci est choisi parmi les cycles azétidine, pyrrolidine, pipéridine, pipérazine et morpholine.

De préférence, le substituant R₁ est en position -8 ou -9.

Les composés de formule (I) peuvent être préparés par cyclisation en présence d'acétate d'ammonium d'un dérivé de formule : 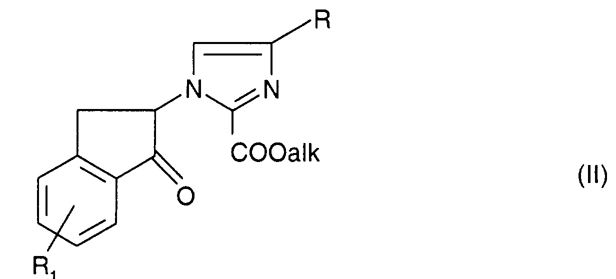 dans laquelle R et R₁ ont les mêmes significations que dans la formule (I) et alk représente un radical alkyle.

Cette cyclisation s'effectue au sein d'un acide organique tel que l'acide acétique, à la température d'ébullition du milieu réactionnel.

Les dérivés de formule (II) sont nouveaux et font partie de l'invention. Ils peuvent être obtenus par action d'une indanone de formule : 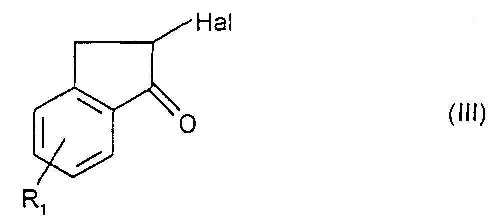 dans laquelle R₁ a les mêmes significations que dans la formule (I) sur un dérivé de formule : 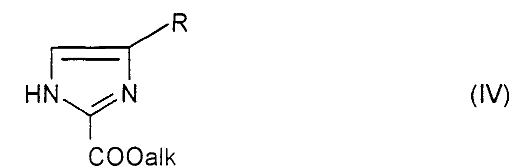 dans laquelle R a les mêmes significations que dans la formule (I) et alk représente un radical alkyle.

Cette réaction s'effectue généralement au sein d'un solvant inerte tel qu'un alcool (méthanol, éthanol par exemple), une cétone (acétone par exemple), un hydrocarbure aromatique (toluène par exemple), le diméthylformamide ou en absence de solvant, éventuellement en présence d'une base telle que l'hydrure de sodium ou le carbonate de potassium avec éventuellement un éther couronne tel que le 18C6, à une température comprise entre 20°C et la température d'ébullition du milieu réactionnel ou la température de fusion du milieu réactionnel.

Les dérivés de formule (III) peuvent être obtenus par application ou adaptation des méthodes décrites par OLIVIER et coll., Bull. Soc. Chim. France, 3092 (1973), dans le brevet DE 2640358 et dans les exemples.

Les dérivés de formule (IV) peuvent être obtenus par application ou adaptation des méthodes décrites par P.S. BRANCO et coll., Tetrahedron, 48 (30), 6335 (1992), J.E. OLIVIER et coll., J. Org. Chem., 38 (7), 1437 (1973) et dans le brevet US 3600399 et dans les exemples.

Les composés de formule (I) pour lesquels R représente un radical carboxy et/ou R₁ représente un radical -alk-COOR₄ dans lequel R₄ représente un atome d'hydrogène ou -CO-NH-R₇ dans lequel R₇ représente un radical -alk-COOR₄ et R₄ représente un atome d'hydrogène peuvent également être préparés par hydrolyse d'un composé de formule (I) correspondant pour lequel R représente un radical alcoxycarbonyle et/ou R₁ représente un radical -alk-COOR₄ dans lequel R₄ représente un radical alkyle ou -CO-NH-R₇ dans lequel R₇ représente un radical -alk-COOR₄ et R₄ représente un radical alkyle.

Cette réaction s'effectue généralement au moyen d'une base telle qu'un hydroxyde de métal alcalin (soude, potasse par exemple) au sein d'un mélange d'un solvant inerte (dioxanne par exemple) et d'eau, à une température comprise entre 20°C et la température d'ébullition du milieu réactionnel ou au moyen d'un acide minéral tel que l'acide chlorhydrique, au sein d'un solvant inerte tel que le dioxanne et l'acide acétique, à la température d'ébullition du milieu réactionnel.

Les composés de formule (I) pour lesquels R représente un radical -PO₃H₂ peuvent également être préparés par hydrolyse d'un dérivé de formule : 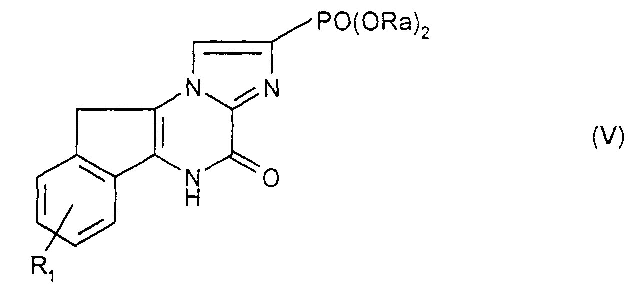 dans laquelle R₁ a les mêmes significations que dans la formule (I) et Ra représente un radical méthyle, éthyle ou benzyle.

Cette réaction s'effectue généralement au moyen de bromotriméthylsilane, au sein d'un solvant chloré (dichlorométhane, chloroforme par exemple), à une température voisine de 20°C. Les dérivés (V) peuvent être obtenus par une cyclisation analogue à celle décrite précédemment pour l'obtention des composés de formule (I) et de la méthode décrite dans les exemples.

Les composés de formule (I) pour lesquels R représente un radical -CH₂OH peuvent également être préparés par réduction d'un composé de formule (I) correspondant pour lequel R représente un radical alcoxycarbonyle.

Cette réduction s'effectue généralement au moyen d'un hydrure de lithium et de bore ou de lithium et d'aluminium, au sein d'un solvant inerte tel que le tétrahydrofuranne, le dioxanne, à une température comprise entre 20°C et la température d'ébullition du milieu réactionnel.

Les composés de formule (I) pour lesquels R représente un radical -CO-NR₄R₅ peuvent également être préparés par action d'un complexe d'aluminium formé à partir d'un trialkylaluminium (triméthylaluminium par exemple) et du chlorhydrate d'une amine HNR₄R₅ sur un composé de formule (I) correspondant pour lequel R représente un radical alcoxycarbonyle.

Cette réaction s'effectue généralement au sein d'un solvant inerte tel qu'un hydrocarbure (toluène par exemple), à une température comprise entre 20°C et la température d'ébullition du milieu réactionnel.

Les amines HNR₄R₅ sont commercialisées ou peuvent être obtenues par réaction de l'amine primaire H₂NR₅ sur un dérivé HalR₄ dans lequel Hal représente un atome d'halogène (chlore, brome par exemple) et R₄ a les mêmes significations que dans la formule (I) à l'exception d'hydrogène. Cette réaction s'effectue généralement au sein d'un solvant inerte tel que le diméthylformamide, en présence d'une trialkylamine (triéthylamine par exemple), à une température voisine de 20°C.

Les composés de formule (I) pour lesquels R₁ représente un radical -alk-NH-CO-R₃ dans lequel R₃ représente un radical alkyle (1C) peuvent également être préparés par action d'un composé de formule (I) correspondant pour lequel R₁ représente un radical -alk-NH₂ sur l'anhydride acétique.

Cette réaction s'effectue généralement sans solvant ou au sein d'un solvant inerte tel que l'acide acétique, le diméthylformamide, à une température voisine de 20°C, éventuellement en présence d'une base telle qu'une trialkylamine (triéthylamine par exemple) ou l'acétate de sodium.

Les composés de formule (I) pour lesquels R₁ représente un radical -alk-NH-CO-R₃ dans lequel R₃ représente un radical -NR₆R₈, R₆ représente un atome d'hydrogène et R₈ représente un atome d'hydrogène ou un radical alkyle, cycloalkyle ou phénylalkyle peuvent également être préparés par action d'un composé de formule (I) correspondant pour lequel R₁ représente un radical -alk-NH₂ sur un isocyanate R_{b}-NCO dans lequel R_{b} représente un radical triméthylsilyle, alkyle, cycloalkyle ou phénylalkyle suivie éventuellement d'une hydrolyse du dérivé silylé.

Cette réaction s'effectue généralement au sein d'un solvant inerte tel que le diméthylformamide, le tétrahydrofuranne, le dioxanne, à une température comprise entre 20°C et la température d'ébullition du milieu réactionnel. L'hydrolyse du dérivé silylé s'effectue à une température comprise entre 20 et 50°C.

Les composés de formule (I) pour lesquels R₁ représente un radical -alk-NH-CO-R₃ dans lequel R₃ représente un radical -NR₆R₈, R₆ représente un atome d'hydrogène et R₈ représente un radical alkyle, cycloalkyle ou phénylalkyle peuvent également être préparés par action d'un composé de formule (I) correspondant pour lequel R₁ représente un radical -alk-NH₂ sur un dérivé de formule : 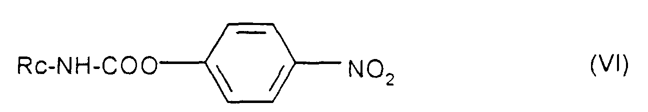 dans laquelle Rc représente un radical alkyle, cycloalkyle ou phénylalkyle.

Cette réaction s'effectue généralement au sein d'un solvant inerte tel que le diméthylformamide, le tétrahydrofuranne, le dioxanne, à une température comprise entre 20°C et la température d'ébullition du milieu réactionnel.

Les dérivés de formule (VI) peuvent être obtenus par application ou adaptation des méthodes décrites par J. IZDEBSKI et coll., Synthesis, 423 (1989) et T. KONAKAHARA et coll., Synthesis, 103 (1993).

Les composés de formule (I) pour lesquels R₁ représente un radical -alk-NH-CO-R₃ dans lequel R₃ représente un radical alkyle, phényle, phénylalkyle, cycloalkyle ou -NR₆R₈, R₆ représente un radical alkyle et R₈ représente un radical alkyle, cycloalkyle ou phénylalkyle peuvent également être préparés par action d'un composé de formule (I) correspondant pour lequel R₁ représente un radical -alk-NH₂ sur un dérivé Hal-CO-R₃ dans lequel Hal représente un atome d'halogène et de préférence un atome de chlore et R₃ a les mêmes significations que précédemment.

Cette réaction s'effectue de préférence au sein d'un solvant inerte tel que le diméthylformamide, en présence d'un accepteur d'acide tel qu'une trialkylamine (triéthylamine par exemple), à une température comprise entre 20°C et la température d'ébullition du milieu réactionnel.

Les dérivés Hal-CO-R₃ sont commercialisés ou ceux pour lesquels R₃ représente un radical -NR₆R₈ peuvent être préparés par action de l'amine HNR₆R₈ avec le phosgène par application ou adaptation de la méthode décrite par H. TILLES, J. Am. Chem. Soc., 81, 714 (1959). Les amines HNR₆R₈ sont commercialisées ou peuvent être obtenues par réaction de l'amine primaire H₂NR₈ sur un dérivé HalR₆ dans lequel Hal représente un atome d'halogène (chlore, brome par exemple) et R₆ a les mêmes significations que dans la formule (I) à l'exception d'hydrogène. Cette réaction s'effectue généralement au sein d'un solvant inerte tel que le diméthylformamide, en présence d'une trialkylamine (triéthylamine par exemple), à une température voisine de 20°C.

Les composés de formule (I) pour lesquels R₁ représente un radical - alk-CO-NR₅R₆ peuvent également être préparés par action d'un composé de formule (I) correspondant pour lequel R₁ représente un radical -alk-COOR₄ ou, lorsque R₄ représente un atome d'hydrogène, un dérivé réactif de cet acide sur une amine HNR₅R₆.

Lorsque l'on met en oeuvre l'acide, on opère en présence d'un agent de condensation utilisé en chimie peptidique tel qu'un carbodiimide (par exemple le N,N'-dicyclohexylcarbodiimide, le 1-(3-diméthylaminopropyl)-3-éthylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, 1,2-dichloroéthane, chloroforme par exemple), éventuellement en présence d'hydroxybenzotriazole, à une température comprise entre 0°C et la température de reflux du mélange réactionnel. Lorsque l'on met en oeuvre un ester, on opère alors soit en milieu organique, éventuellement en présence d'un accepteur d'acide tel qu'une base organique azotée (trialkylamine, pyridine, 1,8-diazabicyclo[5.4.0]undéc-7-ène ou 1,5-diazabicyclo[4.3.0]non-5-ène par exemple), dans un solvant tel que cité ci-dessus, ou un mélange de ces solvants ou un alcool aliphatique inférieur, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les amines HNR₅R₆ sont commercialisées ou peuvent être obtenues par réaction de l'amine primaire H₂NR₅ sur un dérivé HalR₆ dans lequel Hal représente un atome d'halogène (chlore, brome par exemple) et R₆ a les mêmes significations que dans la formule (I) à l'exception d'hydrogène. Cette réaction s'effectue généralement au sein d'un solvant inerte tel que le diméthylformamide, en présence d'un trialkylamine (triéthylamine par exemple), à une température voisine de 20°C.

Les composés de formule (I) pour lesquels R₁ représente un radical -alk(1C)-NH₂ peuvent également être préparés par hydrogénation d'un dérivé de formule : 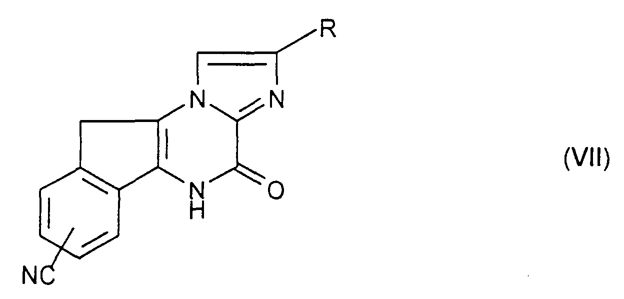 dans laquelle R a les mêmes significations que dans la formule (I).

Cette hydrogénation s'effectue généralement au moyen d'hydrogène, au sein d'un solvant inerte tel que l'acide acétique, l'acide trifluoroacétique ou un mélange de ces solvants, en présence d'un catalyseur d'hydrogénation tel que le charbon palladié, sous pression (50 bar environ), à une température voisine de 20°C.

Les dérivés de formule (VII) peuvent être obtenus par cyclisation d'un dérivé de formule : 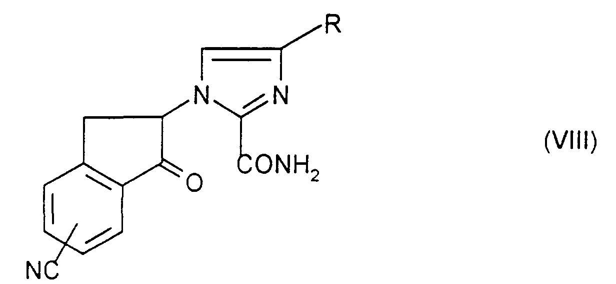 dans laquelle R a les mêmes significations que dans la formule (I).

Cette cyclisation s'effectue généralement au sein de l'acide acétique, à la température d'ébullition du milieu réactionnel.

Les dérivés de formule (VIII) peuvent être obtenus par action d'ammoniac sur l'ester de formule : 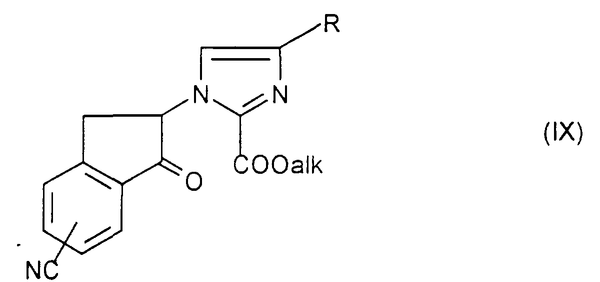 dans laquelle R a les mêmes significations que dans la formule (I) et alk représente un radical alkyle.

Cette réaction s'effectue généralement au sein d'un solvant inerte tel qu'un alcool aliphatique inférieur (méthanol, éthanol par exemple), à une température voisine de 20°C.

Les dérivés de formule (IX) peuvent être obtenus par application ou adaptation de la méthode décrite dans les exemples.

Il est entendu pour l'homme du métier que, pour la mise en oeuvre des procédés selon l'invention décrits précédemment, il peut être nécessaire d'introduire des groupes protecteurs des fonctions amino, carboxy et alcool afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane, les phtalimido qui peuvent être régénérés au moyen d'hydrazine ou de méthylhydrazine ou en milieu acide (acide bromhydrique par exemple). Comme groupes protecteurs des fonctions carboxy, on peut citer les esters (méthoxyméthylester, tétrahydropyranylester, benzylester, tertbutylester par exemple), les oxazoles et les 2-alkyl-1,3-oxazolines. Comme groupes protecteurs des fonctions alcool, on peut citer les esters (benzoylester par exemple), les éthers (méthoxyéthoxyméthyle, titryle, tétrahydropyrranyle) qui peuvent être régénérées en milieu acide. D'autres groupes protecteurs utilisables sont décrits par W. GREENE et coll., Protecting Groups in Organic Synthesis, second edition, 1991, John Wiley & Sons.

Les composés de formule (I) peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les diastéréoisomères des composés de formule (I) peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux tels que le stroke thromboembolique et hémorragique, un arrêt cardiaque, une hypotension artérielle, une intervention chirurgicale cardiaque, vasculaire ou pulmonaire ou une hypoglycémie sévére. Ils sont également utiles dans le traitement des effets dus à une anoxie, qu'elle soit périnatale ou consécutive à une noyade, une haute pression ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113,10-17 (1991), du tinnitus, de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques (DICKENSON et coll., Neurosc. Letters, 121, 263 (1991)), antiinflammatoires (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA ou AMPA, ainsi que les troubles neurologiques associés aux maladies virales telles que les méningites et encéphalites virales, le SIDA (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, I'hydroxybutirique-aminoacidurie, l'encéphalopathie saturnine (intoxication chronique au plomb) et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Les composés de formule (I) préférés sont ceux pour lesquels R représente un atome d'hydrogène ou un radical carboxy, R₁ représente un radical -alk-NH-CO-R₃, -alk-COOR₄, -alk-CO-NR₅R₆ ou -CO-NH-R₇, R₃ représente un radical alkyle ou -NR₆R₈, R4 représente un atome d'hydrogène, R₅ représente un atome d'hydrogène, R₆ représente un radical alkyle, R₇ représente un radical phénylalkyle ou -alk-COOR₄.

Parmi ceux-ci sont préférés les composés suivants :
- acide (4,5-dihydro-4-oxo-10H-imidazo[1,2-a]indéno[1,2-e]pyrazin-9-yl)acétique,
- N-méthyl-2-(4,5-dihydro-4-oxo-10H-imidazo[1,2-a]indéno[1,2-e]pyrazin-9-yl) acétamide,
- N-[(4,5-dihydro-4-oxo-10H-imidazo[1,2-a]indéno[1,2-e]pyrazin-9-yl)méthyl] acétamide,
- 9-[(3-méthyluréido)méthyl]-5H,10H-imidazo[1,2-a]indéno[1,2-e]pyrazin-4-one,
- N-méthyl-[4,5-dihydro-4-oxo-10H-imidazo[1,2-a]indéno[1,2-e]pyrazin-8-yl] acétamide,
- acide 8-N-méthylcarboxamidométhyl-4,5-dihydro-4-oxo-10H-imidazo[1,2-a] indéno[1,2-e]pyrazin-2-carboxylique,
- acide 8-carboxyméthyl-4,5-dihydro-4-oxo-10H-imidazo[1,2-a]indéno[1,2-e] pyrazin-2-carboxylique,
- 8-(3-méthyluréido)méthyl-5H,10H-imidazo[1,2-a]indéno[1,2-e]pyrazin-4-one,
- acide 9-carboxyméthyl-4,5-dihydro-4-oxo-10H-imidazo[1,2-a]indéno[1,2-e] pyrazin-2-carboxylique,
- N-benzyl-(4,5-dihydro-4-oxo-10H-imidazo[1,2-a]indéno[1,2-e]pyrazin-9-yl) carboxamide,
- N-[(4,5-dihydro-4-oxo-10H-imidazo[1,2-a]indéno[1,2-e]pyrazin-9-yl) carbonyl]glycine,
- N-benzyl-(4,5-dihydro-4-oxo-10H-imidazo[1,2-a]indéno[1,2-e]pyrazin-8-yl) carboxamide,
- acide 8-(N-éthylaminocarbonylméthyl)-4,5-dihydro-4-oxo-10H-imidazo[1,2-a]indéno [1,2-e]pyrazin-2-carboxylique,
- 8-(N-éthylaminocarbonylméthyl)-4,5-dihydro-4-oxo-10H-imidazo[1,2-a]indéno[1,2-e]pyrazin-2-carboxylate d'éthyle,
- acide 9-N-benzylcarbamoyl-4,5-dihydro-4-oxo-10H-imidazo[1,2-a]indèno [1,2-e]pyrazin-2-carboxylique,
- acide 8-(2-carboxyéthyl)-4,5-dihydro-4-oxo-10H-imidazo[1,2-a]indéno[1,2-e]pyrazine-2-carboxylique,
- acide 9-[(3-méthyluréido)méthyl]-4,5-dihydro-4-oxo-10H-imidazo[1,2-a] indéno[1,2-e]pyrazin-2-carboxylique,
- acide 9-carboxyméthyl-4,5-dihydro-4-oxo-10H-imidazo[1,2-a]indéno[1,2-e] pyrazin-2-phosphonique,
- acide 9-N-méthylaminocarbonylméthyl-4,5-dihydro-4-oxo-10H-imidazo[1,2-a] indéno[1,2-e]pyrazin-2-carboxylique,
- acide 9-(1-carboxyéthyl)- 4,5-dihydro-4-oxo-10H-imidazo[1,2-a]indéno[1,2-e] pyrazin-2-carboxylique,
leurs sels, leurs énantiomères et diastéréoisomères.

Les exemples suivants illustrent l'invention.

### EXEMPLE 1

A une suspension sous atmosphère d'argon de 3,09 g d'ester éthylique de l'acide (4,5-dihydro-4-oxo-10H-imidazo[1,2-a]indéno[1,2-e]pyrazin-9-yl)acétique dans 150 ml de dioxanne, chauffée à 40°C, on ajoute 37,5 ml d'acide chlorhydrique 8N puis chauffe le mélange pendant 90 heures. Le milieu réactionnel est concentré à sec sous pression réduite (15mm Hg, 2kPa) à 40°C. Le résidu d'évaporation est repris avec 200 ml d'eau distillée, agité, filtré, lavé avec de l'eau puis deux fois avec 40 ml d'éther isopropylique et séché sous pression réduite (1mm Hg; 0,13 kPa). Le produit ainsi obtenu (3,8 g) est dissous dans 125 ml de soude 0,1N par passage aux ultrasons. La phase organique est extraite par 25 ml d'acétate d'éthyle. La phase aqueuse est traitée par 0,3 g de noir végétal, filtrée, acidifiée avec 1,3 ml d'acide chlorhydrique 1N. Le précipité formé est filtré, lavé à l'eau, séché sous pression réduite (1mm Hg; 0,13 kPa) à 60°C. Le produit est mis en solution dans 40 ml de soude 0,1N, la phase aqueuse est filtrée puis diluée par 60 ml d'eau distillée et lyophilisée. On obtient ainsi 1,2 g du sel de sodium de l'acide (4,5-dihydro-4-oxo-10H-imidazo[1,2-a]indéno[1,2-e]pyrazin-9-yl)acétique sous forme de lyophilisat beige clair dont le point de fusion est supérieur à 260°C (Analyse C15H10N3NaO3; % Calculé C : 59,41; H : 3,32; N : 13,86; % Trouvé C : 59,6; H : 3,4; N : 13,50).

### EXEMPLE 2

On chauffe à reflux pendant 18 heures 10,15 g d'ester éthylique de l'acide 1-(4-éthoxycarbonylméthyl-1-oxo-indan-2-yl)imidazole-2-carboxylique et 22 g d'acétate d'ammonium dans 100 ml d'acide acétique. Après refroidissement à une température voisine de 20°C le milieu réactionnel est concentré à sec sous pression réduite (15mm Hg, 2kPa) à 40°C. Le produit obtenu est repris par 200 ml d'eau distillée puis filtré et lavé par 40 ml d'éther isopropylique. Après séchage sous pression réduite (1mm Hg; 0,13 kPa) à 40°C, on obtient 8,9 g de solide beige clair. 1 g de produit est mis en suspension dans 7 ml de soude 0,1N et 13 ml d'eau distillée et agité pendant 15 minutes puis filtré, lavé à l'eau puis à l'éther isopropylique et séché sous pression réduite (15mm Hg, 2kPa) à 20°C. On obtient 0,95 g de solide beige clair purifié par flash-chromatographie sur colonne de silice, en utilisant un mélange dichlorométhane-méthanol (90/10 en volumes) comme éluant. On obtient 0,85 g de solide beige qui est mis en suspension dans 50 ml d'éther éthylique puis filtré et séché sous pression réduite (15mm Hg, 2kPa) à 60°C. On obtient ainsi 0,58 g d'ester éthylique de l'acide (4,5-dihydro-4-oxo-10H-imidazo[1,2-a]indéno[1,2-e]pyrazin-9-yl)acétique sous forme de solide beige dont le point de fusion est supérieur à 260°C (Analyse C17H15N3O3; %Calculé C : 66,01; H : 4,89; N : 13,58; % Trouvé C : 65,89; H : 4,80; N : 13,20).

L'ester éthylique de l'acide 1-(4-éthoxycarbonylméthyl-1-oxo-indan-2-yl)imidazole-2-carboxylique peut être préparé de la manière suivante : 12,7 g d'ester éthylique de l'acide (2-bromo-1-oxo-indan-4-yl)acétique, 6 g de 1-H-imidazole-2-carboxylate d'éthyle et 28,5 g de carbonate de potassium dans 250 ml d'acétone sont portés au reflux pendant 3 heures et 30 minutes sous atmosphère d'argon. Après refroidissement à une température voisine de 20°C, le précipité formé est filtré puis lavé à l'acétone. Le filtrat est concentré à sec sous pression réduite (15mm Hg, 2kPa) à 40°C. Le produit brut est purifié par flash-chromatographie sur colonne de silice en utilisant un mélange acétate d'éthyle-chlorure de méthylène (50/50 en volumes) comme éluant. 10,15 g de produit attendu sont isolés sous forme d'huile brune utilisée telle quelle dans les synthèses ultérieures.

L'ester éthylique de l'acide (2-bromo-1-oxo-indan-4-yl)acétique peut être préparé de la manière suivante : à 10,45 g d'ester éthylique de l'acide (1-oxo-indan-4-yl)acétique dans 130 ml de dichlorométhane sont ajoutés en 10 minutes 2,15 ml d'une solution de brome dans 20 ml de dichlorométhane à une température voisine de 5°C et sous atmosphère d'argon. On laisse revenir le milieu réactionnel à une température voisine de 20°C et poursuit la réaction pendant 2 heures. Le mélange réactionnel est versé dans 100 ml d'eau saturée en chlorure de sodium. La phase organique est lavée par deux fois 100 ml d'eau distillée avant d'être séchée et concentrée pour conduire à 12,7 g de produit attendu sous forme d'une huile brune utilisée telle quelle dans les synthèses ultérieures.

L'ester éthylique de l'acide (1-oxo-indan-4-yl)acétique peut être préparé de la manière suivante : à 9,4 g d'acide (1-oxo-indan-4-yl)acétique dans 200 ml de dichlorométhane sont ajoutés 4,7 ml de chlorure d'oxalyle à température ambiante et sous atmosphère d'argon. Après 4 heures d'agitation à une température voisine de 20°C, 40 ml d'éthanol sont ajoutés au milieu réactionnel et on maintient l'agitation pendant 1 heure. La phase organique est lavée par 2 fois 25 ml d'une solution saturée en hydrogénocarbonate de sodium puis par 2 fois 100 ml d'eau distillée puis séchée et concentrée pour conduire à 10,45 g de produit attendu sous forme d'une huile brune utilisée telle quelle dans les synthèses ultérieures.

L'acide (1-oxo-indan-4-yl)acétique peut être préparé de la manière suivante : 43 g d'acide 3-(2-carboxyméthyl-phényl)propionique dans 250 ml d'acide sulfurique (95%) sont chauffés à 100°C pendant 18 heures. Après refroidissement à une température voisine de 20°C le milieu réactionnel est versé sur 1000 ml d'eau glacée. Le milieu est extrait par trois fois 400 ml d'acétate d'éthyle et la phase organique est-lavée à l'eau, séchée, concentrée pour conduire à 9,56 g d'un solide orangé. Le produit ainsi obtenu est mis en suspension dans 50 ml d'éther de pétrole, filtré puis lavé par 25 ml d'éther isopropylique, séché sous pression réduite (1mm Hg; 0,13 kPa) à 60°C pour conduire à 6,7 g d'un solide jaune orangé fondant à 160°C.

L'acide 3-(2-carboxyméthyl-phényl)propionique peut être préparé de la manière suivante : 39,6 g d'acide 3-(2-carboxyméthyl-phényl)acrylique avec 3 g de charbon palladié à 10% dans 400 ml d'acide acétique sont hydrogénés à une température voisine de 20°C sous une pression de 1,2 bar pendant 4 heures. Après filtration du milieu réactionnel, la phase organique est concentrée sous pression réduite (15 mm Hg; 2 kPa) à 40°C. Le solide blanc obtenu est mis en suspension dans 100 ml d'éther de pétrole puis filtré et séché sous pression réduite (1mm Hg; 0,13 kPa) à 20°C pour conduire à 39,3 g d'un solide blanc fondant à 138°C.

L'acide 3-(2-carboxyméthyl-phényl)acrylique peut être préparé de la manière suivante : 63,8 g d'acide 2-bromophényl acétique, 25,5 ml d'acide acrylique, 3,6 g de tri(2-tolyl)phosphine, 0,67 g d'acétate de palladium dans 211 ml de tributylamine sont chauffés à 100°C pendant 6 heures. Après refroidissement à une température voisine de 20°C le milieu réactionnel est versé sur 420ml d'eau et 80 ml d'acide chlorhydrique concentré. Le milieu est extrait par 3 fois 500 ml d'acétate d'éthyle; la phase organique est filtrée, lavée par 3 fois 500 ml d'eau puis agitée en présence de 800 ml d'eau et 35 g de carbonate de sodium à une température voisine de 20°C pendant 15 minutes. La phase aqueuse est ensuite acidifiée par 700 ml d'acide chlorhydrique 1N. Le précipité formé est filtré, lavé à l'eau puis séché sous pression réduite (1mm Hg; 0,13 kPa) à 60°C pour conduire à 39,6 g d'un solide blanc fondant à 190°C.

### EXEMPLE 3

Une solution de 0,93 g d'ester éthylique de l'acide (4,5-dihydro-4-oxo-10H-imidazo[1,2-a]indéno[1,2-e]pyrazin-9-yl)acétique, 20 ml de méthylamine en solution dans l'éthanol à 33% et 20 ml de méthanol sous atmosphère d'argon est agitée à température voisine de 20°C pendant 18 heures. Le milieu réactionnel est filtré. Le précipité obtenu est lavé avec successivement trois fois 40 ml d'éthanol, trois fois 40 ml de méthyl-tert-butyle éther et séché sous pression réduite (15 mm Hg; 2 kPa) à 20°C. Le solide obtenu est mis en suspension dans un mélange d'acétone (25 ml) et de méthanol (5 ml) et agité pendant 15 minutes puis filtré, lavé avec 10 ml d'acétone et séché sous pression réduite (1mm Hg; 0,13 kPa) à 60°C. On obtient 0,32 g de N-méthyl-2-(4,5-dihydro-4-oxo-10H-imidazo[1,2-a]indéno[1,2-e]pyrazin-9-yl)acétamide sous forme d'un solide blanc dont le point de fusion est supérieur à 260°C (Analyse C16H14N4O2; % Calculé C : 65,30; H : 4,79; N : 19,04; % Trouvé C : 65,00; H : 4,50; N : 18,80).

### EXEMPLE 4

Une solution de 0,56 g d'acide (4,5-dihydro-4-oxo-10H-imidazo[1,2-a]indéno[1,2-e]pyrazin-9-yl)acétique, 0,38 g d'hydrochlorure de 1-(3-diméthylaminopropyl)-3-éthylcarbodiimide, 0,27 g d'hydroxybenzotriazole et 1,1 ml d'aniline dans 20 ml de diméthylformamide est agitée sous atmosphère d'argon pendant 20 heures à une température voisine de 20°C. On ajoute au milieu réactionnel 20 ml d'acide chlorhydrique. Le précipité formé est filtré, lavé à l'eau et à l'éther isopropylique puis séché sous pression réduite (15 mm Hg; 2kPa) à 20°C. Le solide beige obtenu est mis en suspension dans une solution de 20 ml d'eau et 84 mg d'hydrogénocarbonate de sodium, puis filtré, lavé à l'eau. Le solide obtenu est remis en suspension dans 10 ml d'acide chlorhydrique 0,1N puis filtré, lavé à l'eau et séché sous pression réduite (1mm Hg; 0,13 kPa) à 60°C. On obtient 0,2 g de N-phényl-2-(4,5-dihydro-4-oxo-10H-imidazo[1,2-a]indéno[1,2-e]pyrazin-9-yl)acétamide sous forme d'un solide blanc fondant à une température supérieure à 260°C (Analyse C21H16N4O2; % Calculé C : 70,77; H : 4,53; N : 15,72; % trouvé C : 70,74; H : 4,39; N :15,64)

### EXEMPLE 5

Un mélange de 0,25 g de 9-cyano-5H,10H-imidazo[1,2-a]indéno[1,2-e] pyrazin-4-one, 10 ml d'acide acétique, 10 ml d'acide trifluoroacétique et 20 mg de charbon palladié à 10 % est hydrogéné en autoclave sous une pression de 50 bar pendant 20 heures. La suspension est filtrée, lavée avec de l'acide acétique. Le filtrat est concentré à sec sous pression réduite (15 mm Hg; 2Kpa) à 40°C. Le solide obtenu est mis en solution dans un mélange de 50 ml d'acide acétique et 10 ml d'acide chlorhydrique concentré, puis concentré à nouveau à sec sous pression réduite (15 mm Hg; 2kPa) à 40°C. Le résidu obtenu repris par 25 ml de méthanol est filtré puis lavé avec deux fois 25 ml d'éther isopropylique et séché sous pression réduite (1mm Hg; 0,13 kPa) à 20°C. On obtient 0,24 g de dichlorhydrate de 9-aminométhyl-5H,10H-imidazo[1,2-a]indéno[1,2-e]pyrazin-4-one sous forme de solide blanc fondant au-dessus de 260°C (Analyse C14H14Cl2N4O; % calculé C : 51,71; H : 4,34; N : 17,23; % trouvé C : 52,11; H : 4,53; N : 17,04).

La 9-cyano-5H,10H-imidazo[1,2-a]indéno[1,2-e]pyrazin-4-one peut être obtenue de la manière suivante : 1 g de 1-[(4-cyano-1-oxo-indan-2-yl)]imidazole-2-carboxamide est dissous dans 10 ml d'acide acétique et la solution est portée au reflux pendant 22 heures. Après refroidissement du milieu réactionnel, la suspension est filtrée, lavée par 5 ml d'acide acétique, de l'eau distillée et de l'éther éthylique. Le solide est séché sous pression réduite (15 mm Hg; 2kPa) à 30°C, on obtient ainsi 0,4 g de 9-cyano-5H,10H-imidazo[1,2-a]indéno[1,2-e]pyrazine-4-one fondant à une température supérieure à 260°C (Analyse C14 H8 N4 O1; 0,21H20 % calculé C : 67,74; H : 3,25; N : 22,57; O : 6,45; % trouvé C : 68,1; H : 3,3; N : 22,6; O : 6,7).

Le 1-[(4-cyano-1-oxo-indan-2-yl)]imidazole-2-carboxamide peut être obtenu de la manière suivante : une solution de 1,5 g de 1-[(4-cyano-1-oxo-indan-2-yl)]imidazole-2-carboxylate d'éthyle dans 130 ml de méthanol est maintenue saturée pendant une heure a une température voisine de 20°C par un courant de gaz ammoniac. Le milieu réactionnel est ensuite concentré à sec sous pression réduite (15 mm Hg; 2kPa) à 50°C. On obtient ainsi 1,2 g de 1-[(4-cyano-1-oxo-indan-2-yl)]imidazole-2-carboxamide fondant à 216°C.

Le 1-[(4-cyano-1-oxo-indan-2-yl)]imidazole-2-carboxylate d'éthyle peut être obtenu de la manière suivante : un mélange de 3,92 g d'imidazole-2-carboxylate d'éthyle et de 3,4 g de 2-bromo-4-cyano-1-indanone est chauffé à 130°C pendant 20 minutes, refroidi à 20°C et dissous dans 20 ml de dichlorométhane. Le milieu est ensuite concentré à sec sous pression réduite (15 mm Hg; 2kPa) à 40°C. Le résidu ainsi obtenu est purifié par flash-chromatographie sur colonne de silice, sous courant d'azote à moyenne pression (0,5 bar) avec un mélange dichlorométhane-méthanol (95/5 en volumes) comme éluant. On obtient ainsi 1,5 g de 1-[(4-cyano-1-oxo-indan-2-yl)]imidazole-2-carboxylate d'éthyle fondant à 159°C.

La 2-bromo-4-cyano-1-indanone peut être obtenue de la manière suivante : une solution de 6,9 g de 4-cyano-1-indanone et de 95 ml de chloroforme est refroidie à 5°C. Une solution de 7,04 g de brome et de 20 ml de chloroforme est alors ajoutée goutte à goutte en deux heures à une température comprise entre 0 et 5°C. Après avoir laissé le milieu réactionnel sous agitation pendant une heure en conservant la température d'introduction, on laisse revenir la solution à température ambiante et on agite encore une nuit. Le milieu réactionnel est ensuite concentré à sec sous pression réduite (15 mm Hg; 2kPa) à 40°C. Le résidu ainsi obtenu est purifié par flash-chromatographie sur colonne de silice, sous courant d'azote à moyenne pression (0,5 bar) avec un mélange éther éthylique-cyclohexane (20/80 en volumes) comme éluant. On obtient ainsi 3,43 g de 2-bromo-4-cyano-1-indanone fondant à 124°C.

La 4-cyano-1-indanone peut être préparée de la manière suivante : à une solution de 10 g de 4-bromo-1-indanone dans 94 ml de diméthylformamide, on ajoute 12,61 g de cyanure de cuivre, puis le milieu réactionnel est porté au reflux pendant 6 heures et agité à une température voisine de 20°C pendant une nuit. La solution est ensuite versée dans une solution aqueuse de cyanure de sodium (5%), agitée pendant 15 minutes et extraite par trois fois 500 ml d'acétate d'éthyle. Les phases organiques sont séchées sur sulfate de sodium et concentrées sous pression réduite (15 mm Hg; 2kPa) à 40°C. Le résidu ainsi obtenu est purifié par flash-chromatographie sur colonne de silice, sous courant d'azote à moyenne pression (0,5 bar) avec un mélange acétate d'éthyle-cyclohexane (20/80 en volumes) comme éluant. On obtient ainsi 5,9 g de 4-cyano-1-indanone fondant à 122°C.

La 4-bromo-1-indanone peut être préparée comme décrit par F.G. HOLLIMAN, F.G. MANNE et D.A. THORNTON, J. Chem. Soc., 9 (1960) .

### EXEMPLE 6

Un mélange de 1 g de 9-aminométhyl-5H,10H-imidazo[1,2-a]indéno[1,2-e]pyrazin-4-one et de 20 ml d'anhydride acétique est agité sous atmosphère d'argon 2 heures à une température voisine de 20°C. Le milieu réactionnel est filtré, l'insoluble est lavé avec successivement 10 ml d'anhydride acétique, deux fois 40 ml de méthyle-tert-butyle éther puis séché sous pression réduite (15 mm Hg; 2kPa) à 20°C. Le solide blanc est mis en suspension dans un mélange d'acétone (30 ml) et de méthanol (5 ml) puis filtré et séché sous pression réduite (1mm Hg; 0,13 kPa) à 60°C. On obtient 1,03 g de N-[(4,5-dihydro-4-oxo-10H-imidazo[1,2-a)indéno[1,2-e]pyrazin-9-yl)méthyl] acétamide sous forme d'un solide blanc dont le point de fusion est supérieur à 260°C (Analyse C16H14N4O2; % Calculé C : 65,30; H : 4,79; N : 19,04; % Trouvé C : 65,20; H : 5,10; N : 19,30).

### EXEMPLE 7

Une suspension de 0,25 g de 9-aminométhyl-5H,10H-imidazo[1,2-a]indéno[1,2-e]pyrazin-4-one, 0,49 g de 4-nitrophényl N-méthylcarbamate et 10 ml de diméthylformamide sous atmosphère d'argon est agitée 4 heures à une température voisine de 20°C. Le milieu réactionnel est filtré, lavé avec successivement 5 ml de diméthylformamide, deux fois 20 ml de méthyle-tert-butyle éther puis séché sous pression réduite (1mm Hg; 0,13 kPa) à 60°C. On obtient 0,2 g de 9-[(3-méthyluréido)méthyl]-5H,10H-imidazo[1,2-a]indéno[1,2-e]pyrazin-4-one sous forme d'un solide blanc dont le point de fusion est supérieur à 260°C (Analyse C16H15N5O2; % Calculé C : 62,13; H : 4,89; N : 22,64; % Trouvé C : 62,03; H : 4,90; N : 22,26).

### EXEMPLE 8

Un mélange de 24,5 g de 1-(5-éthoxycarbonylméthyl-1-oxo-indan-2-yl)imidazole-2-carboxylate d'éthyle et de 236,5 g d'acétate d'ammonium dans 1 litre d'acide acétique est porté au reflux pendant 8 heures. Après évaporation de l'acide acétique sous pression réduite, on additionne 1 litre d'eau distillée au résidu. L'insoluble est filtré, lavé à l'eau puis séché à l'air. On obtient ainsi 10,6 g d'un solide gris. La recristallisation dans 175 ml d'éthanol d'un échantillon de 0,8 g de ce solide conduit, après séchage à 60°C sous pression réduite (1 mbar), à 0,65 g de [4,5-dihydro-4-oxo-10H-imidazo[1,2-a]indéno[1,2-e]pyrazin-8-yl]acétate d'éthyle sous forme d'un solide marron de point de fusion supérieur à 260°C (Analyse % calculé C : 66,01; H : 4,89; N : 13,58; O : 15,52; % trouvé C : 65,8; H : 5,1; N : 13,2; O : 15,5).

Le 1-(5-éthoxycarbonylméthyl-1-oxo-indan-2-yl)imidazole-2-carboxylate d'éthyle peut être synthétisé comme suit : une suspension sous courant d'azote de 47 g d'imidazole-2-carboxylate d'éthyle et de 50 g d'un mélange 60/40 molaire de 2-bromo-5-éthoxycarbonylméthylindan-1-one et de 2,2-dibromo-5-éthoxycarbonylméthylindan-1-one dans 1,5 litre de toluène est chauffée au reflux pendant 26 heures. Le solvant est évaporé sous pression réduite et le brut réactionnel est repris dans un mélange de 1,5 litre de dichlorométhane et de 500 ml d'eau distillée. La phase aqueuse est éliminée et la phase organique est lavée successivement trois fois avec 500 ml d'eau distillée, une fois avec 500 ml de solution saturée en carbonate de potassium et une fois avec 500 ml d'eau distillée, puis elle est séchée sur sulfate de magnésium et évaporée sous pression réduite. Après purification du résidu par une chromatographie flash sur colonne de silice (éluant : dichlorométhane-acétate d'éthyle (1/1 en volumes)), on obtient 21,3 g de 1-(5-éthoxycarbonylméthyl-1-oxo-indan-2-yl)imidazole-2-carboxylate d'éthyle sous forme d'un solide marron pur à 70 % et utilisé tel quel dans les opérations ultérieures (Rf = 0,35, chromatographie sur couche mince de gel de silice, éluant : acétate d'éthyle-dichorométhane (1/1 en volumes)) (Spectre RMN ¹H dans CDCl₃, T=300K, δ en ppm : 1,20 (3H, t, J=6Hz, CH₃), 1,28 (3H, t, J=6Hz, CH₃), 3,20 et 3,76 (1H chacun, respectivement dd, J=6 et 16Hz, et dd, J=8 et 16Hz, CH₂), 3,67 (2H, s, CH₂), 4,10 (2H, q, J=6Hz, OCH₂), 4,23 (2H, q, J=6Hz, OCH₂), 5,81 (1H, t, J=6Hz, CH), 6,99 (1H, s, CH), 7,13 (1H, s, CH), 7,32 (1H, d, J=7Hz, CH), 7,37 (1H, s, CH), 7,71 (1H, d, J=7Hz, CH)).

L'imidazole-2-carboxylate d'éthyle peut être obtenu comme décrit dans le brevet US 3600399.

La 2-bromo-5-éthoxycarbonylméthylindan-1-one peut être préparée de la façon suivante : à une solution de 38 g de 5-éthoxycarbonylméthylindan-1-one dans un mélange de 175 ml de dichlorométhane et de 350 ml d'éthanol absolu maintenue à une température comprise entre 5 et 10°C, on ajoute goutte à goutte 8,9 ml de brome en solution dans 87 ml de dichlorométhane. La réaction est poursuivie 1 heure et 30 minutes à une température comprise entre 5 et 10°C, puis 15 minutes à une température voisine de 20°C. Le milieu réactionnel est alors évaporé et le résidu est repris par 500 ml de dichlorométhane. La phase organique est lavée successivement trois fois avec 200 ml d'eau distillée, une fois avec 200 ml de solution saturée d'hydrogénocarbonate de sodium et deux fois avec 200 ml d'eau distillée, puis séchée sur sulfate de magnésium et évaporée. On obtient ainsi 50,1 g d'un mélange de 2-bromo-5-éthoxycarbonylméthylindan-1-one (Rf = 0,41, chromatographie sur couche mince de gel de silice, éluant : dichlorométhane) et de 2,5-dibromo-5-éthoxycarbonylméthylindan-1-one (Rf = 0,59, chromatographie sur couche mince de gel de silice, éluant : dichloro-méthane) utilisé tel quel pour la suite de la synthèse (rapport molaire composé monobromé-composé dibromé : 60/40) (Spectre RMN ¹H de la 2-bromo-5-éthoxycarbonyl-méthylindan-1-one dans CDCl₃, T=300K, δ en ppm : 1,25 (3H, t, J=6Hz, CH₃), 3,39 et 3,80 (1H chacun, respectivement dd, J=4 et 16Hz, et dd, J=7 et 16Hz, CH₂), 3,72 (2H, m, CH₂), 4,06 (2H, q, J=6Hz, OCH₂), 4,65 (1H, dd, J=4 et 7 Hz, CH), entre 7,30 et 7,45 (2H, m, H arom.), 7,79 (1H, d, J=7Hz, H arom.)).

La 5-éthoxycarbonylméthylindan-1-one peut être préparée comme suit : une solution de 39,9 g d'acide (1-oxo-indan-5-yl)acétique et de 79,8 ml d'acide méthanesulfonique dans 720 ml d'éthanol absolu est chauffée 2 heures au reflux. L'éthanol est évaporé et le résidu est repris par 500 ml de dichlorométhane. La phase organique ainsi obtenue est lavée successivement quatre fois avec 200 ml d'eau distillée, une fois avec 200 ml de solution aqueuse saturée d'hydrogénocarbonate de sodium et une fois avec 200 ml d'eau distillée, séchée sur sulfate de magnésium et évaporée. On obtient ainsi 41,5 g de (1-oxo-indan-5-yl)acétate d'éthyle sous forme d'un solide crème (Spectre RMN ¹H dans CDCl₃, T=300K, δ en ppm : 1,25 (3H, t, J=6Hz, CH₃), 2,66 (2H, m, COCH₂), 3,12 (2H, t, J=5Hz, CH₂), 3,70 (2H, s, CH₂), 4,16 (2H, t, J=6Hz, OCH₂), 7,26 (1H, d, J=7Hz, CH arom.), 7,40 (1H, s, CH arom.), 7,69 (1H, d, J=7Hz, CH arom.)).

L'acide (1-oxo-indan-5-yl)acétique peut être obtenu de la façon suivante : une solution de 50,9 g de [4-(3-chloro-1-oxopropyl)phényl]acétate d'éthyle dans 950 ml d'acide sulfurique à 95 % est chauffé 1 heure à 100°C. Le milieu réactionnel est refroidi à une température voisine de 20°C puis versé dans 4 litres d'eau glacée. Le mélange est extrait trois fois par 1,5 litre de dichlorométhane. La phase organique est lavée à trois reprises par 1 litre d'eau puis séchée sur sulfate de magnésium et évaporée sous pression réduite. On obtient ainsi 22,2 g d'acide (1-oxo-indan-5-yl)acétique sous forme d'un solide crème fondant à 141°C (Spectre RMN ¹H dans CDCl₃, T=300K, δ en ppm : 2,70 (2H, m, COCH₂), 3,12 (2H, t, J=5Hz, CH₂), 3,68 (2H, s, CH₂), 7,27 (1H, d, J=7Hz, CH arom.), 7,45 (1H, s, CH arom.), 7,76 (1H, d, J=7Hz, CH arom.)).

Le [4-(3-chloro-1-oxopropyl)phényl]acétate d'éthyle peut être synthétisé de la manière suivante : à une suspension de 1,5 kg de chlorure d'aluminium dans 3,5 litres de dichlorométhane, on ajoute goutte à goutte, de façon à ce que la température du milieu réactionnel ne dépasse pas 25°C, une solution de 600 g de phénylacétate d'éthyle et de 384 ml de chlorure de 3-chloropropionyle dans 1 litre de dichlorométhane. Après 24 heures de réaction à une température voisine de 20°C, le mélange réactionnel est versé sur 8 litres d'eau glacée. La phase organique est décantée et la phase aqueuse est extraite deux fois avec 2 litres de dichlorométhane. Les phases organiques sont rassemblées, lavées quatre fois avec 2 litres d'eau distillée, séchées sur sulfate de magnésium et évaporées. Après filtration du résidu sur une colonne de silice (éluant : dichlorométhane), on obtient 605 g d'une huile qui cristallise. Après lavage à l'éthanol absolu et séchage, on obtient 82 g de [4-(3-chloro-1-oxopropyl)phényl]acétate d'éthyle sous forme d'un solide blanc fondant à 69°C (Spectre RMN ¹H dans CDCl₃, T=300K, δ en ppm : 1,26 (3H, t, J=6Hz, CH₃), 3,43 (2H, t, J=6Hz, CH₂Cl), 3,68 (2H, s, CH₂), 3,92 (2H, t, J=6Hz, CH₂), 4,16 (2H, q, J=6Hz, OCH₂), 7,40 (2H, d, J=7Hz, CH arom.), 7,92 (2H, d, J=7Hz, CH arom.)).

### EXEMPLE 9

Une suspension de 0,5 g de [4,5-dihydro-4-oxo-10H-imidazo[1,2-a]indéno [1,2-e]pyrazin-8-yl]acétate d'éthyle dans 50 ml d'acide chlorhydrique 6 N est chauffée au reflux pendant 2 heures. Le mélange est additionné de 25 ml de 1,4-dioxanne et le reflux est prolongé pendant 4 heures. Après retour à une température voisine de 20°C, le solide formé est filtré, lavé avec 5 ml de 1,4-dioxanne puis séché sous pression réduite (1 mbar) à 60°C. On obtient ainsi 0,47 g d'acide [4,5-dihydro-4-oxo-10H-imidazo[1,2-a]indéno[1,2-e]pyrazin-8-yl]acétique sous forme d'un chlorhydrate monohydrate dont le point de fusion est supérieur à 260°C (Analyse % calculé C : 56,7; H : 3,81; Cl : 11,16; N : 13,23; % trouvé C : 56,7; H : 3,9; Cl : 10,9; N : 12,9).

### EXEMPLE 10

Un mélange de 0,618 g de [4,5-dihydro-4-oxo-10H-imidazo[1,2-a]indéno[1,2-e]pyrazin-8-yl]acétate d'éthyle et 10 ml de benzylamine est chauffé à 140°C pendant 17 heures sous atmosphère d'azote. Après retour à température ambiante, le milieu réactionnel est additionné de 50 ml de dichlorométhane. Le solide formé est filtré, lavé avec du dichlorométhane puis recristallisé dans 25 ml de diméthylformamide. On obtient ainsi, après séchage sous pression réduite (1 mbar) à 60°C, 0,65 g de N-benzyl-2-(4,5-dihydro-4-oxo-10H-imi-cazo[1,2-a]indéno[1,2-e]pyrazin-8-yl)acétamide de point de fusion supérieur à 260°C (Analyse % calculé C : 71,34; H : 4,90; N : 15,13; O : 8,64; % calculé C : 71,1; H : 5,0; N : 15,2; O : 9,1).

### EXEMPLE 11

Une suspension sous azote de 2,9 g de chlorhydrate d'acide [4,5-dihydro-4-oxo-10H-imidazo[1,2-a]indéno[1,2-e]pyrazin-8-yl]acétique et 108 ml de diméthylformamide est additionnée de 1,21 ml de triéthylamine. Après 30 minutes d'agitation à une température voisine de 20°C, on ajoute 3,5 g de 1,1'-carbonyldiimidazole au mélange réactionnel et l'agitation est poursuivie 2 heures à une température voisine de 20°C. Le milieu réactionnel est alors refroidi à -10°C et on fait passer un courant d'ammoniac gazeux dans le mélange pendant 15 minutes. Après retour à une température voisine de 20°C, le milieu réactionnel est concentré à sec sous pression réduite. Le résidu d'évaporation est repris avec 100 ml d'eau distillée et le solide en suspension est filtré, lavé avec de l'eau puis dissous dans 125 ml de diméthylsulfoxyde. L'addition de 250 ml d'acétone à la solution provoque l'apparition d'un précipité qui est collecté par filtration, lavé à l'acétone et séché sous pression réduite (1 mbar) à 60°C. On obtient ainsi 1,1 g de [4,5-dihydro-4-oxo-10H-imidazo[1,2-a]indéno[1,2-e]pyrazin-8-yl]acétamide sous forme d'un solide beige de point de fusion supérieur à 260°C (Analyse % calculé C : 64,28; H : 4,32; N : 19,99; O : 11,42; % trouvé C : 63,9; H : 4,3; N : 19,8; O : 10,9).

### EXEMPLE 12

Un mélange de 1,8 g de 1-(5-N-méthylcarboxamidométhyl-1-oxo-indan-2-yl)imidazole-2-carboxylate d'éthyle et de 20,9 g d'acétate d'ammonium dans 88 ml d'acide acétique est porté au reflux pendant 2 heures. Le milieu réactionnel est concentré à sec sous pression réduite. Le résidu d'évaporation est repris par 100 ml d'eau distillée. Le solide en suspension est filtré, lavé à l'eau distillée puis recristallisé dans 25 ml d'acide acétique. Après séchage sous pression réduite (1 mbar) à 60°C on obtient 0,75 g de N-méthyl-[4,5-dihydro-4-oxo-10H-imidazo[1,2-a]indéno[1,2-e]pyrazin-8-yl]acétamide sous forme d'un solide beige de point de fusion supérieur à 260°C (Analyse % calculé C : 65,30; H : 4,79; N : 19,04; O :10,87; % trouvé C : 65,5; H : 4,8; N : 18,9; O : 11,2).

Le 1-(5-N-méthylcarboxamidométhyl-1-oxo-indan-2-yl)imidazole-2-carboxylate d'éthyle peut être obtenu de la façon suivante : un mélange de 1,44 g d'imidazole-2-carboxylate d'éthyle, de 3,55 g de carbonate de potassium et de 68 mg d'éther couronne 18C-6 dans 130 ml de diméthylformamide est chauffé 2 heures à 85°C puis refroidi à 20°C. Le milieu réactionnel est alors additionné d'une solution de 2,9 g de N-méthyl(2-bromo-1-oxo-indan-5-yl)acétamide. La réaction est poursuivie 16 heures à une température voisine de 20°C puis 1 heure à 80°C. Le diméthylformamide est éliminé sous pression réduite et le résidu est repris par 100 ml d'eau distillée et 200 ml d'acétate d'éthyle. La phase aqueuse est extraite quatre fois avec 200 ml d'acétate d'éthyle. Les phases organiques sont rassemblées, lavées quatre fois avec 100 ml d'eau, séchées sur sulfate de magnésium et évaporées. On obtient ainsi 1,4 g de 1-(5-N-méthylcarboxamidométhyl-1-oxo-indan-2-yl)imidazole-2-carboxylate d'éthyle sous forme d'un solide brun (Rf = 0,39, chromatographie sur couche mince de gel de silice, éluant : acétate d'éthyle-méthanol (8/2 en volumes)).

Le N-méthyl(2-bromo-1-oxo-indan-5-yl)acétamide peut être synthétisé comme suit : une solution de 9 g de N-méthyl(1-oxo-indan-5-yl)acétamide dans 200 ml de dichlorométhane et 500 ml d'éthanol absolu est refroidie à une température comprise entre 0 et 5°C. On ajoute goutte à goutte au milieu réactionnel, de façon à ce que la température ne dépasse pas 5°C, 7,1 g de brome dilué dans 100 ml de dichlorométhane. Après 1 heure et 30 minutes d'agitation à une température voisine de 20°C, le solvant est évaporé sous pression réduite. Le brut réactionnel est purifié par une chromatographie flash sur silice (éluant : dichlorométhane-méthanol (96/4 en volumes)). On obtient ainsi 7,8 g de N-méthyl(2-bromo-1-oxo-indan-5-yl)acétamide sous forme d'un solide blanc fondant à 184°C (Spectre RMN ¹H dans DMSO-d6, T=300K, δ en ppm : 2,60 (3H, d, J=5Hz, NCH₃), 3,35 et 3,90 (1H chacun, respectivement :m, et: dd, J=6 et 16Hz, CH₂), 3,56 (2H, d, COCH₂), 5,01 (1H, dd, J=2 et 6Hz, CHBr), 7,40 (1H, d, J=7Hz, CH arom.), 7,45 (1H, s, CH arom.), 7,70 (1H, d, J=7Hz, CH arom.), 8,05 (1H, s large, NH)).

Le N-méthyl(1-oxo-indan-5-yl)acétamide peut être obtenu de la manière suivante : une solution de 12,5 g d'acide (1-oxoindan-5-yl)acétique dans 250 ml de tétrahydrofuranne maintenue à 0°C est additionnée par portion de 16 g de N,N'-carbonyldiimidazole. Après 1 heure de réaction à une température voisine de 20°C, la solution est refroidie à -10°C et on fait passer un courant de monométhylamine dans le mélange pendant 5 minutes. Après retour à une température voisine de 20°C, le milieu réactionnel est évaporé sous pression réduite. Le résidu est repris par 1 litre de dichlorométhane. La phase organique est lavée trois fois avec 500 ml d'eau distillée, séchée sur sulfate de magnésium puis évaporée. Le solide brun résultant est purifié par une chromatographie flash sur silice (éluant dichlorométhane-méthanol (96/4 en volumes)). On obtient ainsi 9 g de N-méthyl(1-oxo-indan-5-yl)acétamide sous forme d'un solide blanc fondant à 169°C (Spectre RMN ¹H dans CDCl₃, T=300K, δ en ppm : 2,69 (2H, m, COCH₂), 2,80 (3H, d, NCH₃), 3,14 (2H, t, J=5Hz, CH₂), 3,65 (2H, s, CH₂), 5,47 (1H, s large, NH), 7,26 (1H, d, J=7Hz, CH arom.), 7,42 (1H, s, CH arom.), 7,74 (1H, d, J=7Hz, CH arom.)).

### EXEMPLE 13

Une suspension sous azote de 1 g de 8-N-méthylcarboxamidométhyl-4,5-dihydro-4-oxo-10H-imidazo[1,2-a]indéno[1,2-e]pyrazin-2-carboxylate d'éthyle, 97 ml de 1,4-dioxanne et 24 ml d'eau distillée est solubilisée par l'addition de 7,26 ml de soude 1 N. Le mélange est agité pendant 18 heures à une température voisine de 20°C. Le milieu réactionnel est acidifié avec de l'acide chlorhydrique 1 N jusqu'à pH 1. Le solide obtenu est filtré, lavé successivement avec du 1,4-dioxanne, de l'eau distillée et de l'éther éthylique. Après séchage sous pression réduite (1 mbar) à 60°C on obtient 0,75 g d'acide 8-N-méthylcarboxamidométhyl-4,5-dihydro-4-oxo-10H-imidazo[1,2-a]indéno [1,2-e]pyrazin-2-carboxylique sous forme d'un solide jaune de point de fusion supérieur à 260°C (Analyse % calculé C : 60,35; H : 4,17; N : 16,56; % trouvé C : 60,4; H : 4,1; N : 16,6).

Le 8-N-méthylcarboxamidométhyl-4,5-dihydro-4-oxo-10H-imidazo[1,2-a]indéno[1,2-e]pyrazin-2-carboxylate d'éthyle peut être synthétisé de la façon suivante : un mélange de 3,4 g de 1-[5-(N-méthylcarboxamidométhyl)-1-oxo-indan-2-yl]imidazole-2,4-dicarboxylate de diéthyle et 33,2 g d'acétate d'ammonium dans 137 ml d'acide acétique glacial est chauffé au reflux pendant 16 heures. Après élimination du solvant par évaporation sous pression réduite, on ajoute au résidu 100 ml d'eau distillée. Le précipité formé est collecté par filtration puis lavé avec 50 ml d'eau distillée et avec 50 ml d'acétone. On obtient ainsi 2 g de 4,5-dihydro-8-N-méthylcarboxamidométhyl-4-oxo-10H-imidazo[1,2-a]indéno[1,2-e]pyrazin-2-carboxylate d'éthyle sous forme d'un solide beige fondant au dessus de 260°C.

Le 1-[5-(N-méthylcarboxamidométhyl)-1-oxo-indan-2-yl]imidazole-2,4-dicar-boxylate de diéthyle peut être préparé de la façon suivante : un mélange de 2,6 g d'imidazole-2,4-dicarboxylate de diéthyle, 4,2 g de carbonate de potassium et 79 mg d'éther couronne 18C-6 dans 150 ml de diméthylformamide est chauffé 2 heures à 85°C puis refroidi à une température voisine de 20°C. Le milieu réactionnel est alors additionné d'une solution de 3,4 g de N-méthyl(2-bromo-1-oxo-indan-5-yl)acétamide dans 75 ml de diméthylformamide. La réaction est poursuivie 16 heures à une température voisine de 20°C. Le diméthylformamide est éliminé sous pression réduite et le résidu est repris par 150 ml d'eau distillée et 200 ml d'acétate d'éthyle. La phase organique est décantée et la phase aqueuse est extraite quatre fois avec 200 ml d'acétate d'éthyle. Les phases organiques sont rassemblées, lavées trois fois avec 100 ml d'eau distillée, séchées sur sulfate de magnésium et évaporées. On obtient ainsi 2,9 g de 1-[5-(N-méthylcarboxamidométhyl)-1-oxo-indan-2-yl]imidazole-2,4-dicarboxylate de diéthyle sous forme d'un solide crème (Spectre RMN ¹H dans DMSO-d6, T=300K, δ en ppm 1,25 (3H, t, J=6Hz, CH₃), 1,37 (3H, t, J=6Hz, CH₃), 2,66 (3H, d, J=4Hz, NCH₃), 3,49 et 3,80 (1H chacun, respectivement dd, J=6 et 16Hz, et dd, J=8 et 16Hz, CH₂), 3,62 (2H, s, CH₂), 4,20 (2H, q, J=6Hz, OCH₂), 4,37 (2H, q, J=6Hz, OCH₂), 5,90 (1H, dd, J=6 et 8Hz, CH), 7,47 (1H, d, J=7Hz, CH), 7,55 (1H, s, CH), 7,76 (1H, d, J=7Hz, CH), 8,17 (1H, s, NH), 8,40 (1H, s, CH)).

L'imidazole-2,4-dicarboxylate de diéthyle peut être synthétisé comme décrit par P.S. BRANCO et coll., Tetrahedron, 48 (30), 6335 (1992).

### EXEMPLE 14

Un mélange de 0,5 g de 8-(N-méthylcarboxamidométhyl)-4,5-dihydro-4-oxo-10H-imidazo[1,2-a]indéno[1,2-e]pyrazin-2-carboxylate d'éthyle, 25 ml de dioxanne et 50 ml d'acide chlorhydrique 6 N est chauffé au reflux 5 heures. Après refroidissement du milieu réactionnel, le précipité formé est filtré, lavé avec 50 ml d'eau distillée et 10 ml de dioxanne. On obtient ainsi 320 mg de chlorhydrate d'acide 8-carboxyméthyl-4,5-dihydro-4-oxo-10H-imidazo[1,2-a]indéno[1,2-e]pyrazin-2-carboxylique (Spectre RMN ¹H dans DMSO-d6, T=300K, δ en ppm : 3,62 (2H, s, CH₂CO), 3,97 (2H, s, CH₂), 7,25 (1H, d, J=7Hz, H arom.), 7,45 (1H, s, CH arom.), 7,78 (1H, d, J=7Hz, CH arom.), 8,48 (1H, s, CH arom.),12,5 (1H, s, NH)).

### EXEMPLE 15

Un mélange de 1 g de 8-aminométhyl-5H,10H-imidazo[1,2-a]indéno[1,2-e]pyrazin-4-one et 1,8 g de 4-nitrophényl-N-méthylcarbamate dans 40 ml de diméthylformamide est agité pendant 18 heures à une température voisine de 20°C. Le milieu réactionnel est filtré et lavé avec 5 ml de diméthylformamide. Le solide est repris dans 25 ml de diméthylformamide et la suspension est chauffée à 100°C pendant 30 minutes. L'insoluble est filtré, lavé avec 5 ml de diméthylformamide puis avec 20 ml d'acétone. Après séchage sous pression réduite (1 mbar) à 60°C, on obtient 1,2 g de 8-(3-méthyluréido)méthyl-5H,10H-imidazo[1,2-a]indéno[1,2-e]pyrazin-4-one sous forme d'un solide jaune dont le point de fusion est supérieur à 260°C (Analyse % calculé C : 62,13; H : 4,89; N : 22,64; % trouvé C : 62,0; H : 4,5; N : 22,6).

Le 4-nitrophényl-N-méthylcarbamate peut être préparé comme décrit par T. KONAKAHARA et coll., Synthesis, 103 (1993).

Le 8-aminométhyl-5H,10H-imidazo[1,2-a]indéno[1,2-e]pyrazin-4-one peut être préparé de la manière suivante : un mélange de 1 g de 8-phtalimidométhyl-5H,10H-imidazo[1,2-a]indéno[1,2-e]pyrazin-4-one, 50 ml d'éthanol absolu, 20 ml d'eau distillée et 1,3 ml d'hydrate d'hydrazine est chauffé au reflux pendant 18 heures. Le solide est filtré, lavé avec de l'eau distillée. Il est ensuite agité dans 15 ml d'acide chlorhydrique 2 N pendant 1 heure et 30 minutes à une température voisine de 20°C, filtré, rincé à l'eau puis à l'acétone. Le produit est repris avec 50 ml de diméthylformamide, agité 1 heure et 30 minutes à 120°C et la suspension est filtrée à chaud. L'insoluble est lavé avec du diméthylformamide puis de l'acétone et séché à 60°C sous presion réduite (1 mbar). On obtient ainsi 0,58 g de chlorhydrate de 8-aminométhyl-5H,10H-imidazo[1,2-a]indéno[1,2-e]pyrazin-4-one sous forme d'un solide beige de point de fusion supérieur à 260°C (Analyse % calculé C : 58,24; H : 4,54; Cl : 12,28; N : 19,40; % trouvé C : 58,3; H : 4,8; Cl : 12,2; N : 19,2).

La 8-phtalimidométhyl-5H,10H-imidazo[1,2-a]indéno[1,2-e]pyrazin-4-one peut être obtenue de la façon suivante : un mélange de 17 g de 1-(5-phtalimidométhyl-1oxo-indan-2yl)imidazole-2-carboxylate d'éthyle et 153 g d'acétate d'ammonium dans 355 ml d'acide acétique glacial est chauffé au reflux pendant 16 heures. Le milieu réactionnel est refroidi à une température voisine de 20°C. Le précipité est filtré sur verre fritté, rincé à l'eau distillée jusqu'à pH neutre puis lavé à l'acétone. Après séchage sous vide (1 mbar) à 80°C, on obtient 10 g de 8-phtalimidométhyl-5H,10H-imidazo[1,2-a]indéno[1,2-e]pyrazin-4-one sous forme d'un solide marron (Spectre RMN ¹H dans DMSO-d6, T=300K, δ en ppm : 4,00 (2H, s, CH₂), 4,83 (2H, s, NCH₂), 7,47 (1H, d, J=7Hz, CH arom.), 7,55 (2H, d, J=7Hz, CH arom.), 7,80 (1H, d, J=7Hz, CH arom.), entre 7,85 et 8,00 (4H, m, H phtal.), 12,30 (1H, s large, NH)).

Le 1-(5-phtalimidométhyl-1oxo-indan-2yl)imidazole-2-carboxylate d'éthyle peut être synthétisé de la manière suivante : une suspension de 28,3 g de 2-bromo-5-phtalimidométhylindan-1-one et 21,4 g d'imidazole-2-carboxylate d'éthyle dans 700 ml de toluène est chauffée au reflux pendant 24 heures. La solution est évaporée sous pression réduite et le résidu est repris avec 800 ml de solution saturée de carbonate de potassium et 800 ml de dichlorométhane. La phase organique est décantée et la phase aqueuse est extraite deux fois avec 500 ml de dichlorométhane. Les phases organiques sont rassemblées, lavées à six reprises avec 800 ml d'eau distillée, séchées sur sulfate de magnésium et évaporées sous pression réduite. Après purification du résidu par une chromatographie flash sur colonne de silice (éluant : dichlorométhane-méthanol (99/1 en volumes)), on obtient 17,5 g de 1-(5-phtalimidométhyl-1oxo-indan-2yl)imidazole-2-carboxylate d'éthyle sous forme d'un solide beige (Spectre RMN ¹H dans DMSO-d6 et quelquesq gouttes de CD₃CO₂D, T=300K, δ en ppm : 1,15 (3H, t, J=7Hz, CH₃), 3,35 et 3,70 (1H chacun, respectivement dd, J=6 et 16Hz, et dd, J=9 et 16Hz, CH₂), 4,10 (2H, q, J=7Hz, OCH₂), 4,93 (2H, s, NCH₂), 5,79 (1H, dd, J=6 et 9Hz, NCH), 7,20 (1H, s, CH), 7,48 (1H, d, J=7Hz, CH arom.), 7,53 (1H, s, CH arom.), 7,56 (1H, s, CH arom.), 7,72 (1H, d, J=7Hz, CH arom.), entre 7,80 et 7,95 (4H, m, H phtal.)).

La 2-bromo-5-phtalimidométhylindan-1-one peut être préparée comme suit : une solution de 22,6 g de 5-phtalimidométhylindan-1-one dans 150 ml de dichlorométhane maintenue à une température comprise entre 0 et 5°C est additionnée de 15,5 g de brome dilué dans 75 ml de dichlorométhane. Après 16 heures à une température voisine de 20°C, le milieu réactionnel est versé sur 300 ml d'eau distillée. La phase organique est décantée et la phase aqueuse est extraite trois fois avec 100 ml de dichlorométhane. Les phases organiques sont rassemblées, lavées deux fois avec 100 ml d'eau distillée, séchées sur sulfate de magnésium et évaporées sous pression réduite. On obtient ainsi 28,3 g de 2-bromo-5-phtalimidométhylindan-1-one sous forme d'un solide beige (Spectre RMN ¹H dans DMSO-d6, T=300K, δ en ppm : 3,30 et 3,87 (1H chacun, respectivement dd, J=4 et 16Hz, et dd, J=7 et 16Hz, CH₂), 4,90 (2H, s, NCH₂), 4,99 (1H, dd, J=4 et 7 Hz, CHBr), 7,46 (1H, d, J=7Hz, CH arom.), 7,51 (1H, s, CH arom.), 7,75 (1H, d, J=7Hz, CH arom.), entre 7,85 et 8,00 (4H, m, H phtal.)).

La 5-phtalimidométhylindan-1-one peut être obtenue comme décrit ci-après : une suspension de 43,1 g d'acide 3-[3-(phtalimidométhyl)phényl]propanoïque dans 900 ml de dichlorométhane est additionnée de 20,4 ml de chlorure de thionyle et de quelques gouttes de diméthylformamide. Le mélange est agité 2 heures à 30°C puis 16 heures à une température voisine de 20°C. L'évaporation du solvant conduit à l'obtention de 46,3 g d'une huile jaune. Cette huile est mise en solution dans 400 ml de 1,2-dichloroéthane et additionnée goutte à goutte à une suspension de 56 g de chlorure d'aluminium sous azote à une température voisine de 20°C. Après 24 heures de réaction à une température voisine de 20°C, le milieu réactionnel est versé sur 400 g de glace. Le mélange est extrait trois fois avec 300 ml d'éther éthylique. Les phases organiques sont rassemblées, lavées avec 100 ml de solution saturée de carbonate de sodium et avec 200 ml d'eau distillée puis séchées sur sulfate de magnésium et évaporées. Après purification du solide résultant par chromatographie flash sur colonne de silice (éluant dichlorométhane-acétate d'éthyle (98/2 en volumes)), on obtient 20,4 g de 5-phtalimidométhylindan-1-one sous forme d'un solide beige (Spectre RMN ¹H dans DMSO-d6, T=300K, δ en ppm : 2,61 (2H, m, COCH₂), 3,08 (2H, t, J=5Hz, CH₂), 4,90 (2H, s, CH₂), 7,38 (1H, d, J=7Hz, CH arom.), 7,51 (1H, s, CH arom.), 7,61 (1H, d, J=7Hz, CH arom.), entre 7,85 et 8,00 (4H, m, H phtal.)).

L'acide 3-[3-(phtalimidométhyl)phényl]propanoïque peut être synthétisé de la façon suivante : une solution de 48,7 g d'acide 3-(phtalimidométhyl) cinnamique dans 730 ml de diméthylformamide et 230 ml d'éthanol absolu est additionnée de 4,9 g de palladium sur charbon à 10 %, et placée sous hydrogène à une pression voisine de 1 bar et à une température voisine de 20°C. Après 4 heures de réaction, le milieu réactionnel est filtré sur célite et le filtrat est évaporé sous pression réduite. On obtient ainsi 43,1 g d'acide 3-[3-(phtalimidométhyl)phényl]propanoïque sous forme d'un solide blanc (Spectre RMN ¹H dans DMSO-d6, T=300K, δ en ppm : 2,52 (2H, m, COCH₂), 2,80 (2H, t, J=7Hz, CH₂), 4,77 (2H, s, NCH₂), entre 7,10 et 7,35 (4H, m, H arom.), entre 7,85 et 8,00 (4H, m, H phtal.), 12,20 (1H, s, COOH)).

L'acide 3-(phtalimidométhyl)cinnamique peut être préparé de la manière suivante : dans un ballon sous courant d'azote sont introduits successivement 114,4 g de 1-bromo-3-(phtalimidométhyl)phényle, 7,5 g de tri-o-tolylphosphine, 0,8 g d'acétate de palladium, 200 ml de tributylamine, puis goutte à goutte 30 ml d'acide acrylique. Le mélange est chauffé 2 heures et 30 minutes à 110°C. On observe pendant ce laps de temps une augmentation de la température du milieu réactionnel qui atteint 140°C, suivie d'une stabilisation à 110°C. Après retour à une température voisine de 20°C, le milieu réactionnel est additionné de 300 ml de diméthylformamide, 300 ml de méthanol et de 11 g de noir animal. La suspension est chauffée 5 minutes à 80°C, filtrée sur célite et le filtrat est évaporé sous pression réduite. Le résidu est repris par 900 ml de méthanol. Le mélange est porté au reflux pendant 15 minutes puis refroidi à une température voisine de 20°C et filtré. On obtient ainsi 88,6 g d'acide acide 3-(phtalimidométhyl)cinnamique sous forme d'un solide blanc (Spectre RMN ¹H dans DMSO-d6, T=300K, δ en ppm : 4,80 (2H, s, NCH₂), 6,55 (1H, d, J=15 Hz, =CH), 7,4 (2H, m, CH arom.), 7,60 (1H, d, J=15 Hz, =CH), 7,65 (2H, m, CH arom.), entre 7,85 et 8,00 (4H, m, H phtal.)).

Le 1-bromo-3-(phtalimidométhyl)phényle peut être obtenu comme suit : une solution de 150 g de bromure de 3-bromobenzyle dans 1,5 litre de diméthylformamide additionnée de 155,6 g de phtalimidate de potassium est chauffée 16 heures à 60°C. Après retour à une température voisine de 20°C, le mélange réactionnel est filtré sur verre fritté et le filtrat est évaporé. Le résidu est repris par 400 ml de méthanol et la suspension résultante est agitée 1 heure à une température voisine de 20°C. On isole par filtration 163,9 g de 1-bromo-3-(phtalimidométhyl)phényle sous forme d'un solide blanc (Spectre RMN ¹H dans DMSO-d6, T=300K, δ en ppm : 4,80 (2H, s, NCH₂), entre 7,25 et 7,35 (2H, m, 2H arom.), 7,49 (1H, m, CH arom.), 7,55 (1H, s, CH arom.), entre 7,85 et 8,00 (4H, m, H phtal.)).

### EXEMPLE 16

Une suspension de 0,8 g de chlorhydrate de 8-aminométhyl-5H,10H-imidazo[1,2-a]indéno[1,2-e]pyrazin-4-one dans 20 ml de diméthylformamide est additionnée de 1,1 ml de triéthylamine puis de 0,52 ml d'anhydride acétique. Après 18 heures de réaction à une température voisine de 20°C, le milieu réactionnel est filtré et le solide est lavé successivement avec du diméthylformamide, de l'eau distillée et de l'acétone puis séché sous pression réduite (1 mbar) à 50°C. On obtient ainsi 0,81 g de N-[(4,5-dihydro-4-oxo-10H-imidazo[1,2-a]indéno[1,2-e]pyrazin-8-yl)méthyl]acétamide sous forme d'un solide blanc fondant au-dessus de 260°C (Analyse % calculé C : 65,30; H : 4,79; N : 19,04; % trouvé C : 65,3; H : 4,8; N : 18,7).

### EXEMPLE 17

Une suspension de 1 g de chlorhydrate de 8-aminométhyl-5H,10H-imidazo[1,2-a]indéno[1,2-e]pyrazin-4-one dans 40 ml de diméthylformamide est additionnée de 1,4 ml de triéthylamine et 0,9 ml de chlorure de phénylacétyle. Après 16 heures de réaction à une température voisine de 20°C, le milieu réactionnel est filtré et le filtrat est évaporé sous pression réduite. Le résidu est mis en suspension dans 50 ml d'acétone. On obtient, après filtration et séchage sous pression réduite (1 mbar) à 60°C, 100 mg de N-[(4,5-dihydro-4-oxo-10H-imidazo[1,2-a]indéno[1,2-e]pyrazin-9-yl)méthyl]phényl acétamide sous forme d'un solide beige fondant au-dessus de 260°C (Spectre RMN ¹H dans DMSO-d6, T=300K, δ en ppm : 3,51 (2H, s, CH₂-Ph), 3,97 (2H, s, CH₂), 4,35 (2H, d, J=4Hz, NCH₂), entre 7,20 et 7,40 (6H, m, H arom.), 7,43 (1H, s, CH arom.), 7,60 (1H, s, CH arom.), 7,78 (1H, d, J=7Hz, CH arom.), 7,98 (1H, s, CH arom.), 8,63 (1H, t large, NH), 12,3 (1H, s, NH)).

### EXEMPLE 18

A une suspension de 1,5 g de 9-éthoxycarbonylméthyl-4,5-dihydro-4-oxo-10H-imidazo[1,2-a]indéno[1,2-e]pyrazin-2-carboxylate d'éthyle dans un mélange de 150 ml de dioxanne et de 40 ml d'eau distillée, on ajoute goutte à goutte 11,8 ml de soude 1 N à une température voisine de 20°C. La solution brune ainsi obtenue est agitée 4 heures à la même température. Le précipité formé est filtré, lavé avec du dioxanne puis avec de l'éther éthylique. On obtient ainsi le sel trisodique de l'acide 9-carboxyméthyl-4-oxo-4,5-dihydro-10H-imidazo[1,2-a]indéno[1,2-e]pyrazin-2-carboxylique hydraté (analyse C₁₆H₈N₃Na₃O₅; % calculé C : 49,12, H : 2,06, N : 10,74, Na : 17,63; % trouvé (sur sec) C : 49,5, H : 2,1, N : 10,6, Na : 17,2). Ce sel est repris dans l'eau distillée et la solution aqueuse ainsi obtenue est acidifiée à l'aide d'acide chlorhydrique 0,5N. Le nouveau précipité est filtré, lavé à l'eau puis au méthanol et séché sous pression réduite (1 mm Hg; 0,13 kPa) à 45°C pour conduire à 0,91 g de dihydrate de l'acide 9-carboxyméthyl-4,5-dihydro-4-oxo-10H-imidazo[1,2-a]indéno[1,2-e]pyrazin-2-carboxylique sous forme de poudre blanche dont le point de fusion est supérieur à 260°C (Analyse C16H15N3O7; 0,35H2O; % calculé C : 53,19; H : 4,18; N : 11,63; O : 31,00 % trouvé C : 53,2; H : 4,0; N : 11,4; O : 31,0); Spectre RMN ¹H : 250 MHz, DMSO-d6, T=300K, δ en ppm : 3,78 (2H, s, COCH₂), 4,03 (2H, s, CH₂), 7,28 (1H, d, J=7Hz, CH arom.), 7,42 (1H, t, J=7Hz, CH arom.), 7,85 (1H, d, J=7Hz, CH arom.), 8,55 (1H, s, CH arom.), 12,5 (1H, s, NH)). Par séchage à 60°C du dihydrate, on obtient le monohydrate (Analyse % calculé C : 55,98, H 3,82, N : 12,24 % trouvé C : 55,2, H 3,6, N 12,0). On peut également obtenir le sel disodique de l'acide 9-carboxyméthyl-4-oxo-4,5-dihydro-10H-imidazo[1,2-a]indéno[1,2-e)pyrazine-2-carboxylique par traitement de l'acide avec 2 équivalents de soude 1N (Analyse C16H9N3O5Na2, 3H2O) % calculé C : 52,04, H : 2,46, N : 11,38, Na : 12,45 % trouvé C : 51,7, H : 1,8, N : 11,4, Na : 12,6).

### EXEMPLE 18A

Le 9-éthoxycarbonylméthyl-4,5-dihydro-4-oxo-10H-imidazo[1,2-a]indéno[1,2-e]pyrazin-2-carboxylate d'éthyle peut être obtenu de la manière suivante : 7,1 g de 1-(4-éthoxycarbonylméthyl-1-oxo-indan-2-yl)imidazole-2,4-dicarboxylate de diéthyle et 83 g d'acétate d'ammonium en suspension dans 140 ml d'acide acétique sont chauffés à reflux pendant 3 heures. Après refroidissement à une température voisine de 20°C, l'insoluble est filtré, lavé à l'eau distillée puis à l'acétone et séché. On obtient 4,7 g de composé attendu sous forme de solide gris clair dont le point de fusion est supérieur à 260°C (Spectre RMN ¹H : 200 MHz, DMSO-d6, T=300K, δ en ppm : 1,21 (3H, t, J=6Hz, CH₃), 1,37 (3H, t, J=6Hz, CH₃), 3,82 (2H, s, COCH₂), 4,01 (2H, s, CH₂), 4,37 (2H, q, J=6Hz, OCH₂), 7,28 (1H, d, J=7Hz, CH arom.), 7,42 (1H, t, J=7Hz, CH arom.), 7,83 (1H, d, J=7Hz, CH arom.), 8,06 (1H, s, CH arom.)).

Le 1-(4-éthoxycarbonylméthyl-1-oxo-indan-2-yl)imidazole-2,4-dicarboxylate de diéthyle peut être préparé de la façon suivante : à une suspension de 6,99 g d'imidazole-2,4-dicarboxylate de diéthyle et de 22 g de carbonate de potassium dans 100 ml d'acétone au reflux, on ajoute goutte à goutte une solution de 9,8 g d'ester éthylique de l'acide (2-bromo-1-oxo-indan-4-yl)acétique dans 100 ml d'acétone. Le milieu réactionel est chauffé au reflux pendant 3 heures et 30 minutes, puis refroidi à une température voisine de 20°C et l'insoluble est filtré et lavé à l'acétone. Le filtrat est alors concentré à sec sous pression réduite et le produit brut ainsi obtenu purifié par flash-chromatographie sur colonne de silice en utilisant un mélange acétate d'éthyle-dichlorométhane (20/80 en volumes) comme éluant. On obtient 7,1 g de produit attendu sous forme d'une huile brune (Spectre RMN ¹H : 250 MHz, DMSO-d6, T=300K, δ en ppm : 1,18 (3H, t, J=6Hz, CH₃), 1,20 (3H, t, J=6Hz, CH₃), 1,35 (3H, t, J=6Hz, CH₃), 3,38 et 3,80 (1H chacun, respectivement dd, J=6 et 16Hz, et dd, J=8 et 16Hz, CH₂), 3,88 (2H, s, COCH₂), 4,12 (4H, m, 2 fois OCH₂), 4,32 (2H, q, J=6Hz, OCH₂), 5,91 (1H, t, J=6 et 8Hz, CH), 7,55 (1H, t, J=7Hz, CH arom.), 7,73 (2H, d, J=7Hz, CH arom.), 8,37(1H,s,CH)).

### EXEMPLE 19

On chauffe à reflux un mélange de 0,9 g de 4-benzylcarbamoyl-2-(2-éthoxycarbonylimidazolyl)-1-indanone, 30 ml d'acide acétique et 17,2 g d'acétate d'ammonium pendant 1 heure et 30 minutes. Le mélange réactionnel est additionné de 30 ml d'eau distillée et refroidi dans un bain d'eau glacée pendant 30 minutes. Le précipité est filtré, lavé à l'eau distillée (3x10 ml), à l'éthanol (10 ml) et enfin à l'acétone (3x10 ml). Après séchage à 50°C sous vide (1 mmHg; 0,13 kPa), on obtient 0,63 g de N-benzyl-(4,5-dihydro-4-oxo-10H-imidazo[1,2-a]indéno[1,2-e]pyrazin-9-yl)carboxamide sous forme de solide beige fondant au-dessus de 260°C (Analyse % calculé C : 70,77, H : 4,53, N : 15,72, O : 8,98, % trouvé C : 70,8, H : 4,5, O : 9,0).

La 4-benzylcarbamoyl-2-(2-éthoxycarbonylimidazolyl)-1-indanone peut être préparée de la façon suivante : on chauffe à reflux pendant 12 heures un mélange de 0,5 g de 4-benzylcarbamoyl-2-bromo-1-indanone, 0,37 g de 2-éthoxycarbonylimidazole et 10 ml de toluène. Le mélange réactionnel est concentré au rotavapor. Le résidu d'évaporation est additionné de dichloro-méthane, filtré et le filtrat est lavé à l'eau distillée, séché sur sulfate de sodium et évaporé au rotavapor. L'huile brune obtenue (0,26 g) est purifiée par chromatographie sur colonne de silice (diamètre : 1,5 cm, hauteur : 30 cm) en éluant avec un mélange dichlorométhane-méthanol (95/5 en volumes). Le produit meringué obtenu est trituré avec 5 ml d'acétate d'éthyle, filtré et le solide est lavé à l'acétate d'éthyle (2x5 ml) et séché à 50°C sous vide (1 mmHg; 0,13 kPa). On obtient 0,07 g de 4-benzylcarbamoyl-2-(2-éthoxycarbonylimidazolyl)-1-indanone sous forme de solide orange fondant à 218°C.

La 4-benzylcarbamoyl-2-bromo-1-indanone peut être préparée de la-manière suivante : à un mélange agité de 4,5 g de 4-benzylcarbamoyl-1-indanone, 40 ml d'acide acétique et 0,55 ml d'acide bromhydrique concentré on ajoute goutte à goutte une solution de 2,72 g de brome dans 10 ml d'acide acétique en maintenant la température du milieu réactionnel vers 16°C. On poursuit l'agitation pendant 30 minutes à environ 16°C, puis pendant 2 heures à une température voisine de 20°C. Le mélange réactionnel est ensuite traité avec 100 ml d'eau et extrait à l'acétate d'éthyle. La phase organique est lavée à l'eau distillée, séchée sur sulfate de sodium et évaporée au rotavapor. Après séchage à 65°C sous 10 mmHg, on obtient 5,7 g de 4-benzylcarbamoyl-2-bromo-1-indanone sous forme d'huile jaune qui ne tarde pas à cristalliser, utilisée telle quelle dans les synthèses ultérieures.

La 4-benzylcarbamoyl-1-indanone peut être préparée de la façon suivante : sous couverture d'azote, à une solution agitée de 5 g d'acide 1-oxo-indane-4-carboxylique dans 270 ml de diméthylformamide on ajoute goutte à goutte une solution de 3,34 ml de benzylamine dans 30 ml de diméthylformamide. On poursuit l'agitation pendant 10 minutes avant d'ajouter 4 g de 1-hydroxybenzotriazole et 5,68 g de 1-(3-diméthylaminopropyl)-3-éthyl-carbodiimide chlorhydrate. L'agitation est maintenue pendant la nuit, puis le mélange réactionnel est traité avec 250 ml d'eau et extrait à l'acétate d'éthyle (3x250 ml). La phase organique est lavée à l'eau distillée (2x250 ml), séchée sur sulfate de sodium et évaporée au rotavapor. Le résidu d'évaporation est purifié par chromatographie sur colonne de silice (diamètre: 2,5 cm, hauteur: 50 cm) en éluant avec un mélange cyclohexane-acétate d'éthyle (50/50 en volumes). On obtient 4,5 g de 4-benzylcarbamoyl-1-indanone sous forme de solide jaune orangé fondant à 124°C.

L'acide 1-oxo-indane-4-carboxylique peut être préparé de la manière suivante : à une solution refroidie dans un bain d'eau glacée constituée de 190,3 g de permanganate de potassium dans 2000 ml d'eau distillée et 20 g de bromure de tétrabutylammonium on ajoute 1500 ml de toluène, puis par portions 90 g d'acide 3-(1-oxo-indan-4-yl)acrylique. Le mélange réactionnel est agité pendant une nuit à une température voisine de 20°C, puis additionné de 200 ml d'eau et de bisulfite de sodium jusqu'à décoloratior. du milieu réactionnel. Après acidification à pH 1 avec de l'acide chlorhydrique 6N, la suspension obtenue est filtrée et le filtrat est décanté. La phase aqueuse est extraite à l'acétate d'éthyle et la phase organique obtenue est ajoutée à la phase toluénique. Après évaporation au rotavapor on obtient 35 g d'acide 1-oxo-indane-4-carboxylique sous forme de solide orange fondant à 215°C.

L'acide 3-(1-oxo-indan-4-yl)acrylique peut être préparé de la façon suivante : à un mélange de 59,1 g de 4-bromo-1-indanone, 107,9 g de tributylamine, 6,1 g de tri-o-tolylphosphine et 0,63 g d'acétate de palladium, on ajoute 20,26 g d'acide acrylique et on chauffe à 100°C pendant 2 heures. Le milieu réactionnel est refroidi à une température voisine de 20°C et traité avec 400 ml d'eau et 50 g d'hydrogénocarbonate de sodium. Le mélange est ensuite filtré et le phase aqueuse est lavée à l'éther éthylique (2x250 ml) et acidifiée avec 100 ml d'acide chlorhydrique 6N. Le précipité formé est filtré, lavé à l'acide chlorhydrique 1N et séché d'abord à l'air, puis à 50°C sous vide (1 mmHg; 0,13 kPa). On obtient 48,7 g d'acide 3-(1-oxo-indan-4-yl)acrylique sous forme de solide jaune pâle fondant à 190°C.

La 4-bromo-1-indanone peut être préparée selon le procédé décrit par M. ADAMCZYK et coll., J. Org. Chem., 49(22), 4226 (1984).

### EXEMPLE 20

On procède comme à l'exemple 19 mais à partir de 0,9 g de 4-phénylcarbamoyl-2-(2-éthoxycarbonylimidazolyl)-1-indanone, 30 ml d'acide acétique et 17,8 g d'acétate d'ammonium. On obtient 0,55 g de N-phényl-(4,5-dihydro-4-oxo-10H-imidazo[1,2-a]indéno[1,2-e]pyrazin-9-yl)carboxamide sous forme de solide rose pâle fondant au-dessus de 260°C (Analyse % calculé C : 70,17, H : 4,12, N : 16,36, O : 9,35, % trouvé C : 70,1, H : 3,8, N : 16,2).

La 4-phénylcarbamoyl-2-(2-éthoxycarbonylimidazolyl)-1-indanone peut être préparée de la manière suivante : on procède comme à l'exemple 19 pour la 4-benzylcarbamoyl-2-(2-éthoxycarbonylimidazolyl)-1-indanone, mais à partir de 3 g de 4-phénylcarbamoyl-2-bromo-1-indanone, 80 ml de toluène et 2,55 g de 2-éthoxycarbonylimidazole. Le produit brut est purifié à l'aide de deux chromatographies sur colonne de silice (diamètre : 2,5 cm, hauteur: 30 cm) en éluant avec de l'acétate d'éthyle. On obtient 0,81 g de 9-phénylcarbamoyl-2-(2-éthoxycarbonylimidazolyl)-1-indanone sous forme de solide jaune pâle fondant à 246°C.

La 4-phénylcarbamoyl-2-bromo-1-indanone peut être préparée de la façon suivante : on procède comme à l'exemple 19 pour la 4-benzylcarbamoyl-2-bromo-1-indanone mais à partir de 4,9 g de 4-phénylcarbamoyl-1-indanone, 60 ml d'acide acétique, 0,6 ml d'acide bromhydrique concentré et 2,97 g de brome. On obtient 4 g de 4-phénylcarbamoyl-2-bromo-1-indanone sous forme de solide jaune pâle fondant à 212°C.

La 4-phénylcarbamoyl-1-indanone peut être préparée de la manière suivante : on procède comme à l'exemple 19 pour la 4-benzylcarbamoyl-1-indanone mais à partir de 5 g d'acide 1-oxo-indane-4-carboxylique, 300 ml de diméthylformamide, 2,85 g d'aniline , 4 g de 1-hydroxybenzotriazole et 5,68 g de 1-(3-diméthylaminopropyl)-3-éthylcarbodiimide chlorhydrate. On obtient 4,9 g 4-phénylcarbamoyl-1-indanone sous forme de solide marron fondant à 150°C.

### EXEMPLE 21

On procède comme à l'exemple 19 mais à partir de 3 g de 4-(méthoxycarbonylméthylcarbamoyl)-2-(2-éthoxycarbonylimidazolyl)-1-indanone, 100 ml d'acide acétique et 60 g d'acétate d'ammonium. Le précipité, après lavage à l'eau, est séché à l'air et cristallisé dans 48 ml de diméthylformamide. Les cristaux sont lavés à l'éther isopropylique et séchés à 60°C sous vide (1 mmHg; 0,13 kPa). On obtient 0,96 g de N-[(4,5-dihydro-4-oxo-10H-imidazo[1,2-a]indéno[1,2-e]pyrazin-9-yl)carbonyl]glycinate de méthyle sous forme de solide rose fondant au-dessus de 260°C (Analyse % calculé C : 60,35, H : 4,17, N : 16,56, O : 18,92, % trouvé H : 4,2, N : 16,2, O : 18,3; (Spectre RMN ¹H : 250 MHz, (CD3)2SO, δ en ppm : 3,7 (s, 3H : méthyl), 4,10 (d, J=6Hz, 2H : CH2N), 4,30 (s, 1H, CH2), 7,52 (t, J=7Hz, 2H : H aromatiques), 7,55 (s, 1H : H hétérocyclique), 7,70 (d, J=7Hz, 1H : H aromatiques), 8,05 (d, J=7Hz, 1H : H aromatique), 8,10 (s, 1H : H hétérocyclique), 9,05 (t, J=6Hz, 1H : CONH), 12,45 (si, 1H : NH)).

La 4-(méthoxycarbonylméthylcarbamoyl)-2-(2-éthoxycarbonylimidazolyl)-1-indanone peut être préparée de la façon suivante : on procède comme à l'exemple 19 pour la 4-benzylcarbamoyl-2-(2-éthoxycarbonylimidazolyl)-1-indanone mais à partir de 10,2 g de 4-(méthoxycarbonylméthylcarbamoyl)-2-bromo-1-indanone, 200 ml de toluène et 7 g de 2-éthoxycarbonylimidazole. Le produit brut, dissous dans un mélange de méthanol et de dichlorométhane, est purifié par chromatographie sur colonne de silice (diamètre : 4,5 cm, hauteur : 47 cm) en éluant avec un mélange acétate d'éthyle-dichlorométhane (80/20 en volumes). On obtient 3 g de 4-(méthoxycarbonyl-méthylcarbamoyl)-2-(2-éthoxycarbonylimidazolyl)-1-indanone sous forme de solide-orange fondant à 143°C.

La 4-(méthoxycarbonylméthylcarbamoyl)-2-bromo-1-indanone peut être préparée de la manière suivante : on procède comme à l'exemple 19 pour la 4-benzylcarbamoyl-2-bromo-1-indanone mais à partir de 9,04 g de 4-(méthoxycarbonylméthylcarbamoyl)-1-indanone, 120 ml d'acide acétique, 1,7 ml d'acide bromhydrique concentré et 6,6 g de brome. On obtient 11 g de 4-(méthoxycarbonylméthylcarbamoyl)-2-bromo-1-indanone sous forme d'huile orange qui ensuite cristallise et est utilisée telle quelle dans les synthèses ultérieures.

La 4-(méthoxycarbonylméthylcarbamoyl)-1-indanone peut être préparée de la façon suivante : on procède comme à l'exemple 19 pour la 4-benzylcarbamoyl-1-indanone mais à partir de 10 g d'acide 1-oxo-indane-4-carboxylique, 400 ml de dichlorométhane, 7,4 g de glycinate de méthyle chlorhydrate, 6 g de triéthylamine, 7,9 g de 1-hydroxybenzotriazole et 11,25 g de 1-(3-diméthylaminopropyl)-3-éthylcarbodiimide chlorhydrate. Le produit brut est purifié par chromatographie sur colonne de silice (diamètre : 2,5 cm, hauteur : 38 cm) en éluant avec un mélange dichlorométhane-acétate d'éthyle (60/40 en volumes). On obtient 8,7 g de 4-(méthoxycarbonylméthylcarbamoyl)-1-indanone sous forme de solide jaune pâle fondant à 122°C.

### EXEMPLE 22

On chauffe à reflux pendant 24 heures un mélange de 1 g 9-(méthoxycarbonylméthylcarbamoyl)-5H, 10H-imidazo[1,2-a]indéno[1,2-e]pyrazin-4-one, 50 ml de dioxanne et 15 ml d'acide chlorhydrique concentré. Le mélange réactionnel est concentré au rotavapor et le résidu d'évaporation est additionné d'éther éthylique et filtré. Le solide obtenu est trituré dans 20 ml d'eau distillée, filtré, lavé à l'eau distillée (20 ml), puis à l'éther éthylique et séché à 60°C sous vide (1 mmHg; 0,13 kPa). On obtient 0,8 g de N-[(4,5-dihydro-4-oxo-10H-imidazo[1,2-a]indéno[1,2-e]pyrazin-9-yl)carbonyl]glycine sous forme de solide jaune contenant 0,66 mole d'acide chlorhydrique et fondant au-dessus de 260°C (Analyse % calculé C : 55,13, H : 3,66, Cl : 6,78, N : 16,07, O : 18,36, % trouvé C : 55,1, H : 3,6, Cl : 6,2, N : 15,8, O : 18,3).

### EXEMPLE 23

On procède comme à l'exemple 19 mais à partir de 0,9 g de 5-phénylcarbamoyl-2-(2-éthoxycarbonylimidazolyl)-1-indanone, 32 ml d'acide acétique et 17,7 g d'acétate d'ammonium. On obtient 0,5 g de N-phényl-(4,5-dihydro-4-oxo-10H-imidazo[1,2-a]indéno[1,2-e]pyrazin-8-yl)carboxamide sous forme de solide marron fondant au-dessus de 260°C (Analyse % calculé C : 70,16, H : 4,12, N : 16,37, O : 9,35, % trouvé C : 70,1, H : 3,6, N : 15,8).

La 5-phénylcarbamoyl-2-(2-éthoxycarbonylimidazolyl)-1-indanone peut être préparée de la manière suivante : on procède comme à l'exemple 19 pour la 4-benzylcarbamoyl-2-(2-éthoxycarbonylimidazolyl)-1-indanone mais à partir de 3,65 g de 5-phénylcarbamoyl-2-bromo-1-indanone, 73 ml de toluène et 3,1 g de 2-éthoxycarbonylimidazole. Le produit brut est purifié par chromatographie sur colonne de silice en éluant d'abord avec un mélange dichlorométhane-méthanol (99,5/0,5 en volumes), puis avec un mélange dichlorométhane-méthanol (98/2 en volumes). On obtient 0,9 g de 5-phénylcarbamoyl-2-(2-éthoxycarbonylimidazolyl)-1-indanone sous forme de solide marron fondant à 122°C.

La 5-phénylcarbamoyl-2-bromo-1-indanone peut être préparée de la façon suivante : on procède comme à l'exemple 19 pour la 4-benzylcarbamoyl-2-bromo-1-indanone mais à partir de 3,1 g de 5-phénylcarbamoyl-1-indanone, 37 ml d'acide acétique, 0,4 ml d'acide bromhydrique concentré et 1,96 g de brome. On obtient 3,7 g de 5-phénylcarbamoyl-2-bromo-1-indanone sous forme de solide vert de gris fondant à 144°C.

La 5-phénylcarbamoyl-1-indanone peut être préparée de la manière suivante : on procède comme à l'exemple 19 pour la 4-benzylcarbamoyl-1-indanone mais à partir de 4,4 g d'acide 1-oxo-indane-5-carboxylique, 2,86 g d'aniline, 100 ml de dichlorométhane, 3,5 g de 1-hydroxybenzotriazole et 5 g de 1-(3-diméthylaminopropyl)-3-éthylcarbodiimide chlorhydrate. Le produit brut est purifié par chromatographie sur colonne de silice en éluant avec un mélange dichlorométhane-méthanol (99/1 en volumes). On obtient 3,1 g de 5-phénylcarbamoyl-1-indanone sous forme de solide marron fondant à 174°C.

L'acide 1-oxo-indane-5-carboxylique peut être préparé de la façon suivante : on procède comme à l'exemple 19 pour l'acide 1-oxo-indane-4-carboxylique mais à partir de 22,3 g d'acide 3-(1-oxo-indan-5-yl)acrylique, 560 ml d'eau distillée, 370 ml de toluène, 4,95 g de bromure de tétrabutylammonium et 47,4 g de permanganate de potassium. On obtient 14,4 g d' acide 1-oxo-indane-5-carboxylique sous forme de solide de couleur crème fondant à 270°C.

L'acide 3-(1-oxo-indan-5-yl)acrylique peut être préparé de la manière suivante : on procède comme à l'exemple 19 pour l'acide 3-(1-oxo-indan-4-yl)acrylique mais à partir de 106,9 g de 5-bromo-1-indanone, 250 ml de tributylamine, 10,9 g de tri-o-tolylphosphine, 1,1 g d'acétate de palladium et 36,6 g d'acide acrylique. Le précipité formé par acidification à l'acide chlorhydrique 6N est purifié par chromatographie sur colonne de silice en éluant avec un mélange de dichlorométhane et de méthanol (97,5-2,5 en volumes). On obtient 22,3 g d'acide 3-(1-oxo-indan-5-yl)acrylique sous forme de solide ocre fondant à 234°C.

La 5-bromo-1-indanone peut être préparée selon le procédé décrit par J.-P. QUERE et E. MARECHAL, Bull. Soc. Chim. Fr., (8), 2983 (1971).

### EXEMPLE 24

On procède comme à l'exemple 19 mais à partir de 0,8 g de 5-benzylcarbamoyl-2-(2-éthoxycarbonylimidazolyl)-1-indanone, 28 ml d'acide acétique et 15,2 g d'acétate d'ammonium. On obtient 0,5 g de N-benzyl-(4,5-dihydro-4-oxo-10H-imidazo[1,2-a]indéno[1,2-e]pyrazin-8-yl)carboxamide sous forme de solide gris fondant au-dessus de 260°C (Analyse % calculé C : 70,77, H : 4,53, N : 15,72, O : 8,98, % trouvé C : 70,8, H : 4,8, O : 8,6).

La 5-benzylcarbamoyl-2-(2-éthoxycarbonylimidazolyl)-1-indanone peut être préparée de la façon suivante : on procède comme à l'exemple 19 pour la 4-benzylcarbamoyl-2-(2-éthoxycarbonylimidazolyl)-1-indanone mais à partir de 4,1 g de 5-benzylcarbamoyl-2-bromo-1-indanone, 82 ml de toluène et 3,3 g de 2-éthoxycarbonylimidazole. Le produit brut est purifié par chromatographie sur colonne de silice en éluant avec un mélange dichlorométhane-acétate d'éthyle (50/50 en volumes). On obtient 0,9 g de 5-benzylcarbamoyl-2-(2-éthoxycarbonylimidazolyl)-1-indanone sous forme de solide ocre fondant à 72°C.

La 5-benzylcarbamoyl-2-bromo-1-indanone peut être préparée de la manière suivante : on procède comme à l'exemple 19 pour la 4-benzylcarbamoyl-2-bromo-1-indanone mais à partir de 3,9 g de 5-benzylcarbamoyl-1-indanone, 45 ml d'acide acétique , 0,47 ml d'acide bromhydrique concentré et 2,35 g de brome. On obtient 5,1 g de 5-benzylcarbamoyl-2-bromo-1-indanone sous forme de masse pâteuse orangée utilisée telle quelle dans les synthèses ultérieures.

La 5-benzylcarbamoyl-1-indanone peut être préparée de la façon suivante : on procède comme à l'exemple 19 pour la 4-benzylcarbamoyl-1-indanone mais à partir de 4,4 g d'acide 1-oxo-indan-5-carboxylique, 88 ml de diméthylformamide, 3 ml de benzylamine, 3,5 g de 1-hydroxybenzotriazole et 5 g de 1-(3-diméthylaminopropyl)-3-éthylcarbodiimide chlorhydrate. Le produit brut est purifié par chromatographie sur colonne de silice en éluant avec un mélange dichlorométhane-méthanol (99/1 en volumes). On obtient 3,9 g de 5-benzylcarbamoyl-1-indanone sous forme de solide jaune fondant à 133°C.

### EXEMPLE 25

Sous courant d'argon on agite à une température voisine de 20°C pendant une nuit un mélange de 850 mg de 8-(N-éthylaminocarbonylméthyl)-4,5-dihydro-4-oxo-10H-imidazo[1,2-a]indéno[1,2-e]pyrazin-2-carboxylate d'éthyle, 10 ml de dioxanne, 40 ml d'eau distillée et 5,8 ml de soude 1N. Le mélange réactionnel est refroidi à une température voisine de 0°C et neutralisé avec 5,8 ml d'acide chlorhydrique 1N. Le précipité obtenu est filtré, lavé à l'eau distillée à plusieurs reprises et séché à l'étuve. On obtient 695 mg d'acide 8-(N-éthylaminocarbonylméthyl)-4,5-dihydro-4-oxo-10H-imidazo[1,2-a]indéno [1,2-e]pyrazin-2-carboxylique sous forme de solide marron fondant au-dessus de 300°C (Spectre RMN ¹H dans DMSO-d6, T=300K, δ en ppm (300 Mhz) : 1,05 (3H, t, J=6Hz, CH₃), 3,10 (2H, m, NCH₂), 3,47 (2H, s, CH₂CO), 4,02 (2H, s, CH₂), 7,30 (1H, d, J=8Hz, CH arom.), 7,48 (1H, s, CH arom.), 7,82 (1H, d, J=8Hz, CH arom.), 8,08 (1H, t, J=5Hz, NH), 8,52 (1H, s, H imidazole)).

### EXEMPLE 26

On chauffe à reflux pendant 3 heures un mélange de 3,9 g de 1-[5-(N-éthylaminocarbonylméthyl)-1-oxo-indan-2-yl]imidazole-2,4-dicarboxylate de diéthyle, 33,4 g d'acétate d'ammonium et 200 ml d'acide acétique. Le mélange réactionnel est évaporé au rotavapor et le résidu d'évaporation est additionné de 250 ml d'eau distillée et filtré. Le solide obtenu est lavé à l'eau distillée à plusieurs reprises et séché à l'étuve. On obtient 2,2 g de 8-(N-éthylaminocarbonylméthyl)-4,5-dihydro-4-oxo-10H-imidazo[1,2-a]indéno[1,2-e]pyrazin-2-carboxylate d'éthyle sous forme de solide marron clair fondant au-dessus de 300°C (Spectre RMN ¹H dans DMSO-d6, T=300K, δ en ppm (300 Mhz) : 1,05 (3H, t, J=6Hz, CH₃), 1,40 (3H, t, J=6Hz, CH₃), 3,12 (2H, m, NCH₂), 3,48 (2H, s, CH₂CO), 4,05 (2H, s, CH₂), 4,40 (2H, q, J=6Hz, CH₂O), 7,32 (1H, d, J=8Hz, CH arom.), 7,42 (1H, s, CH arom.), 7,83 (1H, d, J=8Hz, CH arom.), 8,10 (1H, t, J=5Hz, NH), 8,60 (1H, s, H imidazole)).

Le 1-[5-(N-éthylaminocarbonylméthyl)-1-oxo-indan-2-yl]imidazole-2,4-dicarboxylate de diéthyle peut être préparé de la manière suivante : on chauffe pendant 2 heures à une température voisine de 85°C un mélange de 1,84 g d'imidazole-2,4-dicarboxylate de diéthyle, 150 ml de diméthylformamide et 3,6 g de carbonate de potassium. On laisse la température du milieu réactionnel revenir à environ 20°C et on ajoute sous agitation une solution de 3,3 g de 2-bromo-5-(N-éthylaminocarbonylméthyl)indan-1-one dans 50 ml de diméthylformamide. On poursuit l'agitation durant la nuit. Le mélange réactionnel est évaporé au rotavapor et le résidu d'évaporation est additionné de 200 ml d'eau distillée et extrait avec 5x200 ml d'acétate d'éthyle. La phase organique est séchée sur sulfate de magnésium, filtrée et évaporée au rotavapor. Le résidu obtenu est purifié par chromatographie sur colonne de silice en éluant d'abord avec de l'acétate d'éthyle, puis avec un mélange acétate d'éthyle-méthanol (95-5 en volumes). On obtient 3,9 g de 1-[5-(N-éthylaminocarbonylméthyl)-1-oxo-indan-2-yl]imidazole-2,4-dicarboxylate de diéthyle sous forme de solide blanc (Spectre RMN ¹H dans DMSO-d6, T=300K, δ en ppm (300 Mhz) : 1,05 (3H, t, J=6Hz, CH₃), 1,18 (3H, t, J=6Hz, CH₃), 1,32 (3H, t, J=6Hz, CH₃), 3,10 (2H, m, NCH₂), 3,43 et 3,72 (1H chacun, respectivement dd, J=5 et 13Hz, et dd, J=7 et 13Hz, CH₂), 3,55 (2H, s, COCH₂), 4,15 (2H, q, J=6Hz, CH₂O), 4,30 (2H, q, J=6Hz, CH₂O), 5,88 (1H, dd, J=7 et 5Hz, NCH), 7,42 (1H, d, J=8Hz, CH arom.), 7,50 (1H, s, CH arom.), 7,70 (1H, d, J=8Hz, CH arom.), 8,20 (1H, t, J=5Hz, NH), 8,30 (1H, s, H imidazole)).

La 2-bromo-5-(N-éthylaminocarbonylméthyl)indan-1-one peut être préparée de la façon suivante : sous atmosphère d'argon on refroidit à une température comprise entre 0 et 3°C une solution de 3,2 g de 5-(N-éthylaminocarbonylméthyl)indan-1-one dans 237 ml de dichlorométhane et 183 ml d'éthanol et on ajoute goutte à goutte sous agitation une solution de 0,75 ml de brome dans 36 ml de dichlorométhane en maintenant la température du milieu réactionnel au-dessous de 5°C. On poursuit l'agitation pendant 90 minutes à une température voisine de 20°C, puis on évapore le mélange réactionnel au rotavapor. Le résidu d'évaporation (7,3 g) est chromatographié sur colonne de silice en éluant d'abord avec de l'acétate d'éthyle, puis avec un mélange acétate d'éthyle-méthanol (95-5 en volumes). On obtient 3,3 g de 2-bromo-5-(N-éthylaminocarbonylméthyl)indan-1-one sous forme de solide blanc [Rf= 0,64, chromatographie sur couche mince de silice, éluant: acétate d'éthyle-méthanol (95-5 en volumes)].

La 5-(N-éthylaminocarbonylméthyl)indan-1-one peut être préparée de la manière suivante : sous courant d'argon on refroidit à une température voisine de 0°C une solution de 3 g d'acide (1-oxo-indan-5-yl)acétique dans 75 ml de tétrahydrofurane et on ajoute sous agitation 3,84 g de carbonyldiimidazole. On continue d'agiter à une température de 20°C pendant une heure, puis on refroidit le milieu réactionnel à une température voisine de -10°C et on ajoute 10,3 ml de triéthylamine. On laisse la température du milieu remonter au voisinage de 20°C et on poursuit l'agitation pendant 2 heures. Le mélange réactionnel est concentré au rotavapor et additionné de 150 ml de dichlorométhane. La phase organique est lavée avec 75 ml d'eau distillée, séchée sur sulfate de magnésium, filtrée et évaporée au rotavapor. Le résidu d'évaporation (4,1 g) est purifié par chromatographie sur colonne de silice en éluant d'abord avec du dichlorométhane, puis avec un mélange dichlorométhane-méthanol (96-4 en volumes). On obtient 3,3 g de 5-(N-éthylaminocarbonylméthyl)indan-1-one sous forme de solide rose pâle (Rf= 0,50, chromatographie sur couche mince de silice, éluant: acétate d'éthyle-méthanol (95-5 en volumes); Spectre RMN ¹H dans DMSO-d6, T=300K, δ en ppm (300 Mhz) : 1,03 (3H, t, J=6Hz, CH₃), 2,62 (2H, t, J=6Hz, CH₂CO), entre 2,90 et 3,15 (4H, m, CH₂ et NCH₂), 3,50 (2H, s, CH₂ CO), 7,30 (1H, d, J=8Hz, CH arom.), 7,45 (1H, s, CH arom.), 7,60 (1H, d, J=8Hz, CH arom.), 8,12 (1H, t, J=5Hz, NH)).

### EXMPLE 27

On opère comme à l'exemple 25 mais à partir de 0,3 g de 8-(N,N-diméthylaminocarbonylméthyl)-4,5-dihydro-4-oxo-10H-imidazo[1,2-a]indéno[1,2-e]pyrazin-2-carboxylate d'éthyle, 15 ml de dioxanne, 15 ml d'eau et 2,3 ml de soude 1N. On obtient 0,2 g d'acide 8-(N,N-diméthylaminocarbonylméthyl)-4,5-dihydro-4-oxo-10H-imidazo[1,2-a]indéno[1,2-e]pyrazin-2-carboxylique sous forme de solide ocre fondant au-dessus de 260°C (Analyse C₁₈H₁₆N₄O₄ % calculé C : 61,36, H : 4,58, N : 15,90, O : 18,16, % trouvé C : 61,1, H : 4,5, N : 15,6, O : 18,0).

Le 8-(N,N-diméthylaminocarbonylméthyl)-4,5-dihydro-4-oxo-10H-imidazo[1,2-a]indéno[1,2-e]pyrazine-2-carboxylate d'éthyle peut être préparé de la manière suivante : on opère comme à l'exemple 26 mais à partir de 0,5 g de 1-[5-(N,N-diméthylaminocarbonylméthyl)-1-oxo-indan-2-yl)imidazole-2,4-dicarboxylate de diéthyle, 4,7 g d'acétate d'ammonium et 25 ml d'acide acétique. On obtient 0,3 g de 8-(N,N-diméthylaminocarbonylméthyl)-4,5-dihydro-4-oxo-10H-imidazo[1,2-a]indéno[1,2-e]pyrazine-2-carboxylate d'éthyle sous forme de solide brun fondant au-dessus de 260°C utilisé tel quel dans les synthèses ultérieures.

Le 1-[5-(N,N-diméthylaminocarbonylméthyl)-1-oxo-indan-2-yl)imidazole-2,4-dicarboxylate de diéthyle peut être préparé de la façon suivante : on opère comme dans l'exemple 26 pour la préparation du 1-[5-(N-éthylaminocarbonylméthyl)-1-oxo-indan-2-yl]imidazole-2,4-dicarboxylate de diéthyle mais à partir de 1,2 g d'imidazole-2,4-dicarboxylate de diéthyle, 150 ml de diméthylformamide, 2,37 g de carbonate de potassium et 2,2 g de 2-bromo-5-(N,N-diméthylaminocarbonylméthyl)indan-1-one. Le produit brut est purifié par chromatographie sur colonne de silice en éluant avec un mélange acétate d'éthyle-méthanol (90-10 en volumes). On obtient 0,56 g de 1-[5-(N,N-diméthylaminocarbonylméthyl)-1-oxo-indan-2-yl)imidazole-2,4-dicarboxylate de diéthyle sous forme de meringue de couleur crème (Rf= 0,36, chromatographie sur couche mince de silice, éluant: acétate d'éthyle-méthanol (90-10 en volumes)).

La 2-bromo-5-(N,N-diméthylaminocarbonylméthyl)indan-1-one peut être préparée de la manière suivante : on opère comme à l'exemple 26 pour la préparation de la 2-bromo-5-(N-éthylaminocarbonylméthyl)indan-1-one mais à partir de 2,2 g de 5-(N,N-diméthylaminocarbonylméthyl)indan-1-one, 185 ml de dichlorométhane, 60 ml d'éthanol et 0,5 ml de brome. Le produit brut est chromatographié sur colonne de silice en éluant avec un mélange d'acétate d'éthyle et de méthanol (90-10 en volumes). On obtient 2,2 g de 2-bromo-5-(N,N-diméthylaminocarbonylméthyl)indan-1-one sous forme de solide pâteux orange (Rf= 0,53, chromatographie sur couche mince de silice, éluant: acétate d'éthyle-méthanol (90-10 en volumes)).

La 5-(N,N-diméthylaminocarbonylméthyl)indan-1-one peut être préparée de la façon suivante : à un mélange de 1,9 g d'acide (1-oxo-indan-5-yl)acétique, 50 ml de dichlorométhane et 5 gouttes de diméthylformamide on ajoute sous agitation 0,86 ml de chlorure d'oxalyle et on poursuit l'agitation pendant 3 heures à une température voisine de 20°C. On ajoute ensuite 1,4 ml de triéthylamine, puis 7 ml d'une solution 2M de diméthylamine dans le toluène. On continue l'agitation durant la nuit. Le mélange réactionnel est traité avec 100 ml de dichlorométhane et 30 ml d'eau distillée. La phase organique est séchée sur sulfate de magnésium, filtrée et évaporée au rotavapor. On obtient 2,2 g de 5-(N,N-diméthylaminocarbonylméthyl)indan-1-one sous forme de solide pâteux brun (Rf= 0,44, chromatographie sur couche mince de silice, éluant: acétate d'éthyle-méthanol (90-10 en volumes)).

### EXEMPLE 28

A une suspension de 0,65 g de 9-benzylcarbamoyl-4,5-dihydro-4-oxo-10H-imidazo[1,2-a]indèno[1,2-e]pyrazin-2-carboxylate d'éthyle dans 26 ml de dioxanne, on ajoute 6,5 ml d'acide chlorhydrique (6N) et on porte le milieu réactionnel à une température voisine de 100°C pendant 7 heures. Après refroidissement et maintien de l'agitation pendant une heure à une température voisine de 20°C, le précipité ainsi obtenu est filtré sur verre fritté, lavé par 2x10 ml de dioxanne puis séché à 60°C sous pression réduite. La recristallisation dans 60 ml d'un mélange diméthylformamide-eau (2/1 en volumes) du solide obtenu, conduit, après séchage à 60°C sous pression réduite, à 0,4 g d'acide 9-benzylcarbamoyl-4,5-dihydro-4-oxo-10H-imidazo[1,2-a]indèno[1,2-e]pyrazin-2-carboxylique sous forme de solide blanc dont le point de fusion est supérieur à 260°C (Analyse C₂₂H₁₆N₄O₄ ,1,82 H₂O, 0,57 C₃H₇NO; % calculé : C : 66,00; H : 4,03; N : 13,99; O : 15,98; % trouvé : C : 59,8; H : 4,0; N : 13,9).

Le 9-benzylcarbamoyl-4,5-dihydro-4-oxo-10H-imidazo[1,2-a]indèno[1,2-e]pyrazine-2-carboxylate d'éthyle peut être préparé de la manière suivante : 0,75 g de 1-(4-benzylcarbamoyl-1-oxo-indan-2-yl)imidazole-2,4-dicarboxylate de diéthyle et 6 g d'acétate d'ammonium en suspension dans 20 ml d'acide acétique sont chauffés au reflux pendant une heure. Après refroidissement à une température voisine de 20°C, 20 ml d'eau sont ajoutés au milieu réactionnel qui est gardé sous agitation pendant 30 minutes et l'insoluble formé est filtré sur verre fritté, lavé à l'eau puis séché sous pression réduite à 60°C. On obtient ainsi 0,65 g de 9-benzylcarbamoyl-4,5-dihydro-4-oxo-10H-imidazo[1,2-a]indèno[1,2-e]pyrazine-2-carboxylate d'éthyle, sous forme de solide gris dont le point de fusion est supérieur à 260°C, utilisé tel quel dans les synthèses ultérieures.

Le 1-(4-benzylcarbamoyl-1-oxo-indan-2-yl)imidazole-2,4-dicarboxylate de diéthyle peut être préparé de la manière suivante : à une suspension de 0,31 g d'imidazole-2,4-dicarboxylate de diéthyle et de 1 g de carbonate de potassium dans 7 ml d'acétone au reflux, on ajoute goutte à goutte une solution de 0,5 g de 4-benzylcarbamoyl-2-bromo-1-indanone dans 8 ml d'acétone et on maintient le reflux pendant 15 minutes. Après refroidissement du milieu réactionnel à une température voisine de 20°C, l'insoluble est filtré sur verre fritté et lavé à l'acétone. Le filtrat est concentré à sec sous pression réduite et le produit brut ainsi obtenu est purifié par chromatographie-flash sur colonne de silice en utilisant un mélange acétate d'éthyle-dichlorométhane (30/70 en volumes) comme éluant. On obtient ainsi 0,36 g de 1-(4-benzylcarbamoyl-1-oxo-indan-2-yl)imidazole-2,4-dicarboxylate de diéthyle sous forme d'huile utilisée telle quelle dans les synthèses ultérieures.

### EXEMPLE 29

Sous couverture d'argon, à un mélange agité à une température voisine de 20°C de 1,75 g de 8-(2-éthoxycarbonyléthyl)-4,5-dihydro-4-oxo-10H-imidazo[1,2-a]indéno[1,2-e]pyrazin-2-carboxylate d'éthyle, 220 ml de dioxanne et 60 ml d'eau on ajoute 18 ml de soude 1N et on poursuit l'agitation pendant 6 heures. Le mélange réactionnel est filtré et le solide est lavé avec 2x20 ml de dioxane et additionné de 30 ml d'eau. La solution obtenue est acidifiée à pH 1 avec de l'acide chlorhydrique 1N et le précipité est filtré, lavé avec 2x30 ml d'eau distillée, puis 2x30 ml d'acétone et séché sous vide (1 mmHg; 0,13 kPa) au voisinage de 50°C. On obtient 0,9 g d'acide 8-(2-carboxyéthyl)-4,5-dihydro-4-oxo-10H-imidazo[1,2-a]indéno[1,2-e]pyrazine-2-carboxylique sous forme de solide beige fondant au-dessus de 290°C (Spectre RMN ¹H dans DMSO-d6, T=300K, δ en ppm (200 MHz): 2,60 (2H, t, J=6Hz, CH₂CO ₂H), 2,90 (2H, t, J=6Hz, CH₂), 4,00 (2H, s, CH₂), 7,25 (1H, d, J=8Hz, CH arom.), 7,45 (1H, s, CH arom.), 7,78 (1H, d, J=8Hz, CH arom.), 8,50 (1H, s, H imidazole), 12,5 (3H, s, NHCO et 2 CO₂H)).

Le 8-(2-éthoxycarbonyléthyl)-4,5-dihydro-4-oxo-10H-imidazo[1,2-a]indéno [1,2-e]pyrazine-2-carboxylate d'éthyle peut être préparé de la façon suivante : on porte à reflux pendant 45 minutes un mélange de 6,4 g de 1-[5-(2-éthoxycarbonyléthyl)-1-oxo-indan-2-yl]imidazole-2,4-dicarboxylate de diéthyle, 158 ml d'acide acétique et 110 g d'acétate d'ammonium. Le mélange réactionnel est additionné de 160 ml d'eau et le précipité obtenu est filtré, lavé avec 2x20 ml d'eau distillée, puis 2x20 ml d'acétone et séché à l'air. Le produit brut (3 g) est cristallisé dans 300 ml d'un mélange de diméthylformamide et d'eau distillée (80-20 en volumes) et les cristaux sont rincés avec 2x30 ml d'eau distillée et 2x30 ml d'acétone. Après séchage sous vide (1 mmHg; 0,13 kPa) au voisinage de 50°C on obtient 1,8 g de 8-(2-éthoxycarbonyléthyl)-4,5-dihydro-4-oxo-10H-imidazo[1,2-a]indéno[1,2-e]pyrazine-2-carboxylate d'éthyle sous forme de solide gris clair fondant au-dessus de 290°C (Spectre RMN ¹H dans DMSO-d6, T=300K, δ en ppm (200 Mhz) : 1,20 (3H, t, J=6Hz, CH₃), 1,37 (3H, t, J=6Hz, CH₃), 2,68 (2H, t, J=6Hz, CH₂CO), 2,90 (2H, t, J=6Hz, CH₂), 4,00 (2H, s, CH₂), 4,05 (2H, q, J=6Hz, CH₂O), 4,35 (2H, q, J=6Hz, CH₂O), 7,25 (1H, d, J=8Hz, CH arom.), 7,45 (1H, s, CH arom.), 7,78 (1H, d, J=8Hz, CH arom.), 8,55 (1H, s, H imidazole), 12,5 (1H, s, NHCO)).

Le 1-[5-(2-éthoxycarbonyléthyl)-1-oxo-indan-2-yl]imidazole-2,4-dicarboxylate de diéthyle peut être préparé de la manière suivante : sous atmosphère inerte on porte à reflux un mélange de 4,6 g d'imidazole-2,4-dicarboxylate de diéthyle, 94 ml d'acétone et 15 g de carbonate de potassium. On ajoute alors goutte à goutte une solution de 6,8 g de 3-(2-bromo-1-oxo-indan-5-yl)propionate d'éthyle dans 135 ml d'acétone et l'on poursuit le reflux pendant 1 heure. Le mélange réactionnel est filtré à une température voisine de 20°C et le solide obtenu est lavé avec 2x30 ml d'acétone. Le filtrat est évaporé au rotavapor et le résidu d'évaporation est chromatographié sur colonne de silice en éluant avec un mélange de cyclohexane et d'acétate d'éthyle (75-25-en volumes). On obtient 6,4 g de 1-[5-(2-éthoxycarbonyléthyl)-1-oxo-indan-2-yl]imidazole-2,4-dicarboxylate de diéthyle sous forme de solide orange clair fondant à 106°C (Spectre RMN ¹H dans DMSO-d6, T=300K, δ en ppm (300 Mhz) : 1,15 (6H, t, J=6Hz, 2 CH₃), 1,28 (3H, t, J=6Hz, CH₃), 2,72 (2H, t, J=6Hz, CH₂CO), 2,98 (2H, t, J=6Hz, CH₂), 3,40 et 3,88 (1H chacun, respectivement dd, J=5 et 12Hz, et dd, J=8 et 12Hz, CH₂), entre 3,90 et 4,30 (6H, m, 3 CH₂O), 5,85 (1H, dd, J=8 et 5Hz, NCH), 7,42 (1H, d, J=8Hz, CH arom.), 7,48 (1H, s, CH arom.), 7,65 (1H, d, J=8Hz, CH arom.), 8,30 (1H, s, H imidazole)).

Le 3-(2-bromo-1-oxo-indan-5-yl)propionate d'éthyle peut être préparé de la façon suivante : sous atmosphère inerte on agite pendant 1 heure à une température voisine de 20°C un mélange de 5,8 g de 3-(1-oxo-indan-5-yl)propionate d'éthyle, 100 ml de tétrahydrofurane et 9,4 g de tribromure de phényltriméthylammonium. Le mélange réactionnel est traité avec 250 ml d'une solution aqueuse d'hydrogénocarbonate de sodium à 5% et extrait avec 375 ml d'acétate d'éthyle au total. L'extrait organique est séché sur sulfate de magnésium, filtré et évaporé au rotavapor. Le résidu d'évaporation (10,6 g) est chromatographié sur colonne de silice en éluant avec un mélange de cyclohexane et d'acétate d'éthyle (85-15 en volumes). On obtient 7,3 g de 3-(2-bromo-1-oxo-indan-5-yl)propionate d'éthyle sous forme d'huile jaune clair (Spectre RMN ¹H dans DMSO-d6, T=300K, δ en ppm (300 Mhz) : 1,15 (3H, t, J=6Hz, CH₃), 2,70 (2H, t, J=6Hz, CH₂CO), 3,00 (2H, t, J=6Hz, CH₂), 3,35 et 3,87 (1H chacun, respectivement dd, J=2 et 12Hz, et dd, J=6 et 12Hz, CH₂), 4,05 (2H, q, J=6Hz, CH₂O), 5,02 (1H, dd, J=6 et 2Hz, CHBr), 7,40 (1H, d, J=8Hz, CH arom.), 7,45 (1H, s, CH arom.), 7,67 (1H, d, J=8Hz, CH arom)).

Le 3-(1-oxo-indan-5-yl)propionate d'éthyle peut être préparé de la manière suivante : on hydrogène à une température voisine de 20°C sous une pression de 2 bar pendant 3 heures un mélange de 1,15 g de 3-(1-oxo-indan-5-yl)acrylate d'éthyle, 23 ml d'acétate d'éthyle et 0,11 g de charbon palladié à 10%. Le mélange réactionnel est filtré sous atmosphère inerte et le filtrat est évaporé au rotavapor. Le résidu d'évaporation est chromatographié sur colonne de silice en éluant avec un mélange de cyclohexane et d'acétate d'éthyle (87,5-12,5 en volumes). On obtient 0,6 g de 3-(1-oxo-indan-5-yl)propionate d'éthyle sous forme de solide jaune très clair fondant à 53°C (Spectre RMN ¹H dans DMSO-d6, T=300K, δ en ppm (300 Mhz) : 1,12 (3H, t, J=6Hz, CH₃), 2,57 (2H, t, J=6Hz, CH₂CO), 2,65 (2H, t, J=6Hz, CH₂), 2,98 (2H, t, J=6Hz, CH₂CO), 3,05 (2H, t, J=6Hz, CH₂), 4,05 (2H, q, J=6Hz, CH₂O), 7,30 (1H, d, J=8Hz, CH arom.), 7,42 (1H, s, CH arom.), 7,58 (1H, d, J=8Hz, CH arom)).

Le 3-(1-oxo-indan-5-yl)acrylate d'éthyle peut être préparé de le façon suivante : on chauffe à une température voisine de 100°C un mélange de 63 g 5-bromo-indan-1-one, 90 ml de tributylamine, 40,8 ml d'acrylate d'éthyle, 3,65 g de tri-o-tolylphosphine et 0,67 g d'acétate de palladium. On retire le bain chauffant au voisinage de 105°C et la température du milieu réactionnel atteint rapidement 170°C, puis baisse progressivement. On maintient le milieu à une température voisine de 100°C durant 3 heures. Le mélange réactionnel est additionné de 300 ml d'acide chlorhydrique 1N et extrait avec 750 ml d'acétate d'éthyle au total. La phase organique est séchée sur sulfate de magnésium, filtrée et évaporée au rotavapor. Le résidu d'évaporation (150 g) est chromatographié sur colonne de silice en éluant avec un mélange de cyclohexane et de dichlorométhane (50-50 en volumes). On obtient 49,5 g de 3-(1-oxo-indan-5-yl)acrylate d'éthyle sous forme de solide jaune fondant à 110°C (Spectre RMN ¹H dans DMSO-d6, T=300K, δ en ppm (300 Mhz) : 1,30 (3H, t, J=6Hz, CH₃), 2,70 (2H, t, J=6Hz, CH₂CO), 3,15 (2H, t, J=6Hz, CH₂), 4,22 (2H, q, J=6Hz, CH₂O), 6,80 (1H, d, J=16Hz, CH ethylenique), 7,68 (1H, d, J=7Hz, CH arom.), 7,75 (1H, d, J=16Hz, CH ethylenique), 7,80 (1H, d, J=8Hz, CH arom), 7,95 (1H, s, CH arom.)).

La 5-bromo-indan-1-one peut être synthétisée comme décrit par J.P. QUERE et E. MARECHAL, Bull. Soc. Chim. Fr., 8, 2983 (1971).

### EXEMPLE 30

A une suspension de 0,56 g de 9-[(3-méthyluréido)méthyl]-4,5-dihydro-4-oxo-10H-imidazo[1,2-a]indéno[1,2-e]pyrazin-2-carboxylate d'éthyle dans un mélange dioxanne-eau (4/1 en volumes) agitée à une température voisine de 20°C, on ajoute goutte à goutte 4,4 ml de soude 1N. La solution ainsi obtenue est agitée 5 heures à la même température. Le milieu réactionnel est alors concentré à sec sous pression réduite et le résidu obtenu repris dans l'acide chlorhydrique 0,5 N. Le précipité formé est filtré, puis lavé à l'éther éthylique et à l'acétone pour conduire à 0,23 g d'acide 9-[(3-méthyluréido)méthyl]-4,5-dihydro-4-oxo-10H-imidazo[1,2-a]indéno[1,2-e]pyrazin-2-carboxylique sous forme de solide beige dont le point de fusion est supérieur à 260°C (Spectre RMN ¹H dans DMSO-d6, T=300K, δ en ppm (250 Mhz) : 2,60 (3H,s, NCH₃), 4,05 (2H, s, CH₂), 4,35 (2H, s, NCH₂), 6,00 (1H, s, NH), 6,60 (1H, s, NH), 7,25 (1H, d, J=8Hz, CH arom.), 7,40 (1H, t, J=8Hz, CH arom.), 7,80 (1H, d, J=8Hz, CH arom.), 8,55 (1H, s, H imidazole), 12,50 (1H, s, NHCO)).

Le 9-[(3-méthyluréido)méthyl]-4,5-dihydro-4-oxo-10H-imidazo[1,2-a]indéno [1,2-e]pyrazin-2-carboxylate d'éthyle peut être préparé selon le protocole suivant : à une suspension de 0,8 g de 9-aminométhyl-4,5-dihydro-4-oxo-10H-imidazo[1,2-a]indéno[1,2-e]pyrazin-2-carboxylate d'éthyle dans 60 ml de diméthylformamide, on ajoute goutte à goutte 0,58 ml d'isocyanate de méthyle à une température voisine de 20°C. La réaction est poursuivie une nuit à la même température. L'insoluble est filtré, lavé avec du diméthylformamide puis avec de l'éther éthylique. On obtient ainsi 0,56 g de produit attendu sous forme de solide gris clair dont le point de fusion est supérieur à 260°C (Spectre RMN ¹H dans DMSO-d6 + TFA, T=300K, δ en ppm (300 Mhz) (Spectre très mal résolu) : 1,4 (3H, CH₃), 2,6 (3H, NCH₃), 4,1 (2H, CH₂), 4,4 (4H, NCH₂ et OCH₂), entre 7,2 et 8,6 (4H, 3 CH arom. et 1 CH imidazole; Spectre de masse IE (70 ev) M/z: 381(M⁺), 350, 336, 307, 278, 261, 57)).

Le 9-aminométhyl-4,5-dihydro-4-oxo-10H-imidazo[1,2-a]indéno[1,2-e]pyrazin -2-carboxylate d'éthyle peut être obtenu selon la procédure suivante : on ajoute 1,65 ml d'hydrate d'hydrazine à 1,5 g de 9-phtalimidométhyl-4,5-dihydro-4-oxo-10H-imidazo[1,2-a]indéno[1,2-e]pyrazin-2-carboxylate d'éthyle en suspension dans 80 ml d'éthanol et on porte le milieu réactionnel au reflux pendant 24 heures. Après refroidissement à une température voisine de 20°C, l'insoluble est filtré puis lavé à l'éthanol et à l'éther éthylique. On obtient ainsi 1 g de produit attendu sous forme de solide gris clair (Spectre

RMN ¹H dans DMSO-d6, T=300K, δ en ppm (400 Mhz) : 1,35 (3H, t, J=6Hz, CH₃), 3,85 (2H, s, NCH₂), 4,05 (2H, s, CH₂), 4,38 (2H, q, J=6Hz, CH₂O), entre 7,40 et 8,10 (3H, m, 3 CH arom.), 8,60 (1H, s, H imidazole)).

Le 9-phtalimidométhyl-4,5-dihydro-4-oxo-10H-imidazo[1,2-a]indéno[1,2-e]pyrazin-2-carboxylate d'éthyle peut être préparé de la manière suivante : le mélange constitué de 4,35 g de 1-(4-phtalimidométhyl-1-oxo-indan-2-yl)imidazole-2,4-dicarboxylate d'éthyle, 40,9 g d'acétate d'ammonium et 70 ml d'acide acétique est porté au reflux pendant 7 heures. Après refroidissement à une température voisine de 20°C, l'insoluble est filtré, lavé à l'eau puis à l'acétone pour conduire à 2 g de produit attendu sous forme de solide beige utilisé sans purification supplémentaire dans les synthèses ultérieures (Spectre de masse IE (70 ev) M/z: 454(M⁺), 408, 307, 261, 160)).

Le 1-(4-phtalimidométhyl-1-oxo-indan-2-yl)imidazole-2,4-dicarboxylate d'éthyle peut être synthétisé comme suit : à une suspension de 6,27 g d'imidazole-2,4-dicarboxylate d'éthyle et 20,4 g de carbonate de potassium dans 180 ml d'acétone portés au reflux, on ajoute 15,2 g de 2-bromo-4-phtalimidométhylindan-1-one dans 220 ml d'acétone. La réaction est poursuivie 3 heures et 30 minutes à la même température. Le milieu réactionnel est alors refroidi à une température voisine de 20°C et l'insoluble filtré, puis lavé à l'acétone. Le filtrat est concentré à sec sous pression réduite puis repris dans l'acétate d'éthyle et l'insoluble filtré. Le 1-(4-phtalimidométhyl-1-oxo-indan-2-yl)imidazole-2,4-dicarboxylate d'éthyle (3,6 g) est ainsi obtenu sous forme de meringue beige (Spectre RMN ¹H dans DMSO-d6, T=300K, δ en ppm (250 Mhz) : 1,18 (3H, t, J=6Hz, CH₃), 1,33 (3H, t, J=6Hz, CH₃), 3,50 et 4,00 (1H chacun, respectivement dd, J=16 et 5Hz, et dd, J=16 et 8Hz, CH₂), 4,15 (2H, q, J=6Hz, CH₂O), 4,30 (2H, q, J=6Hz, CH₂O), 4,90 (2H, s, NCH₂), 5,91 (1H, dd, J=8 et 5Hz, NCH), 7,53 (1H, t, J=8Hz, CH arom.), 7,72 (2H, d, J=8Hz, 2 CH arom.), entre 7,80 et 8,00 (4H, m, CH phtalimide), 8,40 (1H, s, H imidazole)).

La 2-bromo-4-phtalimidométhylindan-1-one peut être préparée selon la méthode suivante : sous azote et à une température voisine de 0°C, on ajoute goutte à goutte en environ 45 minutes, 2,64 ml de brome en solution dans 50 ml de dichlorométhane à une suspension de 15 g de 4-phtalimido-méthylindan-1-one dans 100 ml de dichlorométhane. La réaction est poursuivie 6 heures à une temprature voisine de 20°C. Après addition de 100 ml d'eau distillée, les phases sont séparées par décantation et la phase organique lavée à l'eau, séchée sur sulfate de magnésium, filtrée et concentrée à sec sous pression réduite pour conduire à 17 g de composé bromé attendu sous forme de solide blanc fondant à 173°C (Spectre RMN ¹H dans DMSO-d6, T=300K, δ en ppm (250 Mhz) : 3,50 et 4,05 (1H chacun, respectivement dd, J=16 et 2Hz, et dd, J=16 et 8Hz, CH₂), 4,90 (2H, s, NCH₂), 5,10 (1H, dd, J=8 et 2Hz, BrCH), 7,50 (1H, t, J=8Hz, CH arom.), 7,70 (2H, d, J=8Hz, 2 CH arom.), entre 7,80 et 8,00 (4H, m, CH phtalimide)).

La 4-phtalimidométhylindan-1-one peut être préparée de la façon suivante : une solution de chlorure de l'acide 3-[2-(phtalimidométhyl)phényl]propionique (préparé par réaction à 30°C pendant 4 heures et 30 minutes de 83,39 g d'acide 3-[2-(phtalimidométhyl)phényl]propionique avec 39,3 ml de chlorure de thionyle dans 330 ml de dichlorométhane) dans 500 ml de 1,2-dichloroéthane est ajoutée goutte à goutte à 107,9 g de chlorure d'aluminium en suspension dans 800 ml de 1,2-dichloroéthane à une température inférieure à 20°C. La réaction est poursuivie une nuit à 20°C, puis le milieu réactionnel versé sur de la glace. L'insoluble ainsi formé est filtré, lavé avec une solution aqueuse saturée de carbonate de sodium puis avec de l'eau distillée. On obtient après séchage 41,1 g de 4-phtalimidométhylindan-1-one sous forme de poudre blanche fondant à 199°C. L'extraction de la phase organique et la concentration à sec conduisent à la récupération de 30 g supplémentaires de produit attendu (Spectre RMN ¹H dans DMSO-d6, T=300K, δ en ppm (250 Mhz) : 2,70 (2H, t, J=6Hz, CH₂CO), 3,20 (2H, t, J=6Hz, CH₂), 4,90 (2H, s, NCH₂), 7,40 (1H, t, J=8Hz, CH arom.), 7,60 (2H, d, J=8Hz, 2 CH arom.), entre 7,80 et 8,00 (4H, m, CH phtalimide)).

L'acide 3-[2-(phtalimidométhyl)phényl]propionique peut être obtenu selon la procédure suivante : une solution de 95 g d'acide o-(phtalimidométhyl) cinnamique dans 900 ml de diméthylformamide est additionnée de 4 g de palladium sur charbon à 10% et placée sous hydrogène à une pression voisine de 1 bar et à une température voisine de 20°C. Après 15 heures de réaction, le milieu réactionnel est filtré sur célite, l'insoluble lavé avec du diméthylformamide et le filtrat concentré à sec sous pression réduite. Le résidu est repris dans l'éther éthylique et l'insoluble filtré et séché pour conduire à 88,39 g de produit attendu sous forme de poudre blanche fondant à 180°C (Spectre RMN ¹H dans DMSO-d6, T=300K, δ en ppm (200 Mhz) : 2,60 (2H, t, J=6Hz, CH₂CO), 3,02 (2H, t, J=6Hz, CH₂), 4,83 (2H, s, NCH₂), entre 7,00 et 7,40 (4H, m, 4 CH arom.), entre 7,80 et 8,00 (4H, m, CH phtalimide), 12,20 (1H, s, COOH)).

L'acide o-(phtalimidométhyl)cinnamique peut être obtenu selon la méthode suivante : dans un ballon sous courant d'azote sont introduits successivement 149 g de 1-bromo-2-phtalimidométhylbenzène, 337 ml de tributylamine, 5,7 g de tri-o-tolylphosphine, 1 g d'acétate de palladium et 40 ml d'acide acrylique. La suspension est chauffée 2 heures à 100°C puis laissée la nuit à une température voisine de 20°C. Une phase huileuse noire est décantée puis coulée sur une solution aqueuse d'acide chlorhydrique (170 ml d'acide chlorhydrique 12N dans 1 I d'eau distillée). Le précipité formé est filtré, lavé à l'eau et séché. La poudre grisâtre obtenue est reprise dans le diméthylformamide (500 ml) à 60°C et traitée par du noir animal. La filtration sur célite et la concentration à sec du filtrat conduisent à une pâte jaune, reprise dans l'éther éthylique, puis l'insoluble est filtré et séché. On obtient 95,8 g d'acide cinnamique attendu sous forme de poudre blanche fondant à 234°C (Spectre de masse lE (70 ev) M/z: 307 (M⁺), 261, 160, 147, 115).

Le 1-bromo-2-phtalimidométhylbenzène peut être obtenu comme suit: une solution de 150 g de bromure d'o-bromobenzyle dans 1,2 litre de dméthylformamide est traitée par 155,5 g de phtalimidate de potassium et chauffée à 60°C pendant 5 heures. Après refroidissement à une température voisine de 20°C, le mélange réactionnel est filtré et l'insoluble lavé au méthanol et séché. 59 g de produit attendu sont ainsi obtenus sous forme de poudre blanche fondant à 170°C. Le filtrat est concentré à sec sous pression réduite et le résidu repris dans 200 ml de méthanol; l'insoluble est filtré, lavé au méthanol et séché pour conduire à 88,2 g supplémentaires de produit attendu sous forme de poudre blanche fondant à 165°C (Spectre RMN ¹H dans DMSO-d6, T=300K, δ en ppm (200 Mhz) : 4,80 (2H, s, NCH₂), entre 7,10 et 7,40 (3H, m, 3 CH arom.), 7,65 (1H, d, J=8Hz, CH arom.), entre 7,80 et 8,10 (4H, m, CH phtalimide)).

### EXEMPLE 31

Une solution de 0,4 g de 9-éthoxycarbonylméthyl-4,5-dihydro-4-oxo-10H-imidazo[1,2-a]indéno[1,2-e]pyrazin-2-phosphonate de diéthyle dans 10 ml d'acide chlorhydrique 6N est chauffée à 100°C pendant 24 heures. L'insoluble formé est filtré, lavé à l'eau puis à l'acétone pour conduire après séchage à 0,18 g de chlorhydrate de l'acide 9-carboxyméthyl-4,5-dihydro-4-oxo-10H-imidazo[1,2-a]indéno[1,2-e]pyrazin-2-phosphonique sous forme de poudre blanche dont le point de fusion est supérieur à 260°C (Analyse C15H12N3O6P,HCl; % calculé C : 45,30; H : 3,29; N : 10,57; P : 7,79; % trouvé C : 45,6; H : 3,7; N : 10,5; P : 7,5; Spectre RMN ¹H dans DMSO-d6, T=300K, δ en ppm (300 Mhz) : 3,75 (2H, s, COCH₂), 4,00 (2H, s, CH₂), 7,25 (1H, d, J=8Hz, CH arom.), 7,40 (1H, t, J=8Hz, CH arom.), 7,80 (1H, d, J=8Hz, CH arom.), 8,22 (1H, s, H imidazole), 12,50 (1H, s, NHCO)).

Le 9-éthoxycarbonylméthyl-4,5-dihydro-4-oxo-10H-imidazo[1,2-a]indéno[1,2-e]pyrazin-2-phosphonate de diéthyle peut être préparé selon le protocole suivant : le mélange constitué de 0,995 g de 2-éthoxycarbonyl-1-(4-éthoxycarbonylméthyl-1-oxo-indan-2-yl)imidazole-4-phosphonate de diéthyle, 7 g d'acétate d'ammonium et 20 ml d'acide acétique est porté au reflux pendant 4 heures. Après refroidissement à une température voisine de 20°C, le milieu réactionnel est versé dans un mélange d'eau et de glace pilée et la phase organique extraite par de l'acétate d'éthyle, lavée à l'eau, séchée sur sulfate de sodium, filtrée et concentrée à sec sous pression réduite. L'huile brune ainsi obtenue est purifiée par chromatographie sous moyenne pression sur silice en utilisant un mélange de dichlorométhane et de méthanol (98/2 en volumes) comme éluant. On isole 0,4 g de produit attendu sous forme de poudre cristalline grisâtre (Spectre RMN ¹H dans DMSO-d6, T=300K, δ en ppm (300 Mhz) : entre 1,10 et 1,40 (9H, m, 3 CH₃), 3,80 (2H, s, COCH₂), 4,00 (2H, s, CH₂), entre 4,05 et 4,20 (6H, m, 3 CH₂O), 7,22 (1H, d, J=8Hz, CH arom.), 7,40 (1H, t, J=8Hz, CH arom.), 7,80 (1H, d, J=8Hz, CH arom.), 8,45 (1H, s, H imidazole), 12,50 (1H, s, NHCO)).

Le 2-éthoxycarbonyl-1-(4-éthoxycarbonylméthyl-1-oxo-indan-2-yl)imidazole-4-phosphonate de diéthyle peut être obtenu comme suit: à une suspension de 2,1 g de 2-éthoxycarbonyl-imidazole-4-phosphonate de diéthyle et 7 g de carbonate de potassium dans 45 ml d'acétone portés au reflux, on ajoute 3,15 g de (2-bromo-1-oxo-indan-4-yl)acétate d'éthyle dans 20 ml d'acétone. La réaction est poursuivie 2 heures à la même température. Le milieu réactionnel est alors refroidi à une température voisine de 20°C et l'insoluble filtré, puis lavé à l'acétone. Le filtrat est concentré à sec sous pression réduite et le résidu noir purifié par chromatographie flash sur silice en utilisant de l'acétate d'éthyle comme éluant. Le produit attendu (0,995 g) est ainsi obtenu sous forme d'huile jaune (Spectre RMN ¹H dans DMSO-d6, T=300K, δ en ppm (300 Mhz) : entre 1,00 et 1,40 (12H, m, 4 CH₃), 3,35 et 3,95 (1H chacun, respectivement dd, J=16 et 5Hz, et dd, J=16 et 8Hz, CH₂), 3,85 (2H, s, COCH2), entre 4,00 et 4,20 (8H, m, 4 CH₂O), 5,90 (1H, dd, J=8 et 5Hz, NCH), 7,51 (1H, t, J=8Hz, CH arom.), 7,70 (2H, d, J=8Hz, 2 CH arom.), 8,25 (1H, s, H imidazole)).

Le 2-éthoxycarbonyl-imidazole-4-phosphonate de diéthyle peut être préparé de la manière suivante : on refroidit à une température voisine de 10°C une solution de 1,2 g d'(hydroxyamino)iminoacétate d'éthyle dans 20 ml de chloroforme et 1,4 ml de triéthylamine, et on ajoute goutte àgoutte une solution de 1,54 g d'éthynylphosphonate de diéthyle dans 5 ml de chloroforme. Le milieu réactionnel est agité pendant la nuit à une température voisine de 20°C, additionné de 0,15 g d'éthynylphosphonate de diéthyle et chauffé pendant 1 heure à une température voisine de 50°C. Le mélange réactionnel est additionné de 50 ml de dichlorométhane et lavé avec 3x40 ml de solution saturée de chlorure de sodium. La phase organique est évaporée au rotavapor et le résidu d'évaporation est additionné de 40 ml d'éther éthylique et filtré. Le filtrat est évaporé au rotavapor pour donner une huile jaune (2,4 g). A cette huile on ajoute 20 ml de xylène et l'on chauffe à reflux pendant 20 heures. La phase liquide est décantée et évaporée au rotavapor. Le résidu d'évaporation est purifié par chromatographie sur colonne de silice en éluant avec de l'acétate d'éthyle. On obtient 0,5 g 2-éthoxycarbonyl-imidazole-4-phosphonate de diéthyle sous forme d'huile jaune (Spectre de masse (impact électronique) m/z 276 (M)⁺, 247 (276-C₂H₅)⁺, 231 (276-C₂H₅O)⁺, 204 (C₆H₉N₂O₄P)⁺, 157 (C₄H₂N₂O₃P)⁺).

L'(hydroxyamino)iminoacétate d'éthyle peut être synthétisé comme décrit par W.K. WARBURTON, J. Chem. Soc. (C), 1522 (1966).

L'éthynylphosphonate de diéthyle peut être synthétisé comme décrit par D.T. MONAGHAN et coll., Brain Res., 278, 138 (1983).

### EXEMPLE 32

Sous courant d'azote et à une température voisine de 20°C, on ajoute goutte à goutte 8,2 ml de soude 1N à une suspension de 1 g de 9-(N-méthylaminocarbonylméthyl)-4,5-dihydro-4-oxo-10H-imidazo[1,2-a]indéno [1,2-e]pyrazin-2-carboxylate d'éthyle dans un mélange dioxanne eau distillée (100/27 en volumes). La réaction est poursuivie 2 heures à la même température. L'insoluble gris clair est filtré, lavé à l'acétone puis repris dans 15 ml d'eau distillée. Cette solution aqueuse est filtrée, puis acidifiée jusqu'à pH 1 à l'aide d'acide chlorhydrique N. Le précipité formé est filtré, lavé à l'eau, à l'acétone puis à l'éther éthylique pour conduire à 0,59 g de trihydrate de l'acide 9-(N-méthylaminocarbonylméthyl)-4,5-dihydro-4-oxo-10H-imidazo[1,2-a]indéno[1,2-e]pyrazin-2-carboxylique sous forme de poudre blanche dont le point de fusion est supérieur à 260°C (Analyse C17H14N4O4, 3H2O; % calculé C : 60,35; H : 4,17; N : 16,56 % trouvé C : 60,0; H : 4,1; N : 16,3; Spectre RMN ¹H dans CD₃CO₂D, T=300K, δ en ppm (300 Mhz) : 2,7 (3H, s, NCH₃), 3,9 (2H, s, CH₂CO), 4,1 (2H, s, CH₂), 7,2 (1H, d, J=8Hz, CH arom.), 7,4 (1H, t, J=8Hz, CH arom.), 7,7 (1H, d, J=8Hz, CH arom.), 8,5 (1H, s, H imidazole)).

Le 9-(N-méthylaminocarbonylméthyl)-4,5-dihydro-4-oxo-10H-imidazo[1,2-a] indéno[1,2-e]pyrazin-2-carboxylate d'éthyle peut être préparé selon le protocole suivant : le mélange constitué de 1,5 g de 1-[4-(N-méthylaminocarbonylméthyl)-1-oxo-indan-2-yl]imidazole-2,4-dicarboxylate d'éthyle, 14,2 g d'acétate d'ammonium et 60 ml d'acide acétique est porté au reflux pendant 6 heures. Après refroidissement à une température voisine de 20°C, l'insoluble est filtré, lavé à l'eau puis à l'acétone. On isole ainsi 1 g de produit attendu sous forme de solide grisâtre dont le point de fusion est supérieur à 260°C (Spectre RMN ¹H dans DMSO-d6, T=300K, δ en ppm (300 Mhz) : 1,35 (3H, t, J=6Hz, CH₃), 2,60 (3H, d, J=5Hz, NCH₃), 3,60 (2H, s, CH₂CO), 4,05 (2H, s, CH₂), 4,35 (2H, q, J=6Hz, CH₂O), 7,20 (1H, d, J=8Hz, CH arom.), 7,40 (1H, t, J=8Hz, CH arom.), 7,75 (1H, d, J=8Hz, CH arom.), 8,60 (1H, s, H imidazole)).

Le 1-[4-(N-méthylaminocarbonylméthyl)-1-oxo-indan-2-yl]imidazole-2,4-dicarboxylate d'éthyle peut être obtenu comme suit : sous courant d'azote, une solution de 3,77g d'imidazole-2,4-dicarboxylate d'éthyle, 6,14 g de carbonate de potassium et 0,117 g d'éther couronne 18-c-6 dans 225 ml de diméthylformamide est chauffée 2 heures à 85°C. Après refroidissement à une température voisine de 20°C, une solution de 5 g de N-méthyl-(2-bromo-1-oxo-indan-4-yl)acétamide dans 75 ml de diméthylformamide est ajoutée goutte à goutte au milieu réactionnel. La réaction est poursuivie une nuit à la même température. Le milieu réactionnel est concentré à sec sous pression réduite, puis le résidu repris dans l'eau distillée et la phase organique extraite par du dichlorométhane, lavée à l'eau, séchée sur sulfate de magnésium, traitée au noir 3S, filtrée et concentrée à sec sous pression réduite. L'huile brune ainsi obtenue est reprise dans l'acétonitrile et le précipité formé, filtré et séché. Le produit attendu (1,5 g) est obtenu sous forme de solide blanc se décomposant à 166°C (Spectre RMN ¹H dans DMSO-d6, T=300K, δ en ppm (300 Mhz) : 1,12 (3H, t, J=6Hz, CH₃), 1,30 (3H, t, J=6Hz, CH₃), 2,60 (3H, d, J=5Hz, NCH₃), 3,33 et 3,81 (1H chacun, respectivement dd, J=16 et 5Hz, et dd, J=16 et 8Hz, CH₂), 3,55 (2H, s, COCH₂), 4,12 (2H, q, J=6Hz, CH₂O), ), 4,30 (2H, q, J=6Hz, CH₂O), 5,90 (1H, dd, J=8 et 5Hz, NCH), 7,51 (1H, t, J=8Hz, CH arom.), entre 7,60 et 7,75 (2H, m, 2 CH arom.), 8,05 (1H, q, J=5Hz, NH), 8,32 (1H, s, H imidazole)).

Le N-méthyl-(2-bromo-1-oxo-indan-4-yl)acétamide peut être préparé selon la procédure suivante : sous azote et à une température voisine de 0°C, on ajoute goutte à goutte en environ 30 minutes, 1,05 ml de brome en solution dans 25 ml de dichlorométhane à une solution de 4,18 g de N-méthyl-(1-oxo-indan-4-yl)acétamide dans un mélange de dichlorométhane (100 ml) et d'éthanol (150 ml). La réaction est poursuivie 5 heures à une température voisine de 20°C. Le milieu réactionnel est alors concentré à sec sous pression réduite, puis le résidu obtenu repris dans le dichlorométhane. L'insoluble est filtré, lavé avec de l'acétate d'éthyle et séché. On obtient 5 g de composé bromé attendu sous forme de solide jaune utilisé sans purification supplémentaire dans les étapes ultérieures (Spectre RMN ¹H dans DMSO-d6, T=300K, δ en ppm (300 Mhz) : 2,60 (3H, d, J=5Hz, NCH₃), 3,32 et 3,88 (1H chacun, respectivement dd, J=16 et 2Hz, et dd, J=16 et 8Hz, CH₂), 3,50 (2H, s, COCH₂), 5,05 (1H, dd, J=8 et 2Hz, BrCH), entre 7,35 et 7,70 (3H, m, 3 CH arom.), 8,00 (1H, s, NHCO)).

Le N-méthyl-(1-oxo-indan-4-yl)acétamide peut être synthétisé comme suit : sous atmosphère d'azote et à 0°C, une solution de 4,5 g d'acide (1-oxo-indan-4-yl)acétique dans 100 ml de tétrahydrofuranne est traitée par 5,7 g de N,N'-carbonyldiimidazole. La réaction est poursuivie 1heure et 30 minutes à une température voisine de 20°C. Après refroidissement à -5°C, 2,67 g de chlorhydrate de méthylamine sont ajoutés au milieu réactionnel et l'agitation maintenue une nuit à une température voisine de 20°C. La concentration sous pression réduite du milieu réactionnel conduit à un résidu qui est repris dans le dichlorométhane et cette phase organique lavée à l'eau, séchée sur sulfate de magnésium, filtrée et concentrée à sec sous pression réduite. On obtient ainsi 3,9 g d'amide attendu sous forme d'huile jaune qui cristallise (Spectre de masse IE (70 ev) M/z: 203(M^{+.}), 146, 117, 58)).

### EXEMPLE 33

Sous courant d'azote et à une température voisine de 20°C, on ajoute goutte à goutte 19 ml de soude 1N à une suspension de 1,9 g de 9-(1-éthoxycarbonyléthyl)-4,5-dihydro-4-oxo-10H-imidazo[1,2-a]indéno[1,2-e]pyrazin-2-carboxylate d'éthyle dans un mélange de dioxanne (245 ml) et d'eau distillée (65 ml). La réaction est poursuivie 4 heures et 30 minutes à la même température. L'insoluble, formé après évaporation partielle des solvants, est filtré, lavé à l'eau puis avec un mélange de diméthylformamide (10 ml) et de méthanol (80 ml) et séché. Le solide est solubilisé dans 20 ml de soude 1N et agité 4 heures à une température voisine de 20°C. Le milieu réactionnel est alors traité avec de l'acide chlorhydrique 1N jusqu'à pH 1 et le précipité formé filtré, lavé à l'eau, puis à l'acétone et séché pour conduire à 0,62 g d'hydrate de l'acide 9-(1-carboxyéthyl)- 4,5-dihydro-4-oxo-10H-imidazo[1,2-a]indéno[1,2-e]pyrazin-2-carboxylique sous forme de poudre rosée dont le point de fusion est supérieur à 260°C (Analyse C17H13N3O5, 1,4H2O; % calculé C : 60,18; H : 3,86; N : 12,38 % trouvé C : 60,1; H : 3,2; N : 12,5; Spectre RMN ¹H dans DMSO-d6, T=300K, δ en ppm (300 Mhz) : 1,5 (3H, CH₃), 3,9 (1H, CH), 4,1 (2H, CH₂), 7,3, 7,5, 7,9 (1H chacun,3-CH arom), 8,6 (1H, CH imidazole), entre 12 et 13,5 (3H, 2 COOH et NHCO)).

Le 9-(1-éthoxycarbonyléthyl)-4,5-dihydro-4-oxo-10H-imidazo[1,2-a]indéno [1,2-e]pyrazin-2-carboxylate d'éthyle peut être obtenu comme suit : le mélange constitué de 3,67 g de 1-[4-(1-éthoxycarbonyléthyl)-1-oxo-indan-2-yl]imidazole-2,4-dicarboxylate d'éthyle, 40 g d'acétate d'ammonium et 80 ml d'acide acétique est porté au reflux pendant 3 heures. Après refroidissement à une température voisine de 20°C, l'insoluble est filtré et lavé à l'eau. On isole ainsi 1,9 g de produit attendu sous forme de poudre rosée dont le point de fusion est supérieur à 260°C et qui est utilisée sans purification supplémentaire dans les synthèses ultérieures.

Le 1-[4-(1-éthoxycarbonyléthyl)-1-oxo-indan-2-yl]imidazole-2,4-dicarboxylate d'éthyle peut être préparé selon la procédure suivante : sous courant d'azote, une solution de 5,24 g d'imidazole-2,4-dicarboxylate d'éthyle et 16 g de carbonate de potassium dans 100 ml d'acétone est chauffée au reflux. Une solution de 7,7 g de 2-(2-bromo-1-oxo-indan-4-yl)propionate d'éthyle dans 50 ml d'acétone est alors ajoutée goutte à goutte et le reflux poursuivi pendant 3 heures. Après refroidissement à une température voisine de 20°C, l'insoluble est filtré, lavé à l'acétone et le filtrat concentré à sec sous pression réduite. Le produit brut obtenu est purifié par chromatographie flash sur colonne de silice en utilisant du dichlorométhane puis un mélange dichlorométhane-méthanol (98/2 en volumes) comme éluants. Une deuxième chromatographie flash sur colonne de silice en utilisant un mélange d'acétate d'éthyle et de cyclohexane (60/40 en volumes) comme éluant conduit à 3,67 g de produit attendu sous forme d'une huile orangée.

Le 2-(2-bromo-1-oxo-indan-4-yl)propionate d'éthyle peut être préparé de la façon suivant: sous azote et à une température voisine de 15°C, on ajoute goutte à goutte 1,2 ml de brome en solution dans 10 ml de dichlorométhane à une solution de 5,64 g de α-méthyl-(1-oxo-indan-4-yl)acétate d'éthyle dans 50 ml de dichlorométhane. La réaction est poursuivie 2 heures à une temprature voisine de 20°C. 40 ml d'eau distillée sont alors ajoutés au milieu réactionnel et la phase organique séparée, lavée à l'eau, séchée et concentrée à sec sous pression réduite. On obtient 7,7 g de composé bromé attendu sous forme d'une huile rouille utilisée sans purification supplémentaire dans les étapes ultérieures.

Le α-méthyl-(1-oxo-indan-4-yl)acétate d'éthyle peut être synthétisé selon le protocole suivant : à 8,6 g d'acide α-méthyl-(1-oxo-indan-4-yl)acétique en solution dans un mélange de 140 ml de dichlorométhane et 32 ml d'éthanol absolu sont ajoutés goutte à goutte 4 ml de chlorure d'oxalyle en maintenant la température inférieure à 25°C. Après 4 heures d'agitation à une température voisine de 20°C, le milieu réactionnel est concentré à sec sous pression réduite et le résidu repris avec de l'eau distillée et du dichlorométhane. La phase oragnique est séparée, lavée à l'eau, séchée et concentrée à sec sous pression réduite. L'huile brune obtenue est purifiée par chromatographie flash sur colonne de silice en utilisant du dichlorométhane comme éluant. On obtient ainsi 5,64 g d'ester attendu sous forme d'une huile rouille.

L'acide α-méthyl-(1-oxo-indan-4-yl)acétique peut être obtenu selon la méthode suivante : à 40 g d'acide phosphorique on ajoute 50 g de pentoxyde de phosphore et on chauffe le mélange à 130°C pendant 3 heures. Le mélange est alors refroidi à 90°C, température à laquelle on introduit 10,4 g d'acide 3-[o-(1-carboxyéthyl)phényl]propionique. La réaction est poursuivie 30 minutes à 90°C. Après refroidissement à une température voisine de 20°C, le milieu réactionnel est versé sur de la glace pilée. La phase organique est extraite par de l'acétate d'éthyle, lavée à l'eau, séchée sur sulfate de sodium, filtrée et concentrée à sec sous pression réduite. On obtient ainsi 8,6 g d'indanone attendue sous forme de meringue jaune (Spectre RMN ¹H dans DMSO-d6, T=300K, δ en ppm (300 Mhz) : 1,42 (3H, d, J=5Hz, NCH₃), 2,70 (2H, m, COCH₂), 3,12 (2H, t, J=6Hz, CH₂), 3,97 (1H, q, J=6Hz, CH), entre 7,40 et 7,75 (3H, m, 3 CH arom)).

L'acide 3-[o-(1-carboxyéthyl)phényl]propionique peut être préparé comme suit: 13 g d'acide o-(1-carboxyéthyl)cinnamique en solution dans 130 ml de diméthylformamide sont hydrogénés en présence de 0,9 g de palladium sur charbon à 10% à une température voisine de 20°C sous une pression de 1,2 bar pendant 3 heures. Après filtration du mélange réactionnel sur célite et lavage de l'insoluble avec du diméthylformamide, le filtrat est concentré à sec sous pression réduite. L'acide propionique attendu est ainsi obtenu (10,4 g) sous forme de poudre jaune pâle fondant à 154°C (Spectre RMN ¹H dans DMSO-d6, T=300K, δ en ppm (200 MHz): 1,30 (3H, d, J=6Hz, CH₃), 2,50 (2H, m, COCH₂), 2,90 (2H, m, CH₂), 3,90 (1H, q, J=6Hz, CH), entre 7,10 et 7,40 (4H, m, 4 CH arom), 12,30 (2H, s, 2 COOH)).

L'acide o-(1-carboxyéthyl)cinnamique peut être synthétisé de la manière suivante : dans un ballon sous courant d'azote sont introduits successivement 23,34 g de l'acide 2-(o-bromophényl)propionique, 400 ml d'eau distillée, 185 ml d'une solution aqueuse de carbonate de sodium 1,6M, 2,24 g d'acétate de palladium et 10,35 ml d'acide acrylique. La suspension est chauffée 17 heures au reflux. On rajoute alors 5 ml d'acide acrylique et 1 g d'acétate de palladium et on chauffe à nouveau au reflux pendant 18 heures. Après refroidissement à une température vosine de 20°C, le mélange réactionnel est filtré, lavé à l'eau puis acidifié à l'aide d'acide chlorhydrique 6N. Le précipité formé est filtré, lavé à l'eau puis avec du dichlorométhane et séché. On obtient 13 g de l'acide cinnamique attendu sous forme de poudre brune fondant à 176°C (Spectre RMN ¹H dans DMSO-d6, T=300K, δ en ppm (300 MHz): 1,35 (3H, d, J=6Hz, CH₃), 4,08 (1H, q, J=6Hz, CH), 6,48 (1H, d, J=16Hz, CH éthylènique), entre 7,30 et 7,55 (3H, m, 3 CH arom), 7,75 (1H, d, J=8Hz, CH arom), 8,00 (1H, d, J=16Hz, CH ethylénique), 12,50 (2H, s, 2 COOH)).

L'acide 2-(o-bromophényl)propionique peut être préparé comme suit: 17 g de 2-(o-bromophényl)propionate de méthyle sont agités avec 100 ml de soude N dans 100 ml de tétrahydrofuranne à une température voisine de 20°C pendant 4 heures. Le milieu réactionnel est alors acidifié à l'aide d'acide chlorhydrique N et la phase organique est extraite par du dichlorométhane, lavée à l'eau, séchée et concentrée à sec sous pression réduite pour conduire à 16,4 g d'acide attendu sous forme de meringue utilisée sans purification supplémentaire dans les synthèses ultérieures.

Le 2-(o-bromophényl)propionate de méthyle peut être préparé selon la procédure suivante : à une solution de 15 g d'acide o-bromophénylacétique dans 150 ml de dichlorométhane, on ajoute, à une température voisine de 20°C, 54 g de diméthylsulfate. A cette solution refroidie à 5°C, on ajoute 37 g de potasse concassée puis 4,98 g de chlorure de triéthylbenzylammonium. La réaction est poursuivie une nuit à une température voisine de 20°C. Le mélange réactionnel est alors versé dans 100 ml d'eau et la phase organique décantée, lavée avec de l'acide chlorhydrique N, puis à l'eau, séchée et concentrée à sec sous pression réduite. On obtient ainsi 17 g de produit attendu sous forme d'une huile jaune (Spectre RMN ¹H dans DMSO-d6, T=300K, δ en ppm (250 MHz): 1,45 (3H, d, J=6Hz, CH₃), 3,65 (3H, s, OCH₃), 4,20 (1H, q, J=6Hz, CH), entre 7,20 et 7,50 (3H, m, 3 CH arom), 7,67 (1H, d, J=8Hz, CH arom)).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles-qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischèmie cérébrale, les effets dus à une anoxie, l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse, de la maladie de PARKINSON, vis-à-vis des manifestations épileptogènes et/ou convulsives, les traumatismes cérébraux ou spinaux, les traumatismes liés à la dégénérescence de l'oreille interne ou de la rétine, du tinnitus, de l'anxiété, de la dépression, de la schizophrénie, du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques, antiinflammatoires, antianorexiques, antimigraineux, antiémétiques et pour traiter les empoisonnements par des neurotoxines ou d'autres substances agonistes du récepteur NMDA ou AMPA, ainsi que les troubles neurologiques associés aux maladies virales telles que les méningites et encéphalites virales, le SIDA, la rage, la rougeole et le tétanos, pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines, dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule : 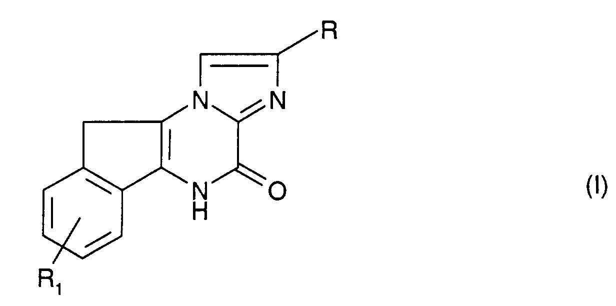 dans laquelle
- R représente un atome d'hydrogène ou un radical carboxy, alcoxycarbonyle, -CO-NR₄R₅, -PO₃H₂ ou -CH₂OH,
- R₁ représente un radical -alk-NH₂, -alk-NH-CO-R₃, -alk-COOR₄, -alk-CO-NR₅R₆ ou -CO-NH-R₇,
- R₃ représente un radical alkyle, phényle, phénylalkyle, cycloalkyle ou -NR₆R₈,
- R₄ représente un atome d'hydrogène ou un radical alkyle,
- R₅ représente un atome d'hydrogène ou un radical alkyle, phényle, cycloalkyle ou phénylalkyle,
- R₆ représente un atome d'hydrogène ou un radical alkyle,
ou bien R₅ et R₆ forment avec l'atome d'azote auquel ils sont rattachés un hétérocycle mono ou polycyclique saturé ou insaturé contenant 1 à 6 atomes de carbone et éventuellement un ou plusieurs autres hétéroatomes choisis parmi O, S, N,
- R₇ représente un radical phényle, phénylalkyle ou -alk-COOR₄,
- R₈ représente un atome d'hydrogène ou un radical alkyle, cycloalkyle ou phénylalkyle,
- alk représente un radical alkylène,
étant entendu que les radicaux et portions alcoxy, alkyle et alkylène contiennent 1 à 6 atomes de carbone et sont en chaîne droite ou ramifiée et les radicaux cycloalkyle contiennent 3 à 6 atomes de carbone,
leurs sels, leurs énantiomères et diastéréoisomères.

2. Composés de formule (I) selon la revendication 1 pour lesquels lorsque R₅ et R₆ forment avec l'atome d'azote auquel ils sont rattachés un hétérocycle celui-ci est choisi parmi les cycles azétidine, pyrrolidine, pipéridine, pipérazine et morpholine.

3. Composés de formule (I) selon les revendications 1 ou 2 pour lesquels le substituant R₁ est en position -8 ou -9.

4. Composés de formule (I) selon la revendication 1 pour lesquels R représente un atome d'hydrogène ou un radical carboxy, R₁ représente un radical -alk-NH-CO-R₃, -alk-COOR₄, -alk-CO-NR₅R₆ ou -CO-NH-R₇, R₃ représente un radical alkyle ou -NR₆R₈, R₄ représente un atome d'hydrogène, R₅ représente un atome d'hydrogène, R₆ représente un radical alkyle, R₇ représente un radical phénylalkyle ou -alk-COOR₄ leurs sels, leurs énantiomères et diastéréoisomères

5. Composés de formule (I) selon la revendication suivants :
- acide (4,5-dihydro-4-oxo-10H-imidazo[1,2-a]indéno[1,2-e]pyrazin-9-yl)acétique,
- N-méthyl-2-(4,5-dihydro-4-oxo-10H-imidazo[1,2-a]indéno[1,2-e]pyrazin-9-yl)acétamide,
- N-[(4,5-dihydro-4-oxo-10H-imidazo[1,2-a]indéno[1,2-e]pyrazin-9-yl)méthyl]acétamide,
- 9-[(3-méthyluréido)méthyl]-5H,10H-imidazo[1,2-a]indéno[1,2-e]pyrazin-4-one,
- N-méthyl[4,5-dihydro-4-oxo-10H-imidazo[1,2-a]indéno[1,2-e]pyrazin-8-yl]acétamide,
- acide 8-N-méthylcarboxamidométhyl-4,5-dihydro-4-oxo-10H-imidazo[1,2-a]indéno[1 ,2-e]pyrazin-2-carboxylique,
- acide 8-carboxyméthyl-4,5-dihydro-4-oxo-10H-imidazo[1,2-a]indéno[1,2-e]pyrazin-2-carboxylique,
- 8-(3-méthyluréido)méthyl-5H,10H-imidazo[1,2-a]indéno[1,2-e]pyrazin-4-one,
- acide 9-carboxyméthyl-4,5-dihydro-4-oxo-10H-imidazo[1,2-a]indéno[1,2-e]pyrazin-2-carboxylique,
- N-benzyl-(4,5-dihydro-4-oxo-10H-imidazo[1,2-a]indéno[1,2-e]pyrazin-9-yl)carboxamide,
- N-[(4,5-dihydro-4-oxo-10H-imidazo[1,2-a]indéno[1,2-e]pyrazin-9-yl)carbonyl]glycine,
- N-benzyl-(4,5-dihydro-4-oxo-10H-imidazo[1,2-a]indéno[1,2-e]pyrazin-8-yl)carboxamide,
- acide 8-(N-éthylaminocarbonylméthyl)-4,5-dihydro-4-oxo-10H-imidazo[1,2-a]indéno [1,2-e]pyrazin-2-carboxylique,
- 8-(N-éthylaminocarbonylméthyl)-4,5-dihydro-4-oxo-10H-imidazo[1,2-a]indéno[1,2-e]pyrazin-2-carboxylate d'éthyle,
- acide 9-N-benzylcarbamoyl-4,5-dihydro-4-oxo-10H-imidazo[1,2-a]indèno[1,2-e]pyrazin-2-carboxylique,
- acide 8-(2-carboxyéthyl)-4,5-dihydro-4-oxo-10H-imidazo[1,2-a]indéno[1,2-e]pyrazine-2-carboxylique,
- acide 9-[(3-méthyluréido)méthyl]-4,5-dihydro-4-oxo-10H-imidazo[1,2-a]indéno[1,2-e]pyrazin-2-carboxylique,
- acide 9-carboxyméthyl-4,5-dihydro-4-oxo-10H-imidazo[1,2-a]indéno[1,2-e]pyrazin-2-phosphonique,
- acide 9-N-méthylaminocarbonylméthyl-4,5-dihydro-4-oxo-10H-imidazo[1,2-a]indéno[1,2-e]pyrazin-2-carboxylique,
- acide 9-(1-carboxyéthyl)- 4,5-dihydro-4-oxo-10H-imidazo[1,2-a]indéno[1,2-e]pyrazin-2-carboxylique,
leurs sels, leurs énantiomères et diastéréoisomères.

6. Procédé de préparation des composés de formule (I) selon la revendication 1 **caractérisé en ce que** l'on cyclise en présence d'acétate d'ammonium un dérivé de formule : 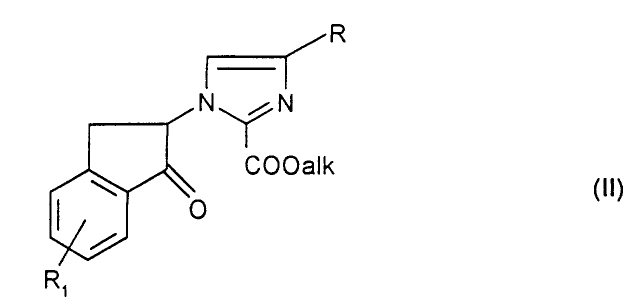 dans laquelle R et R₁ ont les mêmes significations que dans la revendication 1 et alk représente un radical alkyle, isole le produit et le transforme éventuellement en sel.

7. Procédé de préparation des composés de formule (I) selon la revendication 1 pour lesquels R représente un radical carboxy et/ou R₁ représente un radical -alk-COOR₄ dans lequel R₄ représente un atome d'hydrogène ou -CO-NH-R₇ dans lequel R₇ représente un radical -alk-COOR₄ et R₄ représente un atome d'hydrogène **caractérisé en ce que** l'on hydrolyse un composé de formule (I) correspondant pour lequel R représente un radical alcoxycarbonyle et/ou R₁ représente un radical -alk-COOR₄ dans lequel R₄ représente un radical alkyle ou -CO-NH-R₇ dans lequel R₇ représente un radical -alk-COOR₄ et R₄ représente un radical alkyle, isole le produit et le transforme éventuellement en sel.

8. Procédé de préparation des composés de formule (I) selon la revendication 1 pour lesquels R représente un radical -PO₃H₂ **caractérisé en ce que** l'on hydrolyse un dérivé de formule: 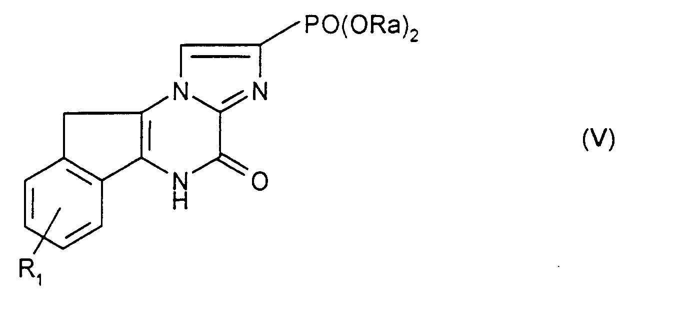 dans laquelle R₁ a les mêmes significations que dans la revendication 1 et Ra représente un radical méthyle, éthyle ou benzyle, isole le produit et le transforme éventuellement en sel.

9. Procédé de préparation des composés de formule (I) selon la revendication 1 pour lesquels R représente un radical -CH₂OH **caractérisé en ce que** l'on réduit un composé de formule (I) correspondant pour lequel R représente un radical alcoxycarbonyle, isole le produit et le transforme éventuellement en sel.

10. Procédé de préparation des composés de formule (I) selon la revendication 1 pour lesquels R représente un radical -CO-NR₄R₅ **caractérisé en ce que** l'on fait réagir un complexe d'aluminium formé à partir d'un trialkylaluminium et du chlorhydrate d'une amine HNR₄R₅ sur un composé de formule (I) correspondant pour lequel R représente un radical alcoxycarbonyle, isole le produit et le transforme éventuellement en sel.

11. Procédé de préparation des composés de formule (I) selon la revendication 1 pour lesquels R₁ représente un radical -alk-NH-CO-R₃ dans lequel R₃ représente un radical alkyle (1C) **caractérisé en ce que** l'on fait réagir un composé de formule (I) correspondant pour lequel R₁ représente un radical -alk-NH₂ sur l'anhydride acétique, isole le produit et le transforme éventuellement en sel.

12. Procédé de préparation des composés de formule (I) selon la revendication 1 pour lesquels R₁ représente un radical -alk-NH-CO-R₃ dans lequel R₃ représente un radical -NR₆R₈, R₆ représente un atome d'hydrogène et R₈ représente un atome d'hydrogène ou un radical alkyle, cycloalkyle ou phénylalkyle **caractérisé en ce que** l'on fait réagir un composé de formule (I) correspondant pour lequel R₁ représente un radical -alk-NH₂ sur un isocyanate R_{b}-NCO dans lequel R_{b} représente un radical triméthylsilyle, alkyle, cycloalkyle ou phénylalkyle suivie éventuellement d'une hydrolyse du dérivé silylé, isole le produit et le transforme éventuellement en sel.

13. Procédé de préparation des composés de formule (I) selon la revendication 1 pour lesquels R₁ représente un radical -alk-NH-CO-R₃ dans lequel R₃ représente un radical -NR₆R₈, R₆ représente un atome d'hydrogène et R₈ représente un radical alkyle, cycloalkyle ou phénylalkyle **caractérisé en ce que** l'on fait réagir un composé de formule (I) correspondant pour lequel R₁ représente un radical -alk-NH₂ sur un dérivé de formule : 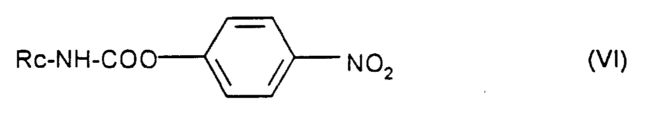 dans laquelle Rc représente un radical alkyle, cycloalkyle ou phénylalkyle, isole le produit et le transforme éventuellement en sel.

14. Procédé de préparation des composés de formule (I) selon la revendication 1 pour lesquels R₁ représente un radical -alk-NH-CO-R₃ dans lequel R₃ représente un radical alkyle, phényle, phénylalkyle, cycloalkyle ou -NR₆R₈, R₆ représente un radical alkyle et R₈ représente un radical alkyle, cycloalkyle ou phénylalkyle **caractérisé en ce que** l'on fait réagir un composé de formule (I) correspondant pour lequel R₁ représente un radical -alk-NH₂ sur un dérivé Hal-CO-R₃ dans lequel Hal représente un atome d'halogène et R₃ a les mêmes significations que précédemment, isole le produit et le transforme éventuellement en sel.

15. Procédé de préparation des composés de formule (I) selon la revendication 1 pour lesquels R₁ représente un radical -alk-CO-NR₅R₆ **caractérisé en ce que** l'on fait réagir un composé de formule (I) correspondant pour lequel R₁ représente un radical -alk-COOR₄ ou lorsque R₄ représente un atome d'hydrogène, un dérivé réactif de cet acide sur une amine HNR₅R₆, isole le produit et le transforme éventuellement en sel.

16. Procédé de préparation des composés de formule (I) selon la revendication 1 pour lesquels R₁ représente un radical -alk(1C)-NH₂ **caractérisé en ce que** l'on hydrogène un dérivé de formule : 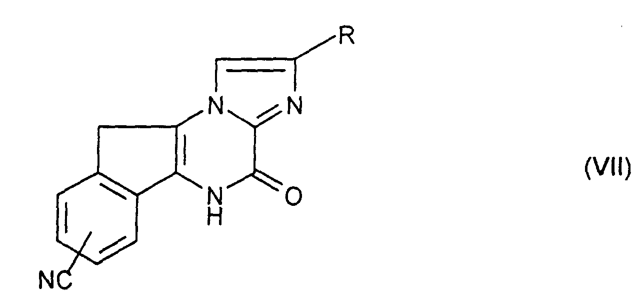 dans laquelle R a les mêmes significations que dans la formule (I), isole le produit et le transforme éventuellement en sel.

17. Médicaments contenant en tant que pricipe actif au moins un composé de formule (I) selon l'une des revendications 1 à 5 ou un sel d'un tel composé.

18. Médicaments antagonistes du récepteur de l'acide α-amino-3-hydroxy-5-méthyl-4-isoxazolepropionique (AMPA) contenant en tant que pricipe actif au moins un composé de formule (I) selon l'une des revendications 1 à 5 ou un sel d'un tel composé.

19. Médicaments antagonistes non compétitifs des sites glycine du récepteur N-méthyl-D-aspartate (NMDA) contenant en tant que pricipe actif au moins un composé de formule (I) selon l'une des revendications 1 à 5 ou un sel d'un tel composé.

20. Produits de formule : 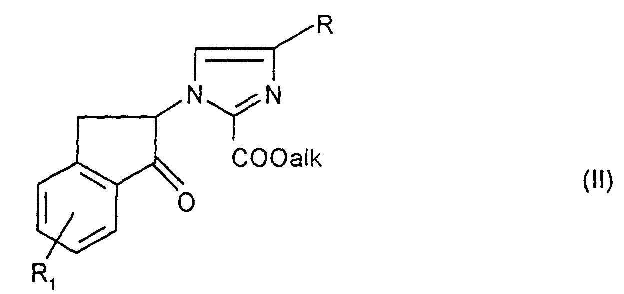 dans laquelle R et R₁ ont les mêmes significations que dans la revendication 1 et alk représente un radical alkyle contenant 1 à 6 atomes de carbone en chaîne droite ou ramifiée.

## Claims

1. Compounds of formula: 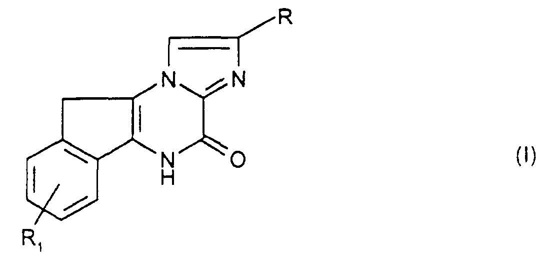 in which
- R represents a hydrogen atom or a carboxy, alkoxycarbonyl, -CO-NR₄R₅, -PO₃H₂ or -CH₂OH radical,
- R₁ represents an -alk-NH₂, -alk-NH-CO-R₃, -alk-COOR₄, -alk-CO-NR₅R₆ or -CO-NH-R₇ radical,
- R₃ represents an alkyl, phenyl, phenylalkyl, cycloalkyl or -NR₆R₈ radical,
- R₄ represents a hydrogen atom or an alkyl radical,
- R₅ represents a hydrogen atom or an alkyl, phenyl, cycloalkyl or phenylalkyl radical,
- R₆ represents a hydrogen atom or an alkyl radical,
or else R₅ and R₆ form, with the nitrogen atom to which they are attached, a saturated or unsaturated, mono- or polycyclic heterocycle containing 1 to 6 carbon atoms and optionally one or a number of other heteroatoms chosen from O, S and N,
- R₇ represents a phenyl, phenylalkyl or -alk-COOR₄ radical,
- R₈ represents a hydrogen atom or an alkyl, cycloalkyl or phenylalkyl radical,
- alk represents an alkylene radical,
it being understood that the alkoxy, alkyl and alkylene radicals and portions contain 1 to 6 carbon atoms and are in a straight or branched chain and the cycloalkyl radicals contain 3 to 6 carbon atoms,
their salts and their enantiomers and diastereoisomers.

2. Compounds of formula (I) according to Claim 1 in which, when R₅ and R₆ form a heterocycle with the nitrogen atom to which they are attached, this heterocycle is chosen from azetidine, pyrrolidine, piperidine, piperazine and morpholine rings.

3. Compounds of formula (I) according to Claims 1 or 2 in which the R₁ substituent is in the 8- or 9-position.

4. Compounds of formula (I) according to Claim 1 in which R represents a hydrogen atom or a carboxy radical, R₁ represents an -alk-NH-CO-R₃, -alk-COOR₄, -alk-CO-NR₅R₆ or -CO-NH-R₇ radical, R₃ represents an alkyl or -NR₆R₈ radical, R₄ represents a hydrogen atom, R₅ represents a hydrogen atom, R₆ represents an alkyl radical and R₇ represents a phenylalkyl or -alk-COOR₄ radical, their salts and their enantiomers and diastereoisomers.

5. Following compounds of formula (I) according to claim 1:
- (4,5-dihydro-4-oxo-10H-imidazo[1,2-a]indeno[1,2-e]pyrazin-9-yl)acetic acid,
- N-methyl-2-(4,5-dihydro-4-oxo-10H-imidazo [1,2-a] indeno [1,2-e]pyrazin-9-yl)acetamide,
- N-[(4,5-dihydro-4-oxo-10H-imidazo[1,2-a]indeno[1,2-e]pyrazin-9-yl)methyl]acetamide,
- 9-[(3-methylureido)methyl]-5H,10H-imidazo [1,2-a]indeno[1,2-e]pyrazin-4-one,
- N-methyl-[4,5-dihydro-4-oxo-10H-imidazo[1,2-a]indeno[1,2-e]pyrazin-8-yl]acetamide,
- 8-N-methylcarboxamidomethyl-4, 5-dihydro-4-oxo-10H-imidazo[1,2-a]indeno[1,2-e]pyrazin-2-carboxylic acid,
- 8-carboxymethyl-4,5-dihydro-4-oxo-10H-imidazo[1,2-a]indeno[1,2-e]pyrazin-2-carboxylic acid,
- 8-(3-methylureido)methyl-5H, 10H-imidazo[1,2-a]indeno[1,2-e]pyrazin-4-one,
- 9-carboxymethyl-4,5-dihydro-4-oxo-10H-imidazo[1,2-a]indeno[1,2-e]pyrazin-2-carboxylic acid,
- N-benzyl-(4,5-dihydro-4-oxo-10H-imidazo[1,2-a]indeno[1,2-e]pyrazin-9-yl)carboxamide,
- N-[(4,5-dihydro-4-oxo-10H-imidazo[1,2-a]indeno[1,2-e]pyrazin-9-yl)carbonyl]glycine,
- N-benzyl-(4,5-dihydro-4-oxo-10H-imidazo[1,2-a]indeno[1,2-e]pyrazin-8-yl)carboxamide,
- 8-(N-ethylaminocarbonylmethyl)-4,5-dihydro-4-oxo-10H-imidazo [1,2-a]indeno[1,2-e]pyrazin-2-carboxylic acid,
- ethyl 8-(N-ethylaminocarbonylmethyl)-4,5-dihydro-4-oxo-10H-imidazo[1,2-a]indeno[1,2-e]pyrazin-2-carboxylate,
- 9-N-benzylcarbamoyl-4,5-dihydro-4-oxo-10H-imidazo[1,2-a]indeno[1,2-e]pyrazin-2-carboxylic acid,
- 8-(2-carboxyethyl)-4,5-dihydro-4-oxo-10H-imidazo[1,2-a]indeno[1,2-e]pyrazine-2-carboxylic acid,
- 9-[(3-methylureido)methyl]-4,5-dihydro-4-oxo-10H-imidazo[1,2-a]indeno[1,2-e]pyrazin-2-carboxylic acid,
- 9-carboxymethyl-4,5-dihydro-4-oxo-10H-imidazo[1,2-a]indeno[1,2-e]pyrazin-2-phosphonic acid,
- 9-N-methylaminocarbonylmethyl-4,5-dihydro-4-oxo-10H-imidazo [1,2-a]indeno[1,2-e]pyrazin-2-carboxylic acid,
- 9-(1-carboxyethyl)-4,5-dihydro-4-oxo-10H-imidazo[1,2-a]indeno[1,2-e]pyrazin-2-carboxylic acid, their salts and their enantiomers and diastereoisomers.

6. Process for the preparation of the compounds of formula (I) according to Claim 1,
**characterized in that** a derivative of formula: 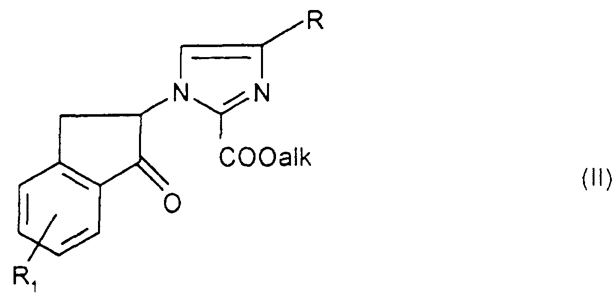 in which R and R₁ have the same meanings as in Claim 1 and alk represents an alkyl radical, is cyclized in the presence of ammonium acetate, the product is isolated and is optionally converted to a salt.

7. Process for the preparation of the compounds of formula (I) according to Claim 1 in which R represents a carboxy radical and/or R₁ represents an -alk-COOR₄ radical in which R₄ represents a hydrogen atom or a -CO-NH-R₇ radical in which R₇ represents an -alk-COOR₄ radical and R₄ represents a hydrogen atom, **characterized in that** a corresponding compound of formula (I), in which R represents an alkoxycarbonyl radical and/or R₁ represents an -alk-COOR₄ radical in which R₄ represents an alkyl radical or a -CO-NH-R₇ radical in which R₇ represents an -alk-COOR₄ radical and R₄ represents an alkyl radical, is hydrolysed, the product is isolated and is optionally converted to a salt.

8. Process for the preparation of the compounds of formula (I) according to Claim 1 in which R represents a -PO₃H₂ radical, **characterized in that** a derivative of formula: 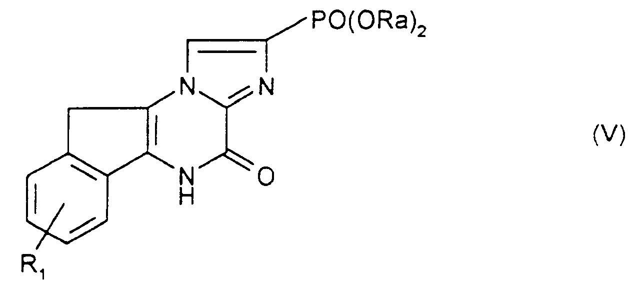 in which R₁ has the same meanings as in Claim 1 and Ra represents a methyl, ethyl or benzyl radical, is hydrolysed, the product is isolated and is optionally converted to a salt.

9. Process for the preparation of the compounds of formula (I) according to Claim 1 in which R represents a -CH₂OH radical, **characterized in that** a corresponding compound of formula (I) in which R represents an alkoxycarbonyl radical is reduced, the product is isolated and is optionally converted to a salt.

10. Process for the preparation of the compounds of formula (I) according to Claim 1 in which R represents a -CO-NR₄R₅ radical, **characterized in that** an aluminium complex formed from a trialkylaluminium and the hydrochloride of an amine HNR₄R₅ is reacted with a corresponding compound of formula (I) in which R represents an alkoxycarbonyl radical, the product is isolated and is optionally converted to a salt.

11. Process for the preparation of the compounds of formula (I) according to Claim 1 in which R₁ represents an -alk-NH-CO-R₃ radical in which R₃ represents a (1C)alkyl radical, **characterized in that** a corresponding compound of formula (I) in which R₁ represents an -alk-NH₂ radical is reacted with acetic anhydride, the product is isolated and is optionally converted to a salt.

12. Process for the preparation of the compounds of formula (I) according to Claim 1 in which R₁ represents an -alk-NH-CO-R₃ radical in which R₃ represents an -NR₆R₈ radical, R₆ represents a hydrogen atom and R₈ represents a hydrogen atom or an alkyl, cycloalkyl or phenylalkyl radical, **characterized in that** a corresponding compound of formula (I) in which R₁ represents an -alk-NH₂ radical is reacted with an isocyanate R_{b}-NCO in which R_{b} represents a trimethylsilyl, alkyl, cycloalkyl or phenylalkyl radical, optionally followed by hydrolysis of the silylated derivative, the product is isolated and is optionally converted to a salt.

13. Process for the preparation of the compounds of formula (I) according to Claim 1 in which R₁ represents an -alk-NH-CO-R₃ radical in which R₃ represents an -NR₆R₈ radical, R₆ represents a hydrogen atom and R₈ represents an alkyl, cycloalkyl or phenylalkyl radical, **characterized in that** a corresponding compound of formula (I) in which R₁ represents an -alk-NH₂ radical is reacted with a derivative of formula: 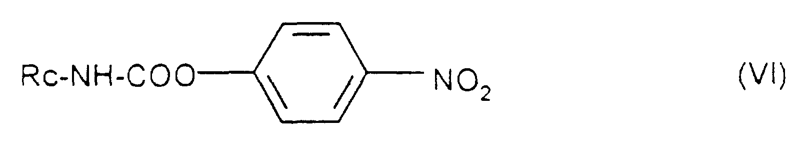 in which Rc represents an alkyl, cycloalkyl or phenylalkyl radical, the product is isolated and is optionally converted to a salt.

14. Process for the preparation of the compounds of formula (I) according to Claim 1 in which R₁ represents an -alk-NH-CO-R₃ radical in which R₃ represents an alkyl, phenyl, phenylalkyl, cycloalkyl or -NR₆R₈ radical, R₆ represents an alkyl radical and R₈ represents an alkyl, cycloalkyl or phenylalkyl radical, **characterized in that** a corresponding compound of formula (I) in which R₁ represents an -alk-NH₂ radical is reacted with a Hal-CO-R₃ derivative in which Hal represents a halogen atom and R₃ has the same meanings as above, the product is isolated and is optionally converted to a salt.

15. Process for the preparation of the compounds of formula (I) according to Claim 1 in which R₁ represents an -alk-CO-NR₅R₆ radical, **characterized in that** a corresponding compound of formula (I) in which R₁ represents an -alk-COOR₄ radical or, when R₄ represents a hydrogen atom, a reactive derivative of this acid is reacted with an amine HNR₅R₆, the product is isolated and is optionally converted to a salt.

16. Process for the preparation of the compounds of formula (I) according to Claim 1 in which R₁ represents a -(1C)alk-NH₂ radical, **characterized in that** a derivative of formula: 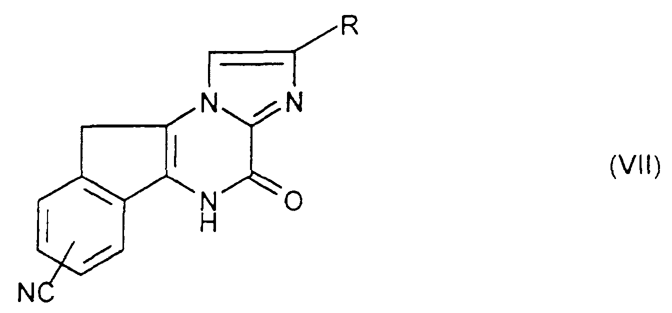 in which R has the same meanings as in the formula (I), is hydrogenated, the product is isolated and is optionally converted to a salt.

17. Medicaments containing, as active ingredient, at least one compound of formula (I) according to one of Claims 1 to 5 or a salt of such a compound.

18. Medicaments which are antagonists of the receptor for α-amino-3-hydroxy-5-methyl-4-isoxazolepropionic acid (AMPA) containing, as active ingredient, at least one compound of formula (I) according to one of Claims 1 to 5 or a salt of such a compound.

19. Medicaments which are non-competitive antagonists of the glycine sites of the N-methyl-D-aspartate (NMDA) receptor containing, as active ingredient, at least one compound of formula (I) according to one of Claims 1 to 5 or a salt of such a compound.

20. Products of formula: 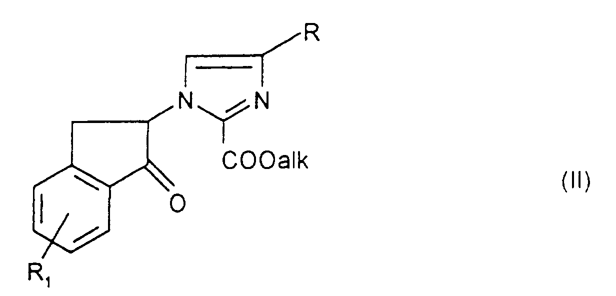 in which R and R₁ have the same meanings as in Claim 1 and alk represents an alkyl radical containing 1 to 6 carbon atoms in a straight or branched chain.

## Patentansprüche

1. Verbindungen der Formel (I) 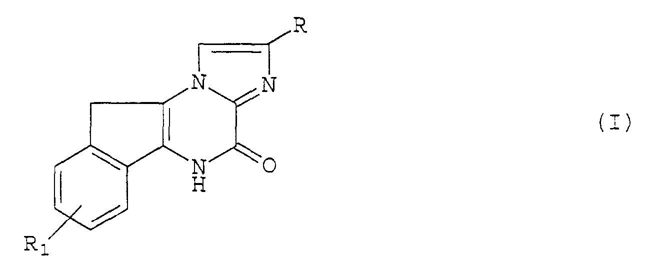 in der
- R ein Wasserstoffatom oder einen Rest Carboxy, Alkoxycarbonyl, -CO-NR₄R₅, -PO₃H₂ oder -CH₂OH darstellt,
- R₁ einen Rest -alk-NH₂, -alk-NH-CO-R₃, -alk-COOR₄, -alk-CO-NR₅R₆ oder -CO-NH-R₇ darstellt,
- R₃ einen Rest Alkyl, Phenyl, Phenylalkyl, Cycloalkyl oder -NR₆R₈ darstellt,
- R₄ ein Wasserstoffatom oder ein Rest Alkyl ist,
- R₅ ein Wasserstoffatom oder einen Rest Alkyl, Phenyl, Cycloalkyl oder Phenylalkyl bedeutet,
- R₆ ein Wasserstoffatom oder ein Rest Alkyl ist,
oder R₅ und R₆ zusammen mit dem Stickstoffatom, an das sie gebunden sind, einen mono- oder polycyclischen, gesättigten oder ungesättigten Heterocyclus mit 1 bis 6 Kohlenstoffatomen und gegebenenfalls e-nem oder mehreren Heteroatomen, ausgewählt unter O, S, N, bilden,
- R₇ einen Rest Phenyl, Phenylalkyl oder -alk-COOR₄ bedeutet,
- R₈ ein Wasserstoffatom oder einen Rest Alkyl, Cycloalkyl oder Phenylalkyl darstellt,
- alk ein Rest Alkylen ist,
mit der Maßgabe, daß die Reste und Teile Alkoxy, Alkyl und Alkylen 1 bis 6 Kohlenstoffatome enthalten und in gerader oder verzweigter Kette vorliegen, und die Reste Cycloalkyl 3 bis 6 Kohlenstoffatome enthalten,
sowie ihre Salze, ihre Enantiomeren und Diastereoisomeren.

2. Verbindungen der Formel (I) nach Anspruch 1, bei denen in dem Fall, wo R₅ und R₆ zusammen mit dem Stickstoffatom, an das sie gebunden sind, einen Heterocyclus bilden, dieser unter den Ringen Azetidin, Pyrrolidin, Piperidin, Piperazin und Morpholin ausgewählt wird.

3. Verbindungen der Formel (I) nach Anspruch 1 oder 2, worin sich der Substituent R₁ in Position -8 oder -9 befindet.

4. Verbindungen der Formel (I) nach Anspruch 1, in der R ein Wasserstoffatom oder einen Rest Carboxy darstellt, R₁ einen Rest - alk-NH-CO-R₃, -alk-COOR₄, -alk-CO-NR₅R₆ oder -CO-NH-R₇ darstellt, R₃ einen Rest Alkyl oder -NR₆R₈ bedeutet, R₄ ein Wasserstoffatom ist, R₅ ein Wasserstoffatom ist, R₆ einen Rest Alkyl darstellt, R₇ einen Rest Phenylalkyl oder -alk-COOR₄ bedeutet, sowie ihre Salze, ihre Enantiomeren und Diastereoisomeren.

5. Die folgenden anspruchsgemäßen Verbindungen der Formel (I):
- (4,5-Dihydro-4-oxo-10H-imidazo[1,2-a]-indeno[1,2-e]-pyrazin-9-yl)-essigsäure,
- N-Methyl-2-(4,5-dihydro-4-oxo-10H-imidazo[1,2-a]-indeno[1,2-e]-pyrazin-9-yl)-acetamid,
- N-[(4,5-Dihydro-4-oxo-10H-imidazo[1,2-a]-indeno[1,2-e]-pyrazin-9-yl) -methyl]-acetamid,
- 9-[(3-Methylureido)-methyl]-5H,10H-imidazo[1,2-a]-indeno-[1,2-e]-pyrazin-4-on,
N-Methyl-(4,5-dihydro-4-oxo-10H-imidazo[1,2-a]-indeno[1,2-e]-pyrazin-8-yl)-acetamid,
- 8-N-Methyl-carboxamidomethyl-4,5-dihydro-4-oxo-10H-imidazo-[1,2-a]-indeno[1,2-e]-pyrazin-2-carbonsäure,
- 8-Carboxymethyl-4,5-dihydro-4-oxo-10H-imidazo[1,2-a]-indeno-[1,2-e]-pyrazin-2-carbonsäure,
- 8-(3-Methylureido)-methyl-5H,10H-imidazo[1,2-a]-indeno-[1,2-e]-pyrazin-4-on,
- 9-Carboxymethyl-4,5-dihydro-4-oxo-10H-imidazo[1,2-a]-indeno-[1,2-e]-pyrazin-2-carbonsäure,
- N-Benzyl-(4,5-dihydro-4-oxo-10H-imidazo[1,2-a]-indeno[1,2-e]-pyrazin-9-yl)-carboxamid,
- N-[(4,5-Dihydro-4-oxo-10H-imidazo[1,2-a]-indeno[1,2-e]-pyrazin-9-yl)-carbonyl]-glycin,
- N-Benzyl-(4,5-dihydro-4-oxo-10H-imidazo[1,2-a]-indeno[1,2-e]-pyrazin-8-yl) -carboxamid,
- 8-(N-Ethylamino-carbonylmethyl)-4,5-dihydro-4-oxo-10H-imidazo[1,2-a]-indeno[1,2-e]-pyrazin-2-carbonsäure,
- 8-(N-Ethylamino-carbonylmethyl) -4,5-dihydro-4-oxo-10H-imidazo[1,2-a]-indeno[1,2-e]-pyrazin-2-carbonsäure-ethylester,
- 9-N-Benzylcarbamoyl-4,5-dihydro-4-oxo-10H-imidazo[1,2-a]-indeno[1,2-e]-pyrazin-2-carbonsäure,
- 8-(2-Carboxyethyl)-4,5-dihydro-4-oxo-10H-imidazo[1,2-a]-indeno[1,2-e]-pyrazin-2-carbonsäure,
- 9-[(3-Methylureido)-methyl]-4,5-dihydro-4-oxo-10H-imidazo-[1,2-a]-indeno[1,2-e]-pyrazin-2-carbonsäure,
- 9-Carboxymethyl-4,5-dihydro-4-oxo-10H-imidazo[1,2-a]-indeno-[1,2-e]-pyrazin-2-phosphonsäure,
- 9-N-Methylaminocarbonylmethyl-4,5-dihydro-4-oxo-10H-imidazo-[1,2-a]-indeno[1,2-e]-pyrazin-2-carbonsäure, 9-(1-Carboxyethyl)-4,5-dihydro-4-oxo-10H-imidazo[1,2-a]-indeno[1,2-e]-pyrazin-2-carbonsäure,
sowie ihre Salze, ihre Enantiomeren und Diastereoisomeren.

6. Verfahren zur Herstellung der Verbindungen der Formel (I) nach Anspruch 1, **dadurch gekennzeichnet, daß** man ein Derivat der Formel (II) 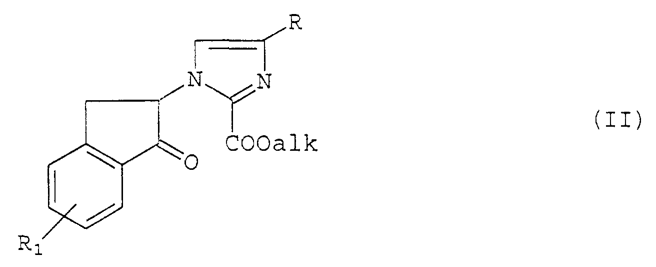 in der
R und R₁ die gleichen Bedeutungen wie in Anspruch 1 besitzen und alk einen Rest Alkyl darstellt, in Anwesenheit von Ammoniumacetat cyclisiert, das Produkt isoliert und gegebenenfalls in Salz überführt.

7. Verfahren zur Herstellung der Verbindungen der Formel (I) nach Anspruch 1, in der R einen Rest Carboxy und/oder R₁ einen Rest -alk-COOR₄ darstellen, worin R₄ ein Wasserstoffatom oder -CO-NH-R₇ ist, worin R₇ einen Rest -alk-COOR₄ darstellt und R₄ ein Wasserstoffatom ist, **dadurch gekennzeichnet, daß** man eine entsprechende Verbindung der Formel (I), in der R einen Rest Alkoxycarbonyl und/oder R₁ einen Rest -alk-COOR₄ darstellen, worin R₄ ein Rest Alkyl oder -CO-NH-R₇ ist, worin R₇ einen Rest -alk-COOR₄ darstellt und R₄ ein Rest Alkyl ist, hydrolysiert, das Produkt isoliert und gegebenenfalls in Salz überführt.

8. Verfahren zur Herstellung der Verbindungen der Formel (I) nach Anspruch 1, in der R einen Rest -PO₃H₂ darstellt, **dadurch gekennzeichnet, daß** man ein Derivat der Formel (V) 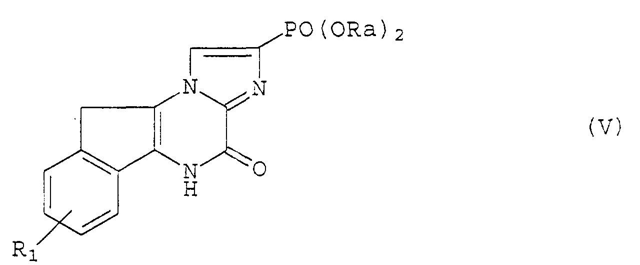 in der R₁ die gleichen Bedeutungen wie in Anspruch 1 besitzt und Ra einen Rest Methyl, Ethyl oder Benzyl darstellt, hydrolysiert, das Produkt isoliert und gegebenenfalls in Salz überführt.

9. Verfahren zur Herstellung der Verbindungen der Formel (I) nach Anspruch 1, in der R einen Rest -CH₂OH darstellt, **dadurch gekennzeichnet, daß** man eine entsprechende Verbindung der Formel (I), in der R einen Rest Alkoxycarbonyl bedeutet, reduziert, das Produkt isoliert und gegebenenfalls in Salz überführt.

10. Verfahren zur Herstellung der Verbindungen der Formel (I) nach Anspruch 1, in der R einen Rest -CO-NR₄R₅ darstellt, **dadurch gekennzeichnet, daß** man einen Aluminiumkomplex, gebildet ausgehend von einem Trialkylaluminium und dem Hydrochlorid eines Amins HNR₄R₅, mit einer entsprechenden Verbindung der Formel (I), in der R einen Rest Alkoxycarbonyl bedeutet, zur Reaktion bringt, das Produkt isoliert und gegebenenfalls in Salz überführt.

11. Verfahren zur Herstellung der Verbindungen der Formel (I) nach Anspruch 1, in der R₁ einen Rest -alk-NH-CO-R₃ darstellt, worin R₃ ein Rest Alkyl (1C) ist, **dadurch gekennzeichnet, daß** man eine entsprechende Verbindung der Formel (I), in der R₁ einen Rest -alk-NH₂ bedeutet, mit Essigsäureanhydrid zur Reaktion bringt, das Produkt isoliert und gegebenenfalls in Salz überführt.

12. Verfahren zur Herstellung der Verbindungen der Formel (I) nach Anspruch 1, in der R₁ einen Rest -alk-NH-CO-R₃ darstellt, worin R₃ ein Rest -NR₆R₈ ist, R₆ ein Wasserstoffatom bedeutet und R₈ ein Wasserstoffatom oder einen Rest Alkyl, Cycloalkyl oder Phenylalkyl darstellt, **dadurch gekennzeichnet, daß** man eine entsprechende Verbindung der Formel (I), in der R₁ einen Rest -alk-NH₂ bedeutet, mit einem Isocyanat Rb-NCO, worin Rb ein Rest Trimethylsilyl, Alkyl, Cycloalkyl oder Phenylalkyl ist, zur Reaktion bringt, gegebenenfalls eine Hydrolyse des silylierten Derivates anschließt, das Produkt isoliert und gegebenenfalls in Salz überführt.

13. Verfahren zur Herstellung der Verbindungen der Formel (I) nach Anspruch 1, in der R₁ einen Rest -alk-NH-CO-R₃ darstellt, worin R₃ ein Rest -NR₆R₈ ist, R₆ ein Wasserstoffatom bedeutet und R₈ einen Rest Alkyl, Cycloalkyl oder Phenylalkyl darstellt, **dadurch gekennzeichnet, daß** man eine entsprechende Verbindung der Formel (I), in der R₁ einen Rest -alk-NH₂ bedeutet, mit einem Derivat der Formel (VI) 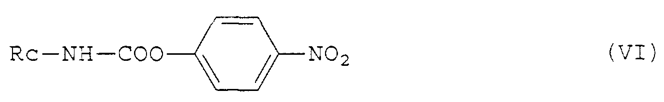 worin Rc ein Rest Alkyl, Cycloalkyl oder Phenylalkyl ist, zur Reaktion bringt, das Produkt isoliert und gegebenenfalls in Salz überführt.

14. Verfahren zur Herstellung der Verbindungen der Formel (I) nach Anspruch 1, in der R₁ einen Rest -alk-NH-CO-R₃ darstellt, worin R₃ ein Rest Alkyl, Phenyl, Phenylalkyl, Cycloalkyl oder -NR₆R₈ ist, R₆ einen Rest Alkyl bedeutet und R₈ einen Rest Alkyl, Cycloalkyl oder Phenylalkyl darstellt, **dadurch gekennzeichnet, daß** man eine entsprechende Verbindung der Formel (I), in der R₁ einen Rest -alk-NH₂ bedeutet, mit einem Derivat Hal-CO-R₃, worin Hal ein Halogenatom ist und R₃ die gleichen Bedeutungen wie vorstehend besitzt, zur Reaktion bringt, das Produkt isoliert und gegebenenfalls in Salz überführt.

15. Verfahren zur Herstellung der Verbindungen der Formel (I) nach Anspruch 1, in der R₁ einen Rest -alk-CO-NR₅R₆ darstellt, **dadurch gekennzeichnet, daß** man eine entsprechende Verbindung der Formel (I), in der R₁ einen Rest -alk-COOR₄ bedeutet, oder, wenn R₄ ein Wasserstoffatom ist, ein reaktives Derivat dieser Säure, mit einem Amin HNR₅R₆ zur Reaktion bringt, das Produkt isoliert und gegebenenfalls in Salz überführt.

16. Verfahren zur Herstellung der Verbindungen der Formel (I) nach Anspruch 1, in der R₁ einen Rest -alk(1C)-NH₂ darstellt, **dadurch gekennzeichnet, daß** man ein Derivat der Formel (VII) 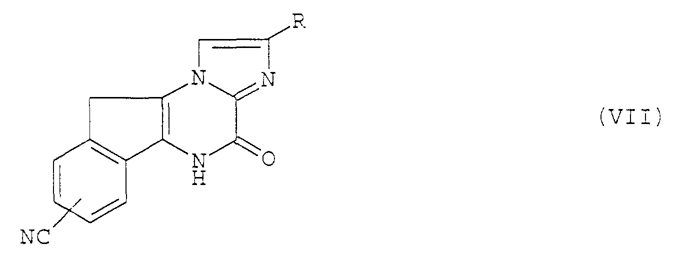 in der R die gleichen Bedeutungen wie in Formel (I) besitzt, hydriert, das Produkt isoliert und gegebenenfalls in Salz überführt .

17. Arzneimittel, enthaltend als Wirkstoff mindestens eine Verbindung der Formel (I) nach einem der Ansprüche 1 bis 5 oder ein Salz einer derartigen Verbindung.

18. Antagonisten-Arzneimittel des Rezeptors der α-Amino-3-hydroxy-5-methyl-4-isoxazol-propionsäure (AMPA), enthaltend als Wirkstoff mindestens eine Verbindung der Formel (I) nach einem der Ansprüche 1 bis 5 oder ein Salz einer derartigen Verbindung.

19. Nicht konkurrierende Antagonisten-Arzneimittel der Glycin-Bereiche des Rezeptors N-Methyl-D-aspartat (NMDA), enthaltend als Wirkstoff mindestens eine Verbindung der Formel (I) nach einem der Ansprüche 1 bis 5 oder ein Salz einer derartigen Verbindung.

20. Produkte der Formel (II) 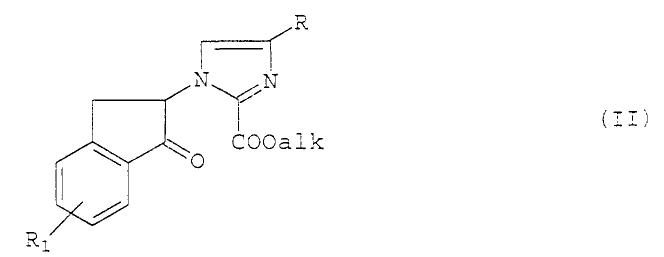 in der
R und R₁ die gleichen Bedeutungen wie in Anspruch 1 besitzen und alk einen Rest Alkyl mit 1 bis 6 Kohlenstoffatomen in gerader oder verzweigter Kette darstellt.
